# EUROPEAN PATENT APPLICATION

(11) **EP 4 650 366 A1**
(43) Date of publication of application: **19.11.2025**
(21) Application number: 24741235.6
(22) Date of filing: 09.01.2024
(51) Int. Cl.: C07K 16/22, C12N 9/00, A61K 38/18, A61K 39/00

(54) **TGFBETA1 BINDING MOLECULE, GARP-TGFBETA1 BINDING MOLECULE AND MEDICAL USE THEREOF**

(30) Priority: 09.01.2023 CN 202310027965; 09.01.2023 CN 202310037692
(71) Applicant: Beijing Tuo Jie Biopharmaceutical Co. Ltd., Beijing 102206 (CN)
(72) Inventor: CAO, Baohua, Beijing 102206 (CN); HE, Xiang, Beijing 102206 (CN); XU, Jinjing, Beijing 102206 (CN); DU, Yanping, Beijing 102206 (CN); WANG, Yu, Beijing 102206 (CN); LIU, Xiao, Beijing 102206 (CN); ZHANG, Xiaoqian, Beijing 102206 (CN); LI, Yuanyuan, Beijing 102206 (CN); SHEN, Chenxi, Beijing 102206 (CN); WANG, Lei, Beijing 102206 (CN)
(74) Representative: Regimbeau
(86) International application number: PCT/CN2024/071321
(87) International publication number: WO 2024/149237

(57) **Abstract**

The present disclosure relates to a TGFβ1 binding molecule, a GARP-TGFβ1 binding molecule and a medical use thereof. In particular, the present disclosure relates to a TGFβ1 binding molecule, a GARP-TGFβ1 binding molecule, a pharmaceutical composition comprising same, a preparation method therefor and a medical use thereof. The present disclosure further relates to a method for treating and/or preventing diseases (e.g., tumors or cancer) by using the TGFβ1 binding molecule, the GARP-TGFβ1 binding molecule, and the pharmaceutical composition comprising same.

## Description

The present disclosure claims priority to Chinese Patent Application No. 202310027965.9 filed on January 9, 2023 and Chinese Patent Application No. 202310037692.6 filed on January 9, 2023, which are incorporated herein by reference in their entirety.

### TECHNICAL FIELD

The present disclosure relates to the field of biopharmaceutics and particularly to a TGFβ1-binding molecule, a pharmaceutical composition comprising the TGFβ1-binding molecule, pharmaceutical use, a preparation method, and a method for preventing or treating a disease associated with the TGFβ1 signaling pathway.

### BACKGROUND

TGFβ (transforming growth factor β) is a class of pleiotropic growth factors, including TGFβ1, TGFβ2, and TGFβ3. They regulate many important physiological processes, such as cell growth, differentiation, proliferation, apoptosis, and matrix production. Studies have found that TGFβ can inhibit tumor growth in early stages of tumorigenesis but promote tumor growth in late stages of tumor progression (Liu S, Ren J, Ten Dijke P. Signal Transduct Target Ther. 2021 Jan 8;6(1):8). An important mechanism by which TGFβ promotes tumor growth in late stages is the suppression of immune cells in the tumor microenvironment. TGFβ1, rather than TGFβ2 and TGFβ3, is a key factor in promoting tumor growth in late stages (Constance J Martin, et al. Sci Transl Med. 2020 Mar 25;12(536):eaay8456). TGFβ1 produced by tumor cells, regulatory T cells (Tregs), inhibitory phagocytes, etc. can directly promote the activity of Treg cells and inhibit effector T cells and antigen-presenting cells, thereby achieving an immunosuppressive effect.

Currently, the tumor immune checkpoint inhibitors anti-PD-1 (programmed cell death-1) antibodies have achieved great success in the immunotherapy of tumors; however, the total response rate in patients is only about 20%. The combination of an anti-TGFβ1 antibody and an anti-PD-1 antibody can significantly overcome the immune evasion of tumors in tumor models such as EMT-6, MBT-2, Cloudman S91, CT26, and MC38, enhancing the efficacy of the anti-PD-1 antibody. However, pan-TGFβ antibodies targeting mature TGFβ1 exhibited dose-dependent *in vivo* toxicity in experiments. Targeting pro/latent TGFβ1 complexes inhibits TGFβ1 production at source. This approach has better efficacy in animal models than targeting mature TGFβ1 and has significantly reduced toxicity.

TGFβ1 production is tightly regulated by a multi-step process. In a TGFβ1 precursor protein, the C-terminal mature TGF-β1 domain remains covalently or non-covalently binding to an N-terminal domain called latency-associated peptide (LAP). Since LAP prevents mature TGF-β1 from binding to its receptor, it is usually inactive. Such a TGFβ1 precursor protein may further form large latent complexes with GARP, LRRC33, LTBP1, LTBP3, etc. In the tumor microenvironment, GARP-TGFβ1 is mainly produced by Tregs and tumor cells, LRRC33-TGFβ1 is mainly produced by immunosuppressive phagocytes, and LTBP1-TGFβ1 and LTBP3-TGFβ1 are mainly present in the tumor stroma. Currently, there are no commercially available antibodies targeting TGFβ1 precursor proteins or TGFβ1 complexes worldwide. There is still a need in the art for drugs that can bind to TGFβ1 precursor proteins or TGFβ1 complexes with high affinities and block their activation, for the treatment of TGFβ-associated diseases such as tumors and fibrosis.

### SUMMARY

The present disclosure provides a TGFβ1-binding molecule, a GARP-TGFβ1-binding molecule, a pharmaceutical composition comprising an aforementioned binding molecule, pharmaceutical use, a preparation method, and a method for preventing or treating a disease associated with a TGFβ signaling pathway, particularly a method for preventing or treating cancer or a tumor.

### TGFβ1-Binding Molecule

In a first aspect, the present disclosure provides a TGFβ1-binding molecule comprising a heavy chain variable region (VH) and a light chain variable region (VL).

In some embodiments, the VH comprises a HCDR1, a HCDR2, and a HCDR3 from the amino acid sequence set forth in SEQ ID NO: 11, and/or the VL comprises a LCDR1, a LCDR2, and a LCDR3 from the amino acid sequence set forth in any one of SEQ ID NOs: 12 and 21-35.

The above CDRs are defined according to the Kabat, IMGT, Chothia, AbM, or Contact numbering scheme. In some specific embodiments, the CDRs are defined according to the Kabat definition scheme.

In some embodiments, the CDRs may also be defined by other numbering schemes, e.g., according to the IMGT, Chothia, AbM, or Contact numbering scheme. Illustratively, the HCDR1, HCDR2, and HCDR3 in a VH set forth in SEQ ID NO: 11 and the LCDR1, LCDR2, and LCDR3 in a VL set forth in SEQ ID NO: 35, defined according to the Kabat, IMGT, Chothia, AbM, or Contact numbering scheme, are provided below.

**Table 1. The HCDR1, HCDR2, and HCDR3 in a VH set forth in SEQ ID NO: 11**

| | HCDR1 | HCDR2 | HCDR3 |
|---|---|---|---|
| IMGT | GFTFSSYA (SEQ ID NO:113) | ISGSGGST (SEQ ID NO:117) | ARVDSSGGYWYFDL (SEQ ID NO:121) |
| Chothia | GFTFSSY (SEQ ID NO:114) | SGSGGS (SEQ ID NO:118) | VDSSGGYWYFDL (SEQ ID NO:15) |
| AbM | GFTFSSYAMS (SEQ ID NO:115) | AISGSGGSTY (SEQ ID NO:119) | VDSSGGYWYFDL (SEQ ID NO:15) |
| Contact | SSYAMS (SEQ ID NO:116) | WVSAISGSGGSTY (SEQ ID NO:120) | ARVDSSGGYWYFD (SEQ ID NO:122) |
| Kabat | SYAMS (SEQ ID NO:13) | AISGSGGSTYYADSVKG (SEQ ID NO:14) | VDSSGGYWYFDL (SEQ ID NO:15) |

**Table 2. The LCDR1, LCDR2, and LCDR3 in a VL set forth in SEQ ID NO: 35**

| | LCDR1 | LCDR2 | LCDR3 |
|---|---|---|---|
| IMGT | QSISDY (SEQ ID NO:123) | TA | QQSYSTPLT (SEQ ID NO:18) |
| Chothia | RASQSISDYLN (SEQ ID NO:59) | TASYLDS (SEQ ID NO:40) | QQSYSTPLT (SEQ ID NO:18) |
| AbM | RASQSISDYLN (SEQ ID NO:59) | TASYLDS (SEQ ID NO:40) | QQSYSTPLT (SEQ ID NO:18) |
| Contact | SDYLNWY (SEQ ID NO:124) | LLIYTASYLD (SEQ ID NO:125) | QQSYSTPL (SEQ ID NO:126) |
| Kabat | RASQSISDYLN (SEQ ID NO:59) | TASYLDS (SEQ ID NO:40) | QQSYSTPLT (SEQ ID NO:18) |

In some embodiments, the TGFβ1-binding molecule comprises a VH and a VL as shown below:
the VH comprises a HCDR1, a HCDR2, and a HCDR3 set forth in SEQ ID NOs: 13-15, and/or the VL comprises a LCDR1, a LCDR2, and a LCDR3 set forth in SEQ ID NOs: 38, 64, and 18,
wherein the amino acid sequence set forth in SEQ ID NO: 38 is RASQX₁ISX₂YLN, wherein X₁ is selected from the group consisting of S, A, F, G, I, P, Y, V, and K, and X₂ is selected from the group consisting of S, D, E, P, and H;
the amino acid sequence set forth in SEQ ID NO: 64 is X₃ASX₄LX₅S, wherein X₃ is selected from the group consisting of A, T, S, and M, X₄ is selected from the group consisting of S, Y, A, E, and G, and X₅ is selected from the group consisting of Q, T, D, and E.

In some embodiments, the amino acid sequence set forth in SEQ ID NO: 38 is RASQX₁ISX₂YLN, wherein X₁ is selected from the group consisting of F, Y, and A, and X₂ is selected from the group consisting of D and P; the amino acid sequence set forth in SEQ ID NO: 64 is X₃ASX₄LX₅S, wherein X₃ is selected from the group consisting of A, T, and S, X₄ is selected from the group consisting of S, Y, and E, and X₅ is selected from the group consisting of Q, D, and E.

In some embodiments, provided is the TGFβ1-binding molecule, wherein:
the amino acid sequence of the HCDR1 is set forth in SEQ ID NO: 13,
the amino acid sequence of the HCDR2 is set forth in SEQ ID NO: 14,
the amino acid sequence of the HCDR3 is set forth in SEQ ID NO: 15,
the amino acid sequence of the LCDR1 is set forth in any one of SEQ ID NOs: 16, 36, 39, 41-42, 44, 46, 49, 52, 54, and 56-59,
the amino acid sequence of the LCDR2 is set forth in any one of SEQ ID NOs: 17, 37, 40, 43, 45, 47-48, 50-51, 53, and 55, and/or,
the amino acid sequence of the LCDR3 is set forth in SEQ ID NO: 18.

In some embodiments, provided is the TGFβ1-binding molecule, wherein:
a) the VH comprises a HCDR1, a HCDR2, and a HCDR3 set forth in SEQ ID NOs: 13-15, and the VL comprises a LCDR1, a LCDR2, and a LCDR3 set forth in SEQ ID NOs: 59, 40, and 18;
b) the VH comprises a HCDR1, a HCDR2, and a HCDR3 set forth in SEQ ID NOs: 13-15, and the VL comprises a LCDR1, a LCDR2, and a LCDR3 set forth in SEQ ID NOs: 36-37 and 18;
c) the VH comprises a HCDR1, a HCDR2, and a HCDR3 set forth in SEQ ID NOs: 13-15, and the VL comprises a LCDR1, a LCDR2, and a LCDR3 set forth in SEQ ID NOs: 39-40 and 18;
d) the VH comprises a HCDR1, a HCDR2, and a HCDR3 set forth in SEQ ID NOs: 13-15, and the VL comprises a LCDR1, a LCDR2, and a LCDR3 set forth in SEQ ID NOs: 41, 40, and 18;
e) the VH comprises a HCDR1, a HCDR2, and a HCDR3 set forth in SEQ ID NOs: 13-15, and the VL comprises a LCDR1, a LCDR2, and a LCDR3 set forth in SEQ ID NOs: 42-43 and 18;
f) the VH comprises a HCDR1, a HCDR2, and a HCDR3 set forth in SEQ ID NOs: 13-15, and the VL comprises a LCDR1, a LCDR2, and a LCDR3 set forth in SEQ ID NOs: 44-45 and 18;
g) the VH comprises a HCDR1, a HCDR2, and a HCDR3 set forth in SEQ ID NOs: 13-15, and the VL comprises a LCDR1, a LCDR2, and a LCDR3 set forth in SEQ ID NOs: 46-47 and 18;
h) the VH comprises a HCDR1, a HCDR2, and a HCDR3 set forth in SEQ ID NOs: 13-15, and the VL comprises a LCDR1, a LCDR2, and a LCDR3 set forth in SEQ ID NOs: 46, 48, and 18;
j) the VH comprises a HCDR1, a HCDR2, and a HCDR3 set forth in SEQ ID NOs: 13-15, and the VL comprises a LCDR1, a LCDR2, and a LCDR3 set forth in SEQ ID NOs: 49-50 and 18;
k) the VH comprises a HCDR1, a HCDR2, and a HCDR3 set forth in SEQ ID NOs: 13-15, and the VL comprises a LCDR1, a LCDR2, and a LCDR3 set forth in SEQ ID NOs: 46, 51, and 18;
l) the VH comprises a HCDR1, a HCDR2, and a HCDR3 set forth in SEQ ID NOs: 13-15, and the VL comprises a LCDR1, a LCDR2, and a LCDR3 set forth in SEQ ID NOs: 52-53 and 18;
m) the VH comprises a HCDR1, a HCDR2, and a HCDR3 set forth in SEQ ID NOs: 13-15, and the VL comprises a LCDR1, a LCDR2, and a LCDR3 set forth in SEQ ID NOs: 54-55 and 18;
n) the VH comprises a HCDR1, a HCDR2, and a HCDR3 set forth in SEQ ID NOs: 13-15, and the VL comprises a LCDR1, a LCDR2, and a LCDR3 set forth in SEQ ID NOs: 56, 53, and 18;
o) the VH comprises a HCDR1, a HCDR2, and a HCDR3 set forth in SEQ ID NOs: 13-15, and the VL comprises a LCDR1, a LCDR2, and a LCDR3 set forth in SEQ ID NOs: 57, 45, and 18;
p) the VH comprises a HCDR1, a HCDR2, and a HCDR3 set forth in SEQ ID NOs: 13-15, and the VL comprises a LCDR1, a LCDR2, and a LCDR3 set forth in SEQ ID NOs: 58, 50, and 18; or,
q) the VH comprises a HCDR1, a HCDR2, and a HCDR3 set forth in SEQ ID NOs: 13-15, and the VL comprises a LCDR1, a LCDR2, and a LCDR3 set forth in SEQ ID NOs: 16-18.

In some embodiments, the TGFβ1-binding molecule comprises a VH and a VL, wherein:
any one of the HCDRs that the VH comprises comprises 0, 1, 2, 3, 4, or 5 amino acid mutations compared to any one of the aforementioned HCDRs, and/or any one of the LCDRs that the VL comprises comprises 0, 1, 2, 3, 4, or 5 amino acid mutations compared to any one of the aforementioned LCDRs.

In some specific embodiments, the amino acid mutations in the HCDRs or LCDRs are conservative substitutions.

In some embodiments, the TGFβ1-binding molecule provided in the present disclosure comprises any one or a combination of any several (e.g., 2, 3, 4, 5, or 6) of the aforementioned HCDR1, HCDR2, HCDR3, LCDR1, LCDR2, and LCDR3.

In some embodiments, the TGFβ1-binding molecule is an anti-TGFβ1 antibody or an antigen-binding fragment thereof.

In some embodiments, the anti-TGFβ1 antibody or the antigen-binding fragment thereof is a murine antibody, a chimeric antibody, a humanized antibody, a human-derived antibody, or an antigen-binding fragment of any one of the foregoing.

In some specific embodiments, the anti-TGFβ1 antibody or the antigen-binding fragment thereof is a humanized antibody or an antigen-binding fragment thereof, or a human-derived antibody or an antigen-binding fragment thereof. In some specific embodiments, the anti-TGFβ1 antibody or the antigen-binding fragment thereof is a human-derived antibody or an antigen-binding fragment thereof.

In some specific embodiments, the anti-TGFβ1 antibody or the antigen-binding fragment thereof is engineered by affinity maturation. The anti-TGFβ1 antibody or the antigen-binding fragment thereof engineered by affinity maturation comprises one or more mutations in the VH and/or the VL. Illustratively, the mutations in the VH comprise a mutation at any one or any combination of the following positions: 99, 100, 53, 56, 58, 31, 33, 95, and 100. Illustratively, the mutations in the VL comprise a mutation at any one or any combination of the following positions: 24, 28, 31, 32, 50, 53, 55, 92, 93, and 94. The positions of the above mutations are numbered according to the Kabat numbering scheme.

In some embodiments, the TGFβ1-binding molecule comprises a VH and a VL, wherein:
the VH comprises the amino acid sequence set forth in SEQ ID NO: 11 or an amino acid sequence having at least 80% sequence identity thereto; and/or,
the VL comprises the amino acid sequence set forth in any one of SEQ ID NOs: 12 and 21-35 or an amino acid sequence having at least 80% sequence identity thereto.

In some embodiments, the TGFβ1-binding molecule comprises a VH and a VL, wherein: the VH comprises 0, 1, 2, 3, 4, 5, 6, 7, 8, 9, or 10 amino acid mutations compared to any one of the aforementioned VHs, and/or the VL comprises 0, 1, 2, 3, 4, 5, 6, 7, 8, 9, or 10 amino acid mutations compared to any one of the aforementioned VLs.

In some embodiments, the TGFβ1-binding molecule provided in the present disclosure comprises any one or a combination of any two of the aforementioned VH and VL.

In some embodiments, the TGFβ1-binding molecule provided in the present disclosure further comprises an immunoglobulin Fc region. In some embodiments, the immunoglobulin Fc region is derived from IgG1, IgG2, IgG3, IgG4, or a variant of any one of the foregoing. In some embodiments, the immunoglobulin Fc region is derived from human IgG4 or a variant thereof. In some embodiments, the human IgG4 variant comprises a mutation that reduces or eliminates an Fc effector function. Illustratively, the human IgG4 variant comprises mutation S228P. The sites of the above mutations are numbered according to the EU numbering scheme.

In some embodiments, the TGFβ1-binding molecule further comprises a light chain constant region and/or a heavy chain constant region.

In some embodiments, the light chain constant region is derived from a κ light chain, a λ light chain, or a variant of any one of the foregoing. In some embodiments, the light chain constant region is derived from a human κ light chain, a human λ light chain, or a variant of any one of the foregoing.

In some specific embodiments, the light chain constant region is derived from a human κ light chain or a variant thereof.

In some specific embodiments, the light chain constant region is derived from a murine κ light chain or a variant thereof.

In some specific embodiments, the light chain constant region comprises the amino acid sequence set forth in SEQ ID NO: 19 or an amino acid sequence having at least 80% sequence identity thereto.

In some embodiments, the heavy chain constant region is derived from IgG1, IgG2, IgG3, IgG4, or a variant of any one of the foregoing. In some embodiments, the heavy chain constant region is derived from human IgG1, human IgG2, human IgG3, human IgG4, or a variant of any one of the foregoing.

In some specific embodiments, the heavy chain constant region is derived from human IgG4 or a variant thereof.

In some specific embodiments, the human IgG4 variant comprises a mutation that reduces or eliminates an Fc effector function. Illustratively, the human IgG4 variant comprises mutation S228P. The sites of the above mutations are numbered according to the EU numbering scheme.

In some specific embodiments, the heavy chain constant region is derived from murine IgG2 or a variant thereof.

In some specific embodiments, the murine IgG2 variant comprises a mutation that reduces or eliminates an ADCC effect. Illustratively, the murine IgG2 variant comprises at least one of the following mutations: L234A/L235E/G237A/D327Q/A330S/P331S. The sites of the above mutations are numbered according to the EU numbering scheme.

In some embodiments, the immunoglobulin Fc region is an Fc region with a reduced or eliminated effector function, for example, comprising a mutation that reduces or eliminates an ADCC effect. Illustratively, the mutation that reduces or eliminates the ADCC effect comprises at least one of the following:
L234A/L235E/G237A/D327Q/A330S/P331S (IgG2a); N297A or N297Q (IgG1); L234A/L235A (IgG1); V234A/G237A (IgG2); L235A/G237A/E318A (IgG4); H268Q/V309L/A330S/A331S (IgG2); C220S/C226S/C229S/P238S (IgG1); C226S/C229S/E233P/L234V/L235A (IgG1); L234F/L235E/P331S (IgG1); or S267E/L328F (IgG1). The positions of the above mutations are numbered according to the EU numbering scheme.

In some specific embodiments, the heavy chain constant region comprises the amino acid sequence set forth in SEQ ID NO: 20 or an amino acid sequence having at least 80% sequence identity thereto.

In some embodiments, the TGFβ1-binding molecule comprises a heavy chain and a light chain, wherein:
the heavy chain comprises the amino acid sequence set forth in SEQ ID NO: 66 or an amino acid sequence having at least 80% sequence identity thereto; and/or,
the light chain comprises the amino acid sequence set forth in any one of SEQ ID NOs: 65 and 67-81 or an amino acid sequence having at least 80% sequence identity thereto.

In some embodiments, the TGFβ1-binding molecule comprises a heavy chain and a light chain, wherein:
the heavy chain comprises the amino acid sequence set forth in SEQ ID NO: 82 or an amino acid sequence having at least 80% sequence identity thereto; and/or,
the light chain comprises the amino acid sequence set forth in SEQ ID NO: 83 or an amino acid sequence having at least 80% sequence identity thereto.

In some embodiments, the TGFβ1-binding molecule comprises a heavy chain and a light chain, wherein: the heavy chain comprises 0, 1, 2, 3, 4, 5, 6, 7, 8, 9, or 10 amino acid mutations compared to any one of the aforementioned heavy chains, and/or the light chain comprises 0, 1, 2, 3, 4, 5, 6, 7, 8, 9, or 10 amino acid mutations compared to any one of the aforementioned light chains.

In some specific embodiments, the amino acid mutations in the heavy chain and/or the light chain are conservative substitutions.

In some embodiments, the TGFβ1-binding molecule of the present disclosure comprises any one or a combination of any two of the aforementioned heavy and light chains.

In the context of the present disclosure, "at least 80%" encompasses 80% or more, e.g., at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, and a numerical range between any two of these numbers.

In some embodiments, the aforementioned TGFβ1-binding molecule specifically binds to a TGFβ1 precursor protein or a TGFβ1 complex.

In some embodiments, the TGFβ1 in the TGFβ1 complex is present as a TGFβ1 precursor protein.

In some embodiments, the TGFβ1 complex comprises any one of the following:
(2) an LTBP1-TGFβ1 complex,
(3) an LTBP3-TGFβ1 complex,
(4) an LRRC33-TGFβ1 complex, and
(5) a GARP-TGFβ1 complex.

As used herein, "TGFβ1" or "TGFβ1 protein" is understood in a broad sense and comprises the pro-protein form (also known as pro-TGFβ1) or latent form (latent TGFβ1) of the transforming growth factor-β1 (TGFβ1) protein, or mature TGFβ1 after release.

In some embodiments, "TGFβ1" or "TGFβ1 protein" as used herein refers to "TGFβ1 precursor protein".

In some embodiments, the TGFβ1 precursor protein comprises: (i) a mature TGFβ1 domain and (ii) a latency-associated peptide (LAP).

Illustratively, the TGFβ1 precursor protein is pro-TGFβ1 or latent TGFβ1. The mature TGFβ1 domain in pro-TGFβ1 covalently binds to latency-associated peptides (LAPs), and the mature TGFβ1 domain in latent TGFβ1 non-covalently binds to latency-associated peptides (LAPs).

In the present disclosure, "pro-TGFβ1" or "latent TGFβ1" is used interchangeably.

During translation, latent TGFβ1 (also known as a small latent complex (SLC)) becomes linked to a "presenting molecule" by a disulfide bridge, thereby forming a large latent complex (LLC), including, for example, forming a GARP-TGFβ1 complex with GARP. The TGFβ1 present in the GARP-TGFβ1 complex may be in a latent form (latent TGFβ1) or in a precursor form (pro-TGFβ1).

In some embodiments, the TGFβ1 precursor protein or TGFβ1 complex further comprises a protein fragment, a functional variant, etc. of any of the aforementioned proteins or protein complexes.

Illustratively, "fragment" or "protein fragment" includes, but is not limited to: growth factor domains, N-terminal prodomains, latency-associated peptides (LAPs), LAP-like domains, straight jacket regions, fastener regions, furin cleavage site regions, arm regions, fingers regions, latency loops, α₁ helical regions (alpha 1 helical regions), α₂ helical regions (alpha 2 helical regions), RGD sequence regions, trigger loop regions, extracellular domains, transmembrane domains, cytoplasmic domains, etc.

Illustratively, "variant" or "functional variant" refers to a protein or protein complex comprising substitution, deletion, and/or addition of one or more amino acids and having comparable bioactivity.

Illustratively, the variant of the TGFβ1 precursor protein comprises an amino acid mutation at a position naturally numbered 4 relative to the amino acid sequence set forth in SEQ ID NO: 6. In some embodiments, the variant of the TGFβ1 precursor protein comprises mutation C4S, wherein the site of the mutation is a site naturally numbered relative to the amino acid sequence set forth in SEQ ID NO: 6.

In some embodiments, the TGFβ1 complex is a complex formed by the TGFβ1 precursor protein with other types of proteins or protein fragments or functional variants thereof, e.g., a complex formed with LTBP1S, LTBP4, fibrillin-1, fibrillin-2, fibrillin-3, fibrillin-4, etc.

In some embodiments, the TGFβ1 precursor protein comprises the amino acid sequence set forth in any one of SEQ ID NOs: 1, 3, 6-7, and 9-10 or an amino acid sequence having at least 80% sequence identity thereto.

Illustratively, the TGFβ1 complex is a GARP-TGFβ1 complex comprising GARP and a TGFβ1 precursor protein, wherein:
GARP comprises the amino acid sequence set forth in any one of SEQ ID NOs: 2, 4, 5, 8, 100, and 103 or an amino acid sequence having at least 80% sequence identity thereto;
and/or the TGFβ1 precursor protein comprises the amino acid sequence set forth in any one of SEQ ID NOs: 1, 3, 6-7, and 9-10 or an amino acid sequence having at least 80% sequence identity thereto.

In some embodiments, the TGFβ1-binding molecule is an anti-TGFβ1 antibody or an antigen-binding fragment thereof that specifically binds to an antigenic epitope of the TGFβ1 precursor protein or the TGFβ1 complex. In some embodiments, the anti-TGFβ1 antibody or the antigen-binding fragment thereof does not prevent TGFβ1 from binding to integrin. For example, in some embodiments, the anti-TGFβ1 antibody or the antigen-binding fragment thereof does not mask the integrin binding site of TGFβ1.

In some embodiments, the TGFβ1-binding molecule binds to a TGFβ1 precursor protein or a TGFβ1 complex (e.g., an LTBP1-TGFβ1 complex, an LTBP3-TGFβ1 complex, an LRRC33-TGFβ1 complex, or a GARP-TGFβ1 complex) with a K_{D} of 10⁻⁷ M, 10⁻⁸ M, 10⁻⁹ M, 10⁻¹⁰ M, or less. In some embodiments, the TGFβ1 complex comprises the TGFβ1 precursor protein.

In some embodiments, the TGFβ1-binding molecule does not bind or binds extremely weakly to a TGFβ2 protein or a TGFβ2 complex. Illustratively, the anti-TGFβ1 antibody or the antigen-binding fragment thereof does not specifically bind or binds extremely weakly to at least one of the following: mature TGFβ2, a TGFβ2 precursor protein, a GARP-TGFβ2 complex, an LTBP-TGFβ2 complex, etc.

In some embodiments, the TGFβ1-binding molecule does not bind or binds extremely weakly to a TGFβ3 protein or a TGFβ3 complex. Illustratively, the anti-TGFβ1 antibody or the antigen-binding fragment thereof does not specifically bind or binds extremely weakly to at least one of the following: mature TGFβ3, a TGFβ3 precursor protein, a GARP-TGFβ3 complex, an LTBP-TGFβ3 complex, etc.

In the present disclosure, "TGFβ2 protein" should be understood in a broad sense and encompasses mature TGFβ2, as well as inactive precursor protein forms such as pro-TGFβ2 and latent TGFβ2. "Pro-TGFβ2" and "latent TGFβ2" are used interchangeably.

In the present disclosure, "TGFβ3 protein" should be understood in a broad sense and encompasses mature TGFβ3, as well as inactive precursor protein forms such as pro-TGFβ3 and latent TGFβ3. "Pro-TGFβ3" and "latent TGFβ3" are used interchangeably.

In some embodiments, the TGFβ1-binding molecule of the present disclosure inhibits TGFβ1 activity. For example, the TGFβ1-binding molecule binds to a TGFβ1 precursor protein (pro/latent TGFβ1) or a TGFβ1 complex (e.g., a GARP-TGFβ1 complex, an LTBP1-TGFβ1 complex, an LTBP3-TGFβ1 complex, or an LRRC33-TGFβ1 complex), thereby selectively inhibiting the activity of TGFβ1 (e.g., inhibiting the activation of TGFβ1, and/or inhibiting the release of mature TGFβ1; and/or inhibiting TGFβ1 signal transduction). Moreover, the activity of TGFβ2 and/or TGFβ3 is not selectively inhibited; for example, the activation of TGFβ1 is selectively inhibited, but the activation of TGFβ2 and/or TGFβ3 is not inhibited. The TGFβ1 in the TGFβ1 complex is present in the form of a TGFβ1 precursor protein.

In some embodiments, the TGFβ1-binding molecule of the present disclosure has improved safety (e.g., reduced *in vivo* toxicity and/or adverse reactions).

In some embodiments, the TGFβ1-binding molecule of the present disclosure further has at least one of the following properties: inhibiting the immunosuppressive activity of regulatory T (T_{reg}) cells, inhibiting tumor growth, and inhibiting fibrosis.

In some embodiments, the TGFβ1-binding molecule is an anti-TGFβ1 antibody or an antigen-binding fragment thereof and is further a Fab, an Fv, an sFv, a Fab', a F(ab')₂, a linear antibody, a single-chain antibody, an scFv, an sdAb, an sdFv, a nanobody, a peptibody, a domain antibody, and a multispecific antibody (a bispecific antibody, a diabody, a triabody, and a tetrabody, a tandem di-scFv, and a tandem tri-scFv), for example, specifically, an scFv, Fv, Fab, or Fab' fragment.

In some embodiments, provided is an anti-TGFβ1 antibody or an antigen-binding fragment thereof that binds to or competes with the aforementioned TGFβ1-binding molecule for binding to the same epitope of a TGFβ1 precursor protein or a TGFβ1 complex (e.g., a human or murine GARP-TGFβ1 complex, an LTBP-TGFβ1 complex, or an LRRC33-TGFβ1 complex). In some embodiments, the TGFβ1 in the TGFβ1 complex is present in the form of a TGFβ1 precursor protein.

In some embodiments, provided is an anti-TGFβ1 antibody or an antigen-binding fragment thereof that blocks the binding of the aforementioned TGFβ1-binding molecule to a TGFβ1 precursor protein or a TGFβ1 complex (e.g., a human or murine GARP-TGFβ1 complex, an LTBP-TGFβ1 complex, or an LRRC33-TGFβ1 complex). In some embodiments, the TGFβ1 in the TGFβ1 complex is present in the form of a TGFβ1 precursor protein.

In some embodiments, provided is an anti-TGFβ1 antibody or an antigen-binding fragment thereof whose binding to a TGFβ1 protein or a TGFβ1 complex (e.g., a human or murine GARP-TGFβ1 complex, an LTBP-TGFβ1 complex, or an LRRC33-TGFβ1 complex) is blocked by the aforementioned TGFβ1-binding molecule. In some embodiments, the TGFβ1 in the TGFβ1 complex is present in the form of a TGFβ1 precursor protein.

### GARP-TGFβ1-Binding Molecule

In a second aspect, the present disclosure provides a GARP-TGFβ1-binding molecule comprising at least one immunoglobulin single variable domain that binds to a TGFβ1 complex.

In some embodiments, the TGFβ1 complex is a GARP-TGFβ1 complex. In some embodiments, the TGFβ1 in the GARP-TGFβ1 complex is a TGFβ1 precursor protein. Illustratively, the TGFβ1 precursor protein is selected from the group consisting of pro-TGFβ1 and latent TGFβ1. In the present disclosure, "pro-TGFβ1" and "latent TGFβ1" are used interchangeably.

In some embodiments, the present disclosure provides a GARP-TGFβ1-binding molecule comprising at least one immunoglobulin single variable domain that binds to a GARP-TGFβ1 complex.

In some embodiments, the immunoglobulin single variable domain comprises three complementarity-determining regions: CDR1, CDR2, and CDR3, wherein:
a) the immunoglobulin single variable domain comprises a CDR1, a CDR2, and a CDR3 from the amino acid sequence set forth in SEQ ID NO: 84;
b) the immunoglobulin single variable domain comprises a CDR1, a CDR2, and a CDR3 from the amino acid sequence set forth in SEQ ID NO: 90;
c) the immunoglobulin single variable domain comprises a CDR1, a CDR2, and a CDR3 from the amino acid sequence set forth in SEQ ID NO: 91; or,
d) the immunoglobulin single variable domain comprises a CDR1, a CDR2, and a CDR3 from the amino acid sequence set forth in SEQ ID NO: 92.

The above CDRs are defined according to the Kabat, IMGT, Chothia, AbM, or Contact numbering scheme. In some specific embodiments, the CDRs are defined according to the Kabat numbering scheme. Illustratively, the CDR1, CDR2, and CDR3 defined by the Kabat, IMGT, Chothia, AbM, or Contact numbering scheme are provided below.

**Table 3. The CDR1, CDR2, and CDR3 in the sequence set forth in SEQ ID NO: 84**

| | CDR1 | CDR2 | CDR3 |
|---|---|---|---|
| IMGT | GFTYCEYD (SEQ ID NO:96) | IASDGRT (SEQ ID NO:107) | KTEAVKYSGNWCVAAPGFAY (SEQ ID NO:111) |
| Chothia | GFTYCEY (SEQ ID NO:97) | ASDGR (SEQ ID NO:108) | EAVKYSGNWCVAAPGFAY (SEQ ID NO:87) |
| AbM | GFTYCEYDMS (SEQ ID NO:98) | RIASDGRTS (SEQ ID NO:109) | EAVKYSGNWCVAAPGFAY (SEQ ID NO:87) |
| Contact | CEYDMS (SEQ ID NO:99) | FVSRIASDGRTS (SEQ ID NO:110) | KTEAVKYSGNWCVAAPGFA (SEQ ID NO:112) |
| Kabat | EYDMS (SEQ ID NO:85) | RIASDGRTSYVDSVKG (SEQ ID NO:86) | EAVKYSGNWCVAAPGFAY (SEQ ID NO:87) |

In some embodiments, the immunoglobulin single variable domain comprises three complementarity-determining regions: CDR1, CDR2, and CDR3, wherein:
the CDR1 comprises the amino acid sequence set forth in SEQ ID NO: 85;
the CDR2 comprises the amino acid sequence set forth in SEQ ID NO: 86; and/or
the CDR3 comprises the amino acid sequence set forth in SEQ ID NO: 87.

In some embodiments, the immunoglobulin single variable domain comprises a CDR1, a CDR2, and a CDR3 set forth in SEQ ID NOs: 85-87.

In some embodiments, the CDR1 that the immunoglobulin single variable domain comprises comprises 0, 1, 2, 3, 4, or 5 amino acid mutations compared to the amino acid sequence set forth in SEQ ID NO: 85; and/or,
the CDR2 that the immunoglobulin single variable domain comprises comprises 0, 1, 2, 3, 4, or 5 amino acid mutations compared to the amino acid sequence set forth in SEQ ID NO: 86; and/or,
the CDR3 that the immunoglobulin single variable domain comprises comprises 0, 1, 2, 3, 4, or 5 amino acid mutations compared to the amino acid sequence set forth in SEQ ID NO: 87.

In some specific embodiments, the amino acid mutations in the CDR1, CDR2, or CDR3 are conservative substitutions.

In some embodiments, the GARP-TGFβ1-binding molecule comprises any one or more (e.g., 1, 2, 3, 4, 5, 6, 7, 8, 9, 10, 20, 30, or 50) of the aforementioned immunoglobulin single variable domains. In some embodiments, the GARP-TGFβ1-binding molecule comprises two or more immunoglobulin single variable domains, and any two of the immunoglobulin single variable domains may be identical or different.

In some embodiments, the GARP-TGFβ1-binding molecule may comprise any one of the aforementioned intact immunoglobulin single variable domains; or a functional portion of any one of the aforementioned immunoglobulin single variable domains, or a variant thereof, such as CDR3, CDR3-FR4, CDR2-FR3-CDR3, CDR2-FR3-CDR3-FR4, FR2-CDR2-FR3-CDR3-FR4, CDR1-FR2-CDR2-FR3-CDR3-FR4, or FR1-CDR1-FR2-CDR2-FR3-CDR3.

In some embodiments, the variant of the functional portion of the immunoglobulin single variable domain may be a polypeptide that retains the serum albumin-binding function of CDR3, CDR3-FR4, CDR2-FR3-CDR3, CDR2-FR3-CDR3-FR4, FR2-CDR2-FR3-CDR3-FR4, CDR1-FR2-CDR2-FR3-CDR3-FR4, or FR1-CDR1-FR2-CDR2-FR3-CDR3 and has at least 80% or at least 90% sequence homology therewith, for example, a polypeptide that retains the TGFβ1 complex-binding function of CDR3 and has a certain level of sequence homology therewith, such as a polypeptide having at least 80% or at least 90% sequence homology with any one of the above CDR3s.

In some embodiments, the immunoglobulin single variable domain is a VHH. Illustratively, the immunoglobulin single variable domain is a camelid VHH, a fully human VHH, or a humanized VHH.

In some specific embodiments, the immunoglobulin single variable domain is a humanized VHH.

In some embodiments, a human germline template of the humanized VHH is at least one template selected from the group consisting of IGHV3-23 and IGJH4. In some specific embodiments, the humanized VHH comprises a FR1, a FR2, and a FR3 derived from IGHV3-23, and the humanized VHH comprises a FR4 derived from IGJH4.

In some embodiments, the humanized VHH comprises at least one of the following back mutations: 23T, 29Y, 30C, 37Y, 44E, 45R, 47F, 71Q, 74A, 75R, 78G, 81E, 93K, and 94T. In some specific embodiments, a framework region of the humanized VHH comprises at least one of the above back mutations.

The sites of the above back mutations are numbered according to the Kabat numbering scheme.

In some embodiments, the immunoglobulin single variable domain comprises the amino acid sequence set forth in any one of SEQ ID NOs: 84 and 90-92 or an amino acid sequence having at least 80% sequence identity thereto.

In some embodiments, the immunoglobulin single variable domain comprises an amino acid sequence having one or more (e.g., 2, 3, 4, 5, 6, 7, 8, 9, or 10) amino acid mutations compared to the sequence set forth in any one of SEQ ID NOs: 84 and 90-92.

In some specific embodiments, the amino acid mutations in the immunoglobulin single variable domain are conservative substitutions.

In some embodiments, the GARP-TGFβ1-binding molecule further comprises an immunoglobulin Fc region. Illustratively, the immunoglobulin Fc region is derived from IgG1, IgG2, IgG3, or IgG4. In some embodiments, the immunoglobulin Fc region is derived from IgG2 or IgG4. In some specific embodiments, the immunoglobulin Fc region is derived from human IgG4.

In some embodiments, the immunoglobulin Fc region comprises a mutation that reduces or eliminates an Fc effector function. Illustratively, the immunoglobulin Fc region comprises a mutation: S228P. The positions of the above mutations are numbered according to the EU numbering scheme.

In some specific embodiments, the immunoglobulin Fc region comprises the amino acid sequence set forth in SEQ ID NO: 88 or an amino acid sequence having at least 80% sequence identity thereto.

In some embodiments, the immunoglobulin Fc region is derived from mouse IgG2.

In some embodiments, the immunoglobulin Fc region is an Fc region with a reduced or eliminated effector function, for example, comprising a mutation that reduces or eliminates an ADCC effect. Illustratively, the mutation that reduces or eliminates the ADCC effect comprises at least one of the following: L234A/L235E/G237A/D327Q/A330S/P331S (IgG2a); N297A or N297Q (IgG1); L234A/L235A (IgG1); V234A/G237A (IgG2); L235A/G237A/E318A (IgG4); H268Q/V309L/A330S/A331S (IgG2); C220S/C226S/C229S/P238S (IgG1); C226S/C229S/E233P/L234V/L235A (IgG1); L234F/L235E/P331S (IgG1); or S267E/L328F (IgG1). The positions of the above mutations are numbered according to the EU numbering scheme.

In some embodiments, the GARP-TGFβ1-binding molecule is an antibody or an antigen-binding fragment thereof that binds to a GARP-TGFβ1 complex.

In some embodiments, the GARP-TGFβ1-binding molecule comprises the amino acid sequence set forth in any one of SEQ ID NOs: 89, 93-95, and 106 or an amino acid sequence having at least 80% or at least 90% sequence identity thereto.

In some embodiments, the GARP-TGFβ1-binding molecule comprises an amino acid sequence comprising one or more (e.g., 0, 1, 2, 3, 4, 5, 6, 7, 8, 9, or 10) amino acid mutations compared to the amino acid sequence set forth in any one of SEQ ID NOs: 89, 93-95, and 106 and having the functional activity of specifically binding to a GARP-TGFβ1 complex.

In some specific embodiments, the amino acid mutations in the GARP-TGFβ1-binding molecule are conservative substitutions.

In the context of the present disclosure, "at least 80%" encompasses 80% or more, e.g., at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99%, and a numerical range between any two of these numbers.

In some embodiments, the aforementioned GARP-TGFβ1-binding molecule specifically binds to a GARP-TGFβ1 complex, thereby inhibiting the release of mature TGFβ1 from the GARP-TGFβ1 complex, and/or inhibiting TGFβ1 activity, and/or inhibiting TGFβ1 signal transduction.

In some embodiments, the GARP-TGFβ1-binding molecule does not bind to free mature TGFβ1.

In some embodiments, the GARP-TGFβ1-binding molecule does not bind or binds extremely weakly to a TGFβ2 protein or a TGFβ2 complex. For example, the TGFβ1-binding molecule does not bind or binds extremely weakly to mature TGFβ2, a TGFβ2 precursor protein, a GARP-TGFβ2 complex, an LTBP-TGFβ2 complex, etc.

In some embodiments, the GARP-TGFβ1-binding molecule does not bind or binds extremely weakly to a TGFβ3 protein or a TGFβ3 complex. For example, the GARP-TGFβ1-binding molecule does not bind or binds extremely weakly to mature TGFβ3, a TGFβ3 precursor protein, a GARP-TGFβ3 complex, an LTBP-TGFβ3 complex, etc.

In the present disclosure, "TGFβ2 protein" should be understood in a broad sense and encompasses mature TGFβ2, as well as inactive precursor protein forms such as pro-TGFβ2 and latent TGFβ2. "TGFβ3 protein" should be understood in a broad sense and encompasses mature TGFβ3, as well as inactive precursor protein forms such as pro-TGFβ3 and latent TGFβ3.

In some embodiments, the aforementioned GARP-TGFβ1-binding molecule is capable of selectively inhibiting TGFβ1 activity but does not inhibit the activity of TGFβ2 and/or TGFβ3. For example, the GARP-TGFβ1-binding molecule selectively inhibits the activation of TGFβ1 but does not inhibit the activation of TGFβ2 and/or TGFβ3. Thus, the GARP-TGFβ1-binding molecule of the present disclosure has improved safety (e.g., reduced *in vivo* toxicity and/or adverse reactions).

In some embodiments of the present disclosure, mature TGFβ1, mature TGFβ2, and/or mature TGFβ3 are present in a free state in a cell.

In some embodiments, the GARP-TGFβ1-binding molecule specifically binds to a GARP-TGFβ1 complex, wherein the GARP-TGFβ1 complex comprises:
a) glycoprotein-A repetitions predominant (GARP);
b) a mature TGFβ1 domain; and,
c) a latency-associated peptide (LAP).

In some embodiments, the GARP comprises the amino acid sequence set forth in SEQ ID NO: 100 or 103 or an amino acid sequence having at least 90% sequence identity thereto, the mature TGFβ1 domain comprises the amino acid sequence set forth in SEQ ID NO: 101 or 104 or an amino acid sequence having at least 90% sequence identity thereto, and/or the LAP comprises the amino acid sequence set forth in SEQ ID NO: 102 or 105 or an amino acid sequence having at least 90% sequence identity thereto.

In some embodiments, the GARP-TGFβ1-binding molecule binds to the GARP-TGFβ1 complex or a fragment thereof with a K_{D} of 10⁻⁷ M, 10⁻⁸ M, 10⁻⁹ M, or lower.

In some embodiments, the GARP-TGFβ1-binding molecule has the following properties: inhibiting TGFβ1 activity, inhibiting the immunosuppressive activity of regulatory T cells, and/or inhibiting tumor growth.

In some embodiments, the GARP-TGFβ1-binding molecule is an anti-GARP-TGFβ1 antibody or an antigen-binding fragment thereof; in some specific embodiments, the GARP-TGFβ1-binding molecule is a chimeric antibody, a camelid antibody, a humanized antibody, a fully human antibody, or an antigen-binding fragment thereof.

In some embodiments, the GARP-TGFβ1-binding molecule is an anti-GARP-TGFβ1 antibody or an antigen-binding fragment thereof; in some specific embodiments, the anti-GARP-TGFβ1 antibody or the antigen-binding fragment thereof includes, but is not limited to: a Fab, an Fv, an sFv, a Fab', a F(ab')₂, a linear antibody, a single-chain antibody, an scFv, an sdAb, an sdFv, a nanobody, a peptibody, a domain antibody, and a multispecific antibody (a bispecific antibody, a diabody, a triabody, and a tetrabody, a tandem di-scFv, and a tandem tri-scFv), for example, an scFv, Fv, Fab, or Fab' fragment.

In some embodiments, provided is an anti-GARP-TGFβ1 antibody or an antigen-binding fragment thereof that binds to or competes with the aforementioned GARP-TGFβ1-binding molecule for binding to the same epitope of a GARP-TGFβ1 complex (e.g., a human or murine GARP-TGFβ1 complex).

In some embodiments, provided is an anti-GARP-TGFβ1 antibody or an antigen-binding fragment thereof that blocks the binding of the aforementioned GARP-TGFβ1-binding molecule to GARP-TGFβ1 (e.g., a human or murine GARP-TGFβ1 complex).

In some embodiments, provided is an anti-GARP-TGFβ1 antibody or an antigen-binding fragment thereof whose binding to a GARP-TGFβ1 complex (e.g., a human or murine GARP-TGFβ1 complex) is blocked by the aforementioned GARP-TGFβ1-binding molecule.

In some embodiments, the GARP-TGFβ1 complex comprises (i) GARP and (ii) a TGFβ1 precursor protein. Illustratively, in the GARP-TGFβ1 complex:
GARP comprises the amino acid sequence set forth in any one of SEQ ID NOs: 2, 4, 5, 8, 100, and 103 or an amino acid sequence having at least 80% sequence identity thereto;
and/or the TGFβ1 precursor protein comprises the amino acid sequence set forth in any one of SEQ ID NOs: 1, 3, 6-7, and 9-10 or an amino acid sequence having at least 80% sequence identity thereto.

### Protein-Binding Molecule

In some embodiments, the present disclosure provides a TGFβ1-binding molecule.

In some embodiments, the TGFβ1-binding molecule specifically binds to a TGFβ1 precursor protein or a TGFβ1 complex. In some embodiments, the TGFβ1 complex comprises a TGFβ1 precursor protein.

As used herein, "TGFβ1" or "TGFβ1 protein" is understood in a broad sense and comprises the pro-protein form (also known as pro-TGFβ1) or latent form (latent TGFβ1) of the transforming growth factor-β1 (TGFβ1) protein, or mature TGFβ1 after release. In some embodiments, TGFβ1 or a TGFβ1 protein refers to a TGFβ1 precursor protein.

In some embodiments, the TGFβ1 precursor protein comprises: (i) a mature TGFβ1 domain and (ii) a latency-associated peptide (LAP).

Illustratively, the TGFβ1 precursor protein is pro-TGFβ1 or latent TGFβ1. The mature TGFβ1 domain in pro-TGFβ1 covalently binds to latency-associated peptides (LAPs), and the mature TGFβ1 domain in latent TGFβ1 non-covalently binds to latency-associated peptides (LAPs).

In the present disclosure, "pro-TGFβ1" or "latent TGFβ1" is used interchangeably.

Illustratively, the mature TGFβ1 domain comprises the amino acid sequence set forth in SEQ ID NO: 101 or 104 or an amino acid sequence having at least 80% sequence identity thereto.

Illustratively, the LAP comprises the amino acid sequence set forth in SEQ ID NO: 102 or 105 or an amino acid sequence having at least 80% sequence identity thereto.

Illustratively, the TGFβ1 precursor protein comprises the amino acid sequence set forth in any one of SEQ ID NOs: 1, 3, 6-7, and 9-10 or an amino acid sequence having at least 80% sequence identity thereto.

In some embodiments, the TGFβ1 precursor protein or TGFβ1 complex further comprises a protein fragment, a functional variant, etc. of any of the aforementioned proteins or protein complexes.

In some embodiments, the TGFβ1 precursor protein or TGFβ1 complex is derived from any vertebrate, including mammals such as primates (e.g., humans) and other species (e.g., mice, rats, guinea pigs, rabbits, dogs, pigs, and sheep).

Illustratively, "fragment" or "protein fragment" includes, but is not limited to: growth factor domains, N-terminal prodomains, latency-associated peptides (LAPs), LAP-like domains, straight jacket regions, fastener regions, furin cleavage site regions, arm regions, fingers regions, latency loops, α₁ helical regions (alpha 1 helical regions), α₂ helical regions (alpha 2 helical regions), RGD sequence regions, trigger loop regions, extracellular domains, transmembrane domains, cytoplasmic domains, etc.

Illustratively, "variant" or "functional variant" refers to a protein or protein complex comprising substitution, deletion, and/or addition of one or more amino acids and having comparable bioactivity.

Illustratively, the variant of the TGFβ1 precursor protein comprises an amino acid mutation at a position naturally numbered 4 relative to the amino acid sequence set forth in SEQ ID NO: 6. In some embodiments, the variant of the TGFβ1 precursor protein comprises mutation C4S, wherein the site of the mutation is a site naturally numbered relative to the amino acid sequence set forth in SEQ ID NO: 6.

In some embodiments, the TGFβ1 precursor protein or TGFβ1 complex may or may not comprise a leader sequence. Illustratively, the leader sequence is a signal peptide sequence. In some embodiments, the TGFβ1 precursor protein or TGFβ1 complex may or may not comprise a tag sequence. Illustratively, the tag sequence is His-tag, AVI-tag, myc-tag, fluorescent tag, etc.

In some embodiments, the TGFβ1 complex comprises a TGFβ1 protein and any second protein selected from the group consisting of a glycoprotein-A repetitions predominant (GARP), a latent TGF-β-binding protein (LTBP; e.g., LTBP1, LTBP1S, LTBP2, LTBP3, or LTBP4), a fibrillin (e.g., fibrillin-1, fibrillin-2, fibrillin-3, or fibrillin-4), LRRC33 (leucine-rich repeat-containing protein 33), a variant or a protein fragment of any one of the foregoing, and the like.

Illustratively, "protein fragment" or "fragment" includes, but is not limited to, an extracellular domain, a transmembrane domain, a cytoplasmic domain, etc.

In some embodiments, the TGFβ1 complex includes, but is not limited to: a GARP-TGFβ1 complex, an LTBP-TGFβ1 complex (e.g., an LTBP1-TGFβ1 complex or an LTBP3-TGFβ1 complex), or an LRRC33-TGFβ1 complex. In some embodiments, the TGFβ1 in the TGFβ1 complex is present in the form of a TGFβ1 precursor protein.

Illustratively, the GARP-TGFβ1 complex comprises:
a) glycoprotein-A repetitions predominant (GARP);
b) a mature TGFβ1 domain; and
c) a latency-associated peptide (LAP).

In some embodiments, the GARP comprises the amino acid sequence set forth in SEQ ID NO: 100 or 103 or an amino acid sequence having at least 80% sequence identity thereto, the TGFβ1 comprises the amino acid sequence set forth in SEQ ID NO: 101 or 104 or an amino acid sequence having at least 80% sequence identity thereto, and/or the LAP comprises the amino acid sequence set forth in SEQ ID NO: 102 or 105 or an amino acid sequence having at least 80% sequence identity thereto.

In some embodiments, the GARP-TGFβ1 complex is formed by the GARP with the TGFβ1 precursor protein.

In some specific embodiments, the GARP-TGFβ1 complex is formed by an extracellular domain of the GARP with the TGFβ1 precursor protein.

In the present disclosure, "GARP" may be wild-type GARP of natural origin, or may be a functional variant of GARP (e.g., comprising one or more modifications, truncations, and/or mutations as compared to wild-type GARP). In some embodiments, the GARP may be full-length or a partial domain thereof (e.g., an extracellular domain, a transmembrane domain, or a cytoplasmic domain). For example, the natural origin refers to sources from any vertebrate, including mammals such as primates (e.g., humans) and other species (e.g., mice, rats, guinea pigs, rabbits, dogs, pigs, and sheep).

In some embodiments, the GARP may or may not comprise a leader sequence. Illustratively, the leader sequence is a signal peptide sequence. In some embodiments, the GARP may comprise one or more tag sequences, e.g., His-tag, AVI-tag, myc-tag, fluorescent tag, etc. In some embodiments, the GARP may not comprise a tag sequence.

Illustratively, the extracellular domain of the GARP comprises the amino acid sequence set forth in SEQ ID NO: 100 or 103 or an amino acid sequence having at least 80% sequence identity thereto.

Illustratively, the GARP comprises the amino acid sequence set forth in any one of SEQ ID NOs: 2, 4, 5, 8, 100, and 103 or an amino acid sequence having at least 80% sequence identity thereto.

In some embodiments, the protein-binding molecule that binds to the TGFβ1 precursor protein or TGFβ1 complex is selected from the group consisting of an antibody or an antigen-binding fragment thereof. Illustratively, the antibody or the antigen-binding fragment thereof includes, but is not limited to, any one of the following: a linear antibody, a single-chain antibody (scFv), a single-domain antibody (sdAb), a nanobody, a peptibody, a domain antibody, a multispecific antibody (a bispecific antibody, a diabody, a triabody, a tetrabody, a tandem di-scFv, and a tandem tri-scFv), a Fab, an Fv, an sFv, a Fab', and a F(ab')2.

In some embodiments, provided is an antibody or an antigen-binding fragment thereof that specifically binds to an epitope of the TGFβ1 precursor protein or TGFβ1 complex. In some embodiments, when the TGFβ1 is in the form of a precursor protein or is present in a complex with GARP, LTBP1, LTBP3, and/or LRRC33, the epitope can be used to be bound by an antibody or an antigen-binding fragment thereof. In some embodiments, the epitope is available due to a conformational change in TGFβ1 upon its complexation with GARP, LTBP, and/or LRRC33. In some embodiments, the epitope in TGFβ1 to which the antibody or the antigen-binding fragment thereof binds is unavailable when TGFβ1 is not complexed with GARP, LTBP, and/or LRRC33, or when TGFβ1 is mature TGFβ1. In some embodiments, the antibody or the antigen-binding fragment thereof does not prevent TGFβ1 from binding to integrin. For example, in some embodiments, the antibody or the antigen-binding fragment thereof does not mask the integrin binding site of TGFβ1.

In some embodiments, the protein-binding molecule does not bind or binds extremely weakly to a TGFβ2 protein or a TGFβ2 complex. Illustratively, the protein-binding molecule does not specifically bind to at least one of the following: mature TGFβ2, a TGFβ2 precursor protein, a GARP-TGFβ2 complex, an LTBP-TGFβ2 complex, etc.

In some embodiments, the protein-binding molecule does not bind or binds extremely weakly to a TGFβ3 protein or a TGFβ3 complex. Illustratively, the protein-binding molecule does not specifically bind to at least one of the following: mature TGFβ3, a TGFβ3 precursor protein, a GARP-TGFβ3 complex, an LTBP-TGFβ3 complex, etc.

In the present disclosure, "TGFβ2 protein" should be understood in a broad sense and encompasses mature TGFβ2, as well as inactive precursor protein forms such as pro-TGFβ2 and latent TGFβ2. "Pro-TGFβ2" and "latent TGFβ2" are used interchangeably.

In the present disclosure, "TGFβ3 protein" should be understood in a broad sense and encompasses mature TGFβ3, as well as inactive precursor protein forms such as pro-TGFβ3 and latent TGFβ3. "Pro-TGFβ3" and "latent TGFβ3" are used interchangeably.

In some embodiments, mature TGFβ1, mature TGFβ2, and/or mature TGFβ3 are present in a free state in a cell.

In some embodiments, the protein-binding molecule specifically binds to a TGFβ1 precursor protein or a TGFβ1 complex (e.g., a GARP-TGFβ1 complex, an LTBP1-TGFβ1 complex, an LTBP3-TGFβ1 complex, an LRRC33-TGFβ1 complex, etc.), and can selectively inhibit the activity of TGFβ1, e.g., inhibiting the activation of TGFβ1, and/or inhibiting the release of mature TGFβ1, and/or inhibiting TGFβ1 signal transduction.

### Polynucleotide

The present disclosure provides a polynucleotide encoding any one of the aforementioned protein-binding molecules. In some embodiments, the present disclosure provides a polynucleotide encoding any one of the aforementioned TGFβ1-binding molecules or GARP-TGFβ1-binding molecules.

In some embodiments, the present disclosure provides a polynucleotide encoding the TGFβ1-binding molecule according to the first aspect of the present disclosure. In some embodiments, the present disclosure provides a polynucleotide encoding the GARP-TGFβ1-binding molecule according to the second aspect of the present disclosure.

In some embodiments, the polynucleotide of the present disclosure may be an RNA, a DNA, or a cDNA.

In some embodiments, the polynucleotide of the present disclosure is an isolated polynucleotide.

In some embodiments, the polynucleotide of the present disclosure may also be in the form of a vector, may be present in a vector, and/or may be part of a vector. The vector may be a eukaryotic vector, a prokaryotic vector, or a viral vector, such as a plasmid, a cosmid, a YAC, or a viral vector. The vector may especially be an expression vector, i.e., a vector that provides for *in vitro* and/or *in vivo* (i.e., in suitable host cells, host organisms, and/or expression systems) expression of the binding molecule (e.g., TGFβ1-binding molecule). The expression vector generally comprises at least one of the polynucleotides of the present disclosure, which is operably linked to one or more suitable expression regulatory elements (e.g., promoters, enhancers, and terminators). The selection of the elements and their sequences for expression in a particular host is within the knowledge of those skilled in the art. Regulatory elements and other elements useful or necessary for expression of the protein-binding molecule, TGFβ1-binding molecule, or GARP-TGFβ1-binding molecule of the present disclosure include, for example, promoters, enhancers, terminators, integrons, selection labels, leader sequences, and reporter genes.

The polynucleotide of the present disclosure may be prepared or obtained by known means (e.g., by automatic DNA synthesis and/or recombinant DNA techniques) based on information on the amino acid sequence of the polypeptide of the present disclosure, and/or may be isolated from a suitable natural source.

In some embodiments, the polynucleotide and the vector in the present disclosure can be used to prepare a TGFβ1-binding molecule. In some embodiments, the polynucleotide and the vector in the present disclosure are used to express a TGFβ1-binding molecule *in vitro* or *in vivo.* The TGFβ1-binding molecule binds to a TGFβ1 precursor protein or a TGFβ1 complex for various purposes such as detection, diagnosis, treatment, and regulation.

In some embodiments, the polynucleotide and the vector in the present disclosure can be used to prepare a GARP-TGFβ1-binding molecule. In some embodiments, the polynucleotide and the vector in the present disclosure are used to express a GARP-TGFβ1-binding molecule *in vitro* or *in vivo.* The GARP-TGFβ1-binding molecule binds to a GARP-TGFβ1 complex for various purposes such as detection, diagnosis, treatment, and regulation.

### Host Cell

The present disclosure provides a host cell expressing one or more of the protein-binding molecules of the present disclosure. In some embodiments, the host cell provided by the present disclosure comprises any one of the aforementioned polynucleotides or vectors; or the host cell expresses any one of the aforementioned TGFβ1-binding molecules or GARP-TGFβ1-binding molecules.

In some embodiments, the host cell expresses the TGFβ1-binding molecule according to the first aspect of the present disclosure. In some embodiments, the host cell expresses the GARP-TGFβ1-binding molecule according to the second aspect of the present disclosure.

In some embodiments, the host cell is a bacterial cell, a fungal cell, or a mammalian cell.

Illustratively, bacterial cells include, e.g., cells of gram-negative bacterial strains (e.g., *Escherichia coli* strains, *Proteus* strains, and *Pseudomonas* strains), and gram-positive bacterial strains (e.g., *Bacillus* strains, *Streptomyces* strains, *Staphylococcus* strains, and *Lactococcus* strains).

Illustratively, fungal cells include, e.g., cells of the species *Trichoderma, Neurospora,* and *Aspergillus;* or cells of the species *Saccharomyces* (e.g., *Saccharomyces cerevisiae*)*, Schizosaccharomyces* (e.g., *Schizosaccharomyces pombe*)*, Pichia* (e.g., *Pichia pastoris* and *Pichia methanolica*)*,* and *Hansenula.*

Illustratively, mammalian cells are, e.g., monkey kidney CV1 lines (COS-7), human embryonic kidney lines (293 or 293T cells), baby hamster kidney cells (BHK), mouse sertoli cells (TM4 cells), monkey kidney cells (CV1), African green monkey kidney cells (VERO-76), canine kidney cells (MDCK), buffalo rat liver cells (BRL3A), human lung cells (W138), human liver cells (HepG2), human glioblastoma cells (LN229 cells), human cervical cancer cells (HeLa cells), human breast cancer cells (MCF-7), human prostate cancer cells (PC3), mouse breast cancer cells (EMT-6 cells), mouse mammary tumor cells (MMT060562), mouse colon cancer cells (CT26 cells), TRI cells (as described, for example, in Mather et al., Annals N. Y. Acad Sci 383, 44-68 (1982)), MRC5 cells and FS4 cells, Chinese hamster ovary (CHO) cells, or myeloma cell lines such as YO, NS0, P3X63, and Sp2/0.

Amphibian cells, insect cells, plant cells, and any other cells used in the art for expressing heterologous proteins may also be used in the present disclosure.

The cells of the present disclosure are unable to develop into complete plants or animal individuals.

### Production or Preparation Method

The present disclosure provides a method for preparing the protein-binding molecule of the present disclosure.

In some embodiments, the present disclosure provides a method for preparing the TGFβ1-binding molecule according to the first aspect described above, the method comprising the following steps:
a. forming a TGFβ1 precursor protein or a TGFβ1 complex comprising the TGFβ1 precursor protein as an antigen protein;
b. constructing a human-derived antibody phage library; and
c. screening a TGFβ1-binding molecule that specifically binds to the antigen protein by using the phage library.

In some embodiments, the method for preparing the TGFβ1-binding molecule further comprises the following step:
d. performing affinity maturation engineering on the TGFβ1-binding molecule.

The preparation method described above is also a screening method for a TGFβ1-binding molecule that specifically binds to a TGFβ1 precursor protein or a TGFβ1 complex comprising the TGFβ1 precursor protein.

In some embodiments, the present disclosure provides a method for preparing the GARP-TGFβ1-binding molecule according to the second aspect described above, the method comprising the following steps:
a. forming a complex comprising human GARP and a human TGFβ1 precursor protein as an antigen protein;
b. immunizing an animal with the antigen protein to construct a phage library; and
c. screening a GARP-TGFβ1-binding molecule comprising an immunoglobulin single variable domain that binds to the GARP-TGFβ1 complex by using the phage library.

In some embodiments, the method for preparing the GARP-TGFβ1-binding molecule further comprises the following step:
d. humanizing the GARP-TGFβ1-binding molecule.

In some specific embodiments, step b further comprises:
immunizing a camel by using the antigen protein, collecting peripheral blood of the immunized camel, extracting a nucleic acid, and constructing a phage library by using the nucleic acid.

The preparation method described above is also a screening method for a GARP-TGFβ1-binding molecule that specifically binds to a GARP-TGFβ1 complex.

In some other embodiments, the present disclosure provides a method for preparing any one of the aforementioned TGFβ1-binding molecules or GARP-TGFβ1-binding molecules, the method comprising:
- culturing the host cell of the present disclosure under conditions that allow expression of the TGFβ1-binding molecule or the GARP-TGFβ1-binding molecule of the present disclosure; and
- isolating the protein of interest expressed by the host cell from the culture; and
- optionally, further purifying and/or modifying the protein of interest of the present disclosure.

The TGFβ1-binding molecule or the GARP-TGFβ1-binding molecule of the present disclosure can be produced intracellularly (e.g., in the cytoplasm, in the periplasm, or in inclusion bodies) in a cell as described above, followed by isolation from the host cell and optionally further purification; or it may be produced extracellularly (e.g., in the medium in which the host cell is cultured), followed by isolation from the medium and optionally further purification.

Methods and reagents for recombinant production of proteins or polypeptides, e.g., specific suitable expression vectors, transformation or transfection methods, selection labels, methods for inducing protein expression, culture conditions, and the like, are known in the art. Similarly, isolation and purification techniques suitable for use in the preparation of proteins of interest, such as the binding molecules or antibodies of the present disclosure, are well known to those skilled in the art. Methods for producing and purifying antibodies are well known in the prior art and can be found in, for example, "Antibodies: A Laboratory Manual", Cold Spring Harbor Press (chapters 5-8 and 15). The engineered antibodies of the present disclosure may also be prepared and purified by conventional methods. For example, cDNA sequences encoding heavy and light chains can be cloned and recombined into an expression vector. Recombinant immunoglobulin expression vectors can be used to stably transfect cells. Mammalian expression systems may result in glycosylation of antibodies, particularly at the highly conserved N-terminus of the Fc region. Stable clones are obtained by expression of antibodies specifically binding to human antigens. Positive clones are expanded in a serum-free culture medium in a bioreactor to produce antibodies. The culture solution with the secreted antibodies can be purified and collected by conventional techniques. The antibodies can be filtered and concentrated by conventional methods. Soluble mixtures and multimers can also be removed by conventional methods, such as molecular sieves and ion exchange. The resulting products need to be immediately frozen, such as at -70 °C, or lyophilized.

However, the TGFβ1-binding molecule or the GARP-TGFβ1-binding molecule of the present disclosure may also be obtained by other methods for producing proteins known in the art, such as chemical synthesis, including solid-phase or liquid-phase synthesis.

### Pharmaceutical Composition

The present disclosure provides a pharmaceutical composition comprising any one selected from the group consisting of the following or a combination thereof: the protein-binding molecule or the polynucleotide encoding same described above. In some embodiments, the pharmaceutical composition comprises a prophylactically or therapeutically effective amount of the TGFβ1-binding molecule or the GARP-TGFβ1-binding molecule according to any one of the foregoing, or the polynucleotide or vector encoding the TGFβ1-binding molecule or the GARP-TGFβ1-binding molecule.

In some embodiments, the pharmaceutical composition further comprises one or more pharmaceutically acceptable auxiliary materials, diluents, buffers, or excipients.

In some embodiments, the pharmaceutical composition may be formulated into any dosage form known in the medical field, and the dosage form is selected depending on the intended mode of administration and therapeutic use.

In some embodiments, the pharmaceutical composition further comprises an immune checkpoint inhibitor. For example, the immune checkpoint inhibitor includes, but is not limited to, one or more inhibitors of PD-1, PD-L1, and PD-L2. In some embodiments, the pharmaceutical composition further comprises an anti-PD-1 antibody or an antigen-binding fragment thereof.

The TGFβ1-binding molecule or the GARP-TGFβ1-binding molecule in the present disclosure is capable of inhibiting TGFβ1 activity and inhibiting the immunosuppressive activity of regulatory T cells. The combination of the TGFβ1-binding molecule or the GARP-TGFβ1-binding molecule with the anti-PD-1 antibody can relieve the immunosuppressive microenvironment of tumors. In the examples of the present disclosure, it is found that the TGFβ1-binding molecule or the GARP-TGFβ1-binding molecule in combination with the anti-PD-1 antibody showed a significantly improved therapeutic effect in the treatment of tumors.

In some embodiments, a unit dose of the pharmaceutical composition may comprise 0.01-99 wt% of the TGFβ1-binding molecule. In some other specific embodiments, the amount of the TGFβ1-binding molecule in a unit dose of the pharmaceutical composition is 0.1-2000 mg; in some specific embodiments, the amount is 1-1000 mg.

In some embodiments, a unit dose of the pharmaceutical composition may comprise 0.01-99 wt% of the GARP-TGFβ1-binding molecule. In some other specific embodiments, the amount of the GARP-TGFβ1-binding molecule in a unit dose of the pharmaceutical composition is 0.1-2000 mg; in some specific embodiments, the amount is 1-1000 mg.

### Combination therapy

The present disclosure provides use of any one of the protein-binding molecules or the polynucleotides encoding same in combination with an immune checkpoint inhibitor in the treatment of a disease associated with a TGFβ signaling pathway. Illustratively, the immune checkpoint inhibitor includes, but is not limited to, one or more inhibitors of PD-1, PD-L1, and PD-L2.

In some embodiments, provided is use of a TGFβ1-binding molecule in combination with an immune checkpoint inhibitor in the treatment of a disease associated with a TGFβ signaling pathway.

In some embodiments, provided is use of a TGFβ1-binding molecule in combination with an anti-PD-1 antibody or an antigen-binding fragment thereof in the treatment of a disease associated with a TGFβ signaling pathway.

In some embodiments, provided is use of a GARP-TGFβ1-binding molecule in combination with an immune checkpoint inhibitor in the treatment of a disease associated with a TGFβ signaling pathway.

In some embodiments, provided is use of a GARP-TGFβ1-binding molecule in combination with an anti-PD-1 antibody or an antigen-binding fragment thereof in the treatment of a disease associated with a TGFβ signaling pathway.

The term "disease associated with a TGFβ signaling pathway" refers to any disease, disorder, and/or condition associated with the expression, activity, and/or metabolism of TGFβ family proteins or any disease, disorder, and/or condition that may benefit from regulation of the activity and/or level of one or more TGFβ family proteins. The disease associated with the TGFβ signaling pathway may include, but is not limited to, tumors or cancers.

In some embodiments, the disease associated with the TGFβ signaling pathway is cancer. The present disclosure provides use of a TGFβ1-binding molecule or a GARP-TGFβ1-binding molecule in combination with an anti-PD-1 antibody or an antigen-binding fragment thereof in the treatment of cancer.

The TGFβ1-binding molecule or the GARP-TGFβ1-binding molecule significantly improves the immunosuppressive microenvironment of tumors, and when used in combination with an anti-PD-1 antibody, it shows a significantly improved therapeutic effect in the treatment of tumors.

In some embodiments, the cancer is selected from the group consisting of lung cancer, intestinal cancer, renal cancer, bladder cancer, liver cancer, gastric cancer, breast cancer, colon cancer, cervical cancer, prostate cancer, and head and neck cancer.

### Kit (or Kit-of-Parts)

The present disclosure provides a kit or kit-of-parts, which comprises one or more containers each independently comprising any one selected from the group consisting of the following or a combination thereof: the protein-binding molecule (e.g., the TGFβ1-binding molecule or the GARP-TGFβ1-binding molecule), the polynucleotide encoding same, or the vector thereof of the present disclosure.

### Method for Preventing and Treating Disease and Pharmaceutical Use

The present disclosure provides pharmaceutical use of a protein-binding molecule, a polynucleotide encoding same, a vector thereof, or a pharmaceutical composition thereof, and a method for preventing, treating, or alleviating a disease or symptom.

In some embodiments, the present disclosure provides use of a TGFβ1-binding molecule, an encoding polynucleotide, a vector, or a pharmaceutical composition in at least one of the following:
(1) inhibiting TGFβ1 activity, e.g., inhibiting the activation of TGFβ1, inhibiting the release of mature TGFβ1 from a TGFβ1 complex, and/or inhibiting TGFβ1 signal transduction;
(2) manufacturing a medicament for inhibiting TGFβ1 activity;
(3) inhibiting the immunosuppressive activity of regulatory T cells;
(4) manufacturing a medicament for inhibiting the immunosuppressive activity of regulatory T cells;
(5) preventing or treating a disease or disorder associated with a TGFβ signaling pathway.

In some embodiments, the present disclosure provides a method for preventing or treating a disease or disorder associated with a TGFβ signaling pathway, the method comprising administering to a subject a prophylactically or therapeutically effective amount of a TGFβ1-binding molecule, an encoding polynucleotide, a vector, or a pharmaceutical composition.

In some embodiments, the present disclosure provides a method for inhibiting TGFβ1 activity *in vitro,* the method comprising administering a TGFβ1 antibody or an antigen-binding fragment thereof, an encoding polynucleotide, a vector, or a pharmaceutical composition *in vitro.* In some specific embodiments, an inhibitory effective amount of the TGFβ1-binding molecule, the encoding polynucleotide, the vector, or the pharmaceutical composition is administered.

In some embodiments, the present disclosure provides a method for inhibiting TGFβ1 activity *in vivo,* the method comprising administering to a subject a TGFβ1-binding molecule, an encoding polynucleotide, a vector, or a pharmaceutical composition. In some specific embodiments, an inhibitory effective amount of the TGFβ1-binding molecule, the encoding polynucleotide, the vector, or the pharmaceutical composition is administered to the subject.

In some specific embodiments, the subject has a disease or disorder associated with a TGFβ signaling pathway.

The term "disease associated with a TGFβ signaling pathway" refers to any disease, disorder, and/or condition associated with the expression, activity, and/or metabolism of TGFβ family proteins or any disease, disorder, and/or condition that may benefit from regulation of the activity and/or level of one or more TGFβ family proteins. The disease associated with the TGFβ signaling pathway may include, but is not limited to, a disease or disorder associated with fibrosis, a tumor, or cancer.

In some embodiments, the disease associated with the TGFβ signaling pathway is cancer.

In some embodiments, the disease or disorder associated with the TGFβ signaling pathway is fibrosis.

In some embodiments, provided is a method for preventing or treating cancer, the method comprising co-administering a prophylactically or therapeutically effective amount of the following components shown in 1) and 2):
1) an anti-TGFβ1 antibody or an antigen-binding fragment thereof, or an encoding polynucleotide, a vector, or a pharmaceutical composition;
2) an immune checkpoint inhibitor.

Illustratively, the PD-1 signaling pathway inhibitor includes, but is not limited to, one or more inhibitors of PD-1, PD-L1, and PD-L2. In some embodiments, the immune checkpoint inhibitor is an anti-PD-1 antibody or an antigen-binding fragment thereof.

In some embodiments, the cancer is selected from the group consisting of lung cancer, intestinal cancer, renal cancer, bladder cancer, liver cancer, gastric cancer, breast cancer, colon cancer, cervical cancer, prostate cancer, and head and neck cancer.

In some embodiments, the TGFβ1-binding molecule or the pharmaceutical composition of the present disclosure may be administered by any suitable method known in the art, which may be systemic or local.

In some embodiments, the administration regimen may be adjusted to provide the optimum desired response (e.g., a therapeutic or prophylactic response). For example, a single dose may be administered, several doses may be administered over a period of time, or the dose may be proportionally reduced or increased depending on the exigencies of the therapeutic situation.

In some embodiments, the present disclosure provides use of a GARP-TGFβ1-binding molecule, an encoding polynucleotide, a vector, or a pharmaceutical composition in at least one of the following:
(1) inhibiting TGFβ1 activity, e.g., inhibiting the activation of TGFβ1, inhibiting the release of mature TGFβ1 from a TGFβ1 complex, and/or inhibiting TGFβ1 signal transduction;
(2) manufacturing a medicament for inhibiting TGFβ1 activity;
(3) inhibiting the immunosuppressive activity of regulatory T cells;
(4) manufacturing a medicament for inhibiting the immunosuppressive activity of regulatory T cells;
(5) preventing or treating a disease or disorder associated with a TGFβ signaling pathway.

In some embodiments, the present disclosure provides a method for preventing or treating a disease or disorder associated with a TGFβ signaling pathway, the method comprising administering to a subject a prophylactically or therapeutically effective amount of a GARP-TGFβ1-binding molecule, an encoding polynucleotide, a vector, or a pharmaceutical composition.

In some embodiments, the present disclosure provides a method for preventing or treating a disease associated with a TGFβ signaling pathway, the method comprising administering to a subject a prophylactically or therapeutically effective amount of a GARP-TGFβ1-binding molecule, an encoding polynucleotide, a vector, or a pharmaceutical composition; and a prophylactically or therapeutically effective amount of an immune checkpoint inhibitor.

Illustratively, the immune checkpoint inhibitor includes, but is not limited to, one or more inhibitors of PD-1, PD-L1, and PD-L2. In some embodiments, the immune checkpoint inhibitor is an anti-PD-1 antibody or an antigen-binding fragment thereof.

In some embodiments, the present disclosure provides a method for inhibiting TGFβ1 activity *in vitro,* the method comprising administering a GARP-TGFβ1-binding molecule, an encoding polynucleotide, a vector, or a pharmaceutical composition *in vitro.* In some specific embodiments, an inhibitory effective amount of the GARP-TGFβ1-binding molecule, the encoding polynucleotide, the vector, or the pharmaceutical composition is administered.

In some embodiments, the present disclosure provides a method for inhibiting the activation of TGFβ1 or inhibiting the release of mature TGFβ1 from a GARP-TGFβ1 complex *in vitro,* the method comprising administering a GARP-TGFβ1-binding molecule, an encoding polynucleotide, a vector, or a pharmaceutical composition *in vitro.* In some specific embodiments, an inhibitory effective amount of the GARP-TGFβ1-binding molecule, the encoding polynucleotide, the vector, or the pharmaceutical composition is administered.

In some embodiments, the present disclosure provides a method for inhibiting TGFβ1 activity *in vivo,* the method comprising administering to a subject a GARP-TGFβ1-binding molecule, an encoding polynucleotide, a vector, or a pharmaceutical composition. In some specific embodiments, an inhibitory effective amount of the GARP-TGFβ1-binding molecule, the encoding polynucleotide, the vector, or the pharmaceutical composition is administered to the subject.

In some embodiments, the present disclosure provides a method for inhibiting the activation of TGFβ1 or inhibiting the release of mature TGFβ1 from a GARP-TGFβ1 complex *in vivo,* the method comprising administering to a subject a GARP-TGFβ1-binding molecule, an encoding polynucleotide, a vector, or a pharmaceutical composition. In some specific embodiments, an inhibitory effective amount of the GARP-TGFβ1-binding molecule, the encoding polynucleotide, the vector, or the pharmaceutical composition is administered to the subject.

In some specific embodiments, the subject has a disease or disorder associated with a TGFβ signaling pathway.

In some embodiments, the disease associated with the TGFβ signaling pathway is cancer.

In some embodiments, the cancer is selected from the group consisting of lung cancer, intestinal cancer, renal cancer, bladder cancer, liver cancer, gastric cancer, breast cancer, colon cancer, cervical cancer, prostate cancer, and head and neck cancer.

In some embodiments, the GARP-TGFβ1-binding molecule or the pharmaceutical composition of the present disclosure may be administered by any suitable method known in the art, which may be systemic or local.

In some embodiments, the administration regimen may be adjusted to provide the optimum desired response (e.g., a therapeutic or prophylactic response). For example, a single dose may be administered, several doses may be administered over a period of time, or the dose may be proportionally reduced or increased depending on the exigencies of the therapeutic situation.

### Definition

In order to facilitate the understanding of the present disclosure, some technical and scientific terms are specifically defined below. Unless otherwise specifically defined in the present disclosure, all other technical and scientific terms used in the present disclosure have the meanings generally understood by those of ordinary skill in the art to which the present disclosure pertains.

Unless the context clearly requires otherwise, throughout the description and the claims, the words "comprise", "have", "include", and the like are to be understood in an inclusive sense as opposed to an exclusive or exhaustive sense; that is to say, in the sense of "including, but not limited to".

"Optional" or "optionally" means that the event or circumstance subsequently described may, but does not necessarily, occur, and this description includes instances where the event or circumstance occurs or does not occur.

"About" or "approximately" means that a numerical value is within an acceptable error range for the specific value determined by those of ordinary skill in the art, and the numerical value depends in part on how the value is measured or determined (i.e., the limitations of the measurement system). For example, "about" may mean a standard deviation within 1 or more than 1. Alternatively, "about" or "substantially comprise" may mean a range of at most 20%, e.g., a change of between 1% and 15%, between 1% and 10%, between 1% and 5%, between 0.5% and 5%, or between 0.5% and 1%. In the present disclosure, every instance where a number or numerical range is preceded by the term "about" also includes embodiments of the given number. Unless otherwise stated, when a specific value is provided in the present application and claims, the meaning of "about" or "substantially comprise" should be assumed to be within an acceptable error range for that specific value.

The three-letter and single-letter codes for amino acids used in the present disclosure are as described in J. biol. chem., 243, p3558 (1968).

The term "transforming growth factor-β" family consists of a class of structurally and functionally related polypeptide growth factor subfamilies and is involved in many different biological pathways. In addition to TGF-β, activins, inhibins, growth and differentiation factors (GDFs), bone morphogenetic proteins (BMPs), and the like are also included. TGFβ has 3 forms of isomers: TGFβ1, TGFβ2, and TGFβ3, and is widely expressed in almost all cell types in mammals.

Unlike other cytokines, members of the TGFβ superfamily are secreted not as active growth factors, but as dimeric precursor proteins consisting of an N-terminal prodomain and a C-terminal growth factor domain. Cleavage of pro-TGFβ1 by furin separates the homodimeric growth factor domain from its prodomain (also known as latency-associated peptide (LAP)). However, the growth factor remains non-covalently binding to LAP, thereby forming a latent complex that is unable to bind to its receptor and induce signaling. During translation, latent TGFβ1 (also known as a small latent complex (SLC)) becomes linked to a "presenting molecule" by a disulfide bridge, thereby forming a large latent complex (LLC). These molecules allow pro-TGFβ1 to be present in specific cell or tissue contexts. Two cysteines near the N-terminus of the latent TGFβ1 are linked to appropriately positioned cysteines on the presenting molecule. The identity of the presenting molecule depends on the environment and the type of cell that produces the latent TGFβ1. For example, fibroblasts secrete latent TGFβ1 tethered to TGFβ-binding proteins (LTBPs), which then bind to proteins (i.e., fibronectin and fibrillin-1) in the extracellular matrix (ECM) to link the latent TGFβ to the ECM (Robertson et al., Matrix Biol, 47:44-53 (2015)). On the surface of activated regulatory T cells, the latent TGFβ1 is covalently linked to a transmembrane protein GARP, and proteins closely related to GARP and LRRC33 have recently been identified as presenting molecules of TGFβ1 on the surface of monocytes, macrophages, and microglia (Wang, R. et al., Mol Biol Cell, 2012. 23(6): p. 1129-39 and T.A. Springer, Int. BMP Conference 2016).

The TGFβ1 protein should be understood in its broadest sense within the scope of the present disclosure. The term encompasses natural forms and naturally occurring variants of TGFβ1 in nature, also including artificially expressed forms, functional variants, etc. TGFβ1 encompasses mature TGFβ1, a TGFβ1 precursor protein, and a protein epitope thereof, unless otherwise specified in the context. The TGFβ1 precursor protein encompasses pro-TGFβ1, latent TGFβ1, and fragments thereof. The TGFβ1 sequence can be obtained from GenBank, UniProt, etc., such as human pro-TGFβ1 (Uniprot: P01137) and mouse pro-TGFβ1 (Uniprot: P04202).

In the context of the present disclosure, "pro-TGFβ1" and "latent TGFβ1" are used interchangeably.

As used herein, the term "TGFβ1 complex" refers to a complex formed by linking TGFβ1 to a protein molecule including but not limited to GARP, LRRC33, LTBP3, or LTBP1 via a disulfide bridge, also known as an LTBP1-TGFβ1 complex, an LTBP3-TGFβ1 complex, an LRRC33-TGFβ1 complex, or a GARP-TGFβ1 complex. In the TGFβ1 complex, TGFβ1 is present in the form of a TGFβ1 precursor protein (e.g., pro/latent TGFβ1).

The term "functional variant" includes, but is not limited to, a homolog, a fragment, a truncation, a mutant, a modification, and the like of a wild-type protein. The functional variant of a protein has increased, decreased, or maintained protein activity as compared to the wild-type protein.

The term "binding molecule" encompasses any molecule capable of specifically binding to an antigen or an antigenic epitope, e.g., the antibody, the antigen-binding fragment thereof, the conjugate thereof, or the fusion protein thereof as defined in the present disclosure.

"Antibody" is used in the broadest sense and encompasses a variety of antibody structures, including, but not limited to monoclonal antibodies, polyclonal antibodies; monospecific antibodies, multispecific antibodies (e.g., bispecific antibodies); and full-length antibodies, and antibody fragments (or antigen-binding fragments, or antigen-binding moieties) so long as they exhibit the desired antigen-binding activity. An antibody may refer to an immunoglobulin that is a four-peptide-chain structure formed by linking two identical heavy chains and two identical light chains by interchain disulfide bonds. The heavy chain constant regions of an immunoglobulin differ in their amino acid composition and arrangement, and thus in their antigenicity. Accordingly, immunoglobulins can be divided into five classes, or isotypes of immunoglobulins, namely IgM, IgD, IgG, IgA, and IgE, with their corresponding heavy chains being µ chain, δ chain, γ chain, α chain, and ε chain, respectively. Ig of the same class can be divided into different subclasses according to differences in the amino acid composition of the hinge regions and the number and positions of disulfide bonds of the heavy chains; for example, IgG can be divided into IgG1, IgG2, IgG3, and IgG4. Light chains are divided into κ or λ chains according to differences in the constant regions. Each of the five classes of Ig may have a κ chain or λ chain. In the antibody heavy and light chains, the sequences of about 110 amino acids near the N-terminus vary considerably and thus are referred to as variable regions (V regions); the remaining amino acid sequences near the C-terminus are relatively stable and thus are referred to as constant regions (C regions). The variable regions comprise 3 hypervariable regions (CDRs) and 4 framework regions (FRs) with relatively conservative sequences. The 3 hypervariable regions determine the specificity of the antibody and thus are also known as complementarity-determining regions (CDRs). Each of the light chain variable regions (VLs) and the heavy chain variable regions (VHs) consists of 3 CDRs and 4 FRs arranged from the amino-terminus to the carboxyl-terminus as follows: FR1, CDR1, FR2, CDR2, FR3, CDR3, and FR4. The 3 CDRs of the light chain refer to LCDR1, LCDR2, and LCDR3; and the 3 CDRs of the heavy chain refer to HCDR1, HCDR2, and HCDR3.

For the determination or definition of "CDRs", the deterministic depiction of CDRs and identification of residues comprising antigen-binding sites of the antibody can be accomplished by resolving the structure of the antibody and/or resolving the structure of the antibody-ligand complex. This can be accomplished by any one of a variety of techniques known to those skilled in the art, such as X-ray crystallography. A variety of analysis methods can be used to identify CDRs, including but not limited to the Kabat numbering scheme, the Chothia numbering scheme, the AbM numbering scheme, the IMGT numbering scheme, the contact definition, and the conformational definition.

The Kabat numbering scheme is a standard for numbering residues in antibodies and is generally used to identify CDRs (see, e.g., Johnson & Wu, 2000, Nucleic Acids Res., 28:214-8). The Chothia numbering scheme is similar to the Kabat numbering scheme, except that it takes into account the position of certain structural loop regions (see, e.g., Chothia et al., 1986, J. Mol. Biol., 196:901-17; Chothia et al., 1989, Nature, 342:877-83). The AbM numbering scheme adopts a computer program integration suite for modeling antibody structures manufactured by Oxford Molecular Group (see, e.g., Martin et al., 1989, Proc Natl Acad Sci (USA), 86:9268-9272; "AbMTM, A Computer Program for Modeling Variable Regions of Antibodies", Oxford, UK; Oxford Molecular, Ltd.). The AbM numbering scheme adopts a combination of a knowledge database and the de-novo method to model the tertiary structure of antibodies from basic sequences (see those described in Samudrala et al., 1999, "Ab Initio Protein Structure Prediction Using a Combined Hierarchical Approach", PROTEINS, Structure, Function and Genetics Suppl., 3:194-198). The contact definition is based on the analysis of the available complex crystal structures (see, e.g., MacCallum et al., 1996, J. Mol. Biol., 5:732-45). In the conformational definition, the positions of the CDRs can be identified as residues that contribute enthalpy to the antigen binding (see, e.g., Makabe et al., 2008, Journal of Biological Chemistry, 283:1156-1166). In addition, other CDR boundary definitions may not strictly follow one of the methods described above, but still overlap with at least a portion of the Kabat CDRs, although they may be shortened or lengthened based on predictions or experimental results that a particular residue or a particular group of residues do not significantly affect the antigen binding. As used in the present disclosure, a CDR may refer to a CDR defined by any method known in the art, including combinations of methods. The correspondence between the various numbering schemes is well known to those skilled in the art, and examples are shown in Table 4 below.

**Table 4. The relationships between the numbering schemes for CDRs**

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

"Domain" of a polypeptide or protein refers to a folded protein structure that is capable of maintaining its tertiary structure independently of the rest of the protein. In general, a domain is responsible for a single functional property of a protein, and in many cases may be added, deleted, or transferred to other proteins without loss of functions of the rest of the protein and/or the domain.

"Immunoglobulin variable domain" refers to an immunoglobulin domain essentially consisting of four "framework regions" referred to in the art and hereinafter as "framework region 1" or "FR1", "framework region 2" or "FR2", "framework region 3" or "FR3", and "framework region 4" or "FR4", wherein the framework regions are interrupted by three "complementarity-determining regions" or "CDRs" referred to in the art and hereinafter as "complementarity-determining region 1" or "CDR1", "complementarity-determining region 2" or "CDR2", and "complementarity-determining region 3" or "CDR3". Thus, the general structure or sequence of an immunoglobulin variable domain can be expressed as follows: FR1-CDR1-FR2-CDR2-FR3-CDR3-FR4. Immunoglobulin variable domains possess specificity for an antigen by virtue of having an antigen-binding site.

"Antibody framework (FR)" refers to a portion of a variable domain, which serves as a framework for the antigen-binding loops (CDRs) of the variable domain.

"Immunoglobulin single variable domain" is generally used to refer to an immunoglobulin variable domain (which may be a heavy or light chain domain, including a VH, VHH or VL domain) that can form a functional antigen-binding site without interacting with other variable domains (e.g., without VH/VL interactions as are required between the VH and VL domains of conventional four-chain monoclonal antibodies). Examples of "immunoglobulin single variable domains" include nanobodies (including a VHH, humanized VHH and/or camelized VH, e.g. a camelized human VH), IgNAR, domains, (single-domain) antibodies as VH domains or derived from VH domains (such as dAbs^{™}) and (single-domain) antibodies as VL domains or derived from VL domains (such as dAbs^{™}). Immunoglobulin single variable domains based on and/or derived from heavy chain variable domains (such as VH or VHH domains) are generally preferred. A specific example of an immunoglobulin single variable domain is a "VHH domain" (or "VHH" for short) as defined below.

"VHH domain", also known as heavy chain single-domain antibody, VHH, V_{H}H domain, VHH antibody fragment, VHH antibody or nanobody, is a variable domain of an antigen-binding immunoglobulin known as a "heavy-chain antibody" (i.e., "an antibody devoid of light chains") (Hamers-Casterman C, Atarhouch T, Muyldermans S, Robinson G, Hamers C, Songa EB, Bendahman N, Hamers R., "Naturally occurring antibodies devoid of light chains"; Nature 363, 446-448 (1993)). The term "VHH domain" is used to distinguish the variable domain from the heavy chain variable domain (which is referred to in the present disclosure as a "VH domain") and the light chain variable domain (which is referred to in the present disclosure as a "VL domain") present in conventional antibodies with a four-peptide-chain structure. VHH domains specifically bind to an epitope without the need for an additional antigen-binding domain (as opposed to the VH or VL domain in conventional antibodies with a four-peptide-chain structure, in which case the epitope is recognized by the VL domain together with the VH domain). A VHH domain is a small, stable, and efficient antigen recognition unit formed by a single immunoglobulin domain. The terms "heavy chain single-domain antibody", "VHH domain", "VHH", "V_{H}H domain", "VHH antibody fragment", "VHH antibody", "Nanobody", and "Nanobody domain" are used interchangeably. "VHH domains" include, but are not limited to, natural antibodies produced by camelids, antibodies produced by camelids and then humanized, or fully human-derived antibodies obtained by screening with phage display techniques. The total number of amino acid residues in a VHH domain will usually be in the range of 110 to 120, often between 112 and 115. However, it should be noted that smaller and longer sequences may also be suitable for the purposes described in the present disclosure. Methods for obtaining VHHs that bind to a particular antigen or epitope have been previously disclosed in the following documents: R. van der Linden et al., Journal of Immunological Methods, 240 (2000) 185-195; Li et al., J Biol Chem., 287 (2012) 13713-13721; Deffar et al., African Journal of Biotechnology Vol. 8 (12), pp.2645-2652, 17 June, 2009 and WO94/04678.

As is well known in the art for VH domains and for VHH domains, the total number of amino acid residues in each of the CDRs may vary and may not correspond to the total number of amino acid residues indicated by the Kabat numbering (that is, one or more positions according to the Kabat numbering may not be occupied in the actual sequence, or the actual sequence may contain more amino acid residues than the number allowed for by the Kabat numbering). This means that, in general, the numbering according to Kabat may or may not correspond to the actual numbering of the amino acid residues in the actual sequence. Other numbering systems or numbering schemes include Chothia, IMGT, and AbM.

"Humanized antibody", also known as CDR-grafted antibody, refers to an antibody produced by grafting non-human CDR sequences into the framework of variable regions of a human antibody. Chimeric antibodies, due to their significant non-human protein content, can induce a strong immune response. This issue can be overcome by using humanized antibodies. To avoid the decrease in activity caused by the decrease in immunogenicity, the variable regions of a fully human antibody can be subjected to minimum reverse mutation to maintain activity. Examples of "humanization" include "humanization" of VHH domains derived from camelidae by replacing one or more amino acid residues in the amino acid sequence of the original VHH sequence with one or more amino acid residues present at the corresponding positions in a VH domain of a human conventional antibody with a four-peptide-chain structure (also referred to in the present disclosure as "sequence optimization"; in addition to humanization, "sequence optimization" may also encompass other modifications to the sequence by one or more mutations providing improved properties of the VHH, such as removal of potential post-translational modification sites). The humanized VHH domain may contain one or more fully human framework region sequences. Additionally, in order to avoid the decrease in activity caused by the decrease in immunogenicity, the framework region sequence in the human antibody variable region can be subjected to minimum reverse mutation or back mutation to maintain activity.

"Fully human antibody" or "Fully human-derived antibody" includes antibodies having variable and constant regions derived from human germline immunoglobulin sequences. The fully human antibody of the present disclosure may include amino acid residues not encoded by human germline immunoglobulin sequences (e.g., mutations introduced by random or site-specific mutagenesis *in vitro* or by somatic mutations *in vivo*)*.* "Fully human antibody" does not include "humanized antibodies".

"Compete", when used in a case where antigen-binding proteins (e.g., neutralizing antigen-binding proteins or neutralizing antibodies) compete for the same epitope, refers to the competition between the antigen-binding proteins, which is measured by the following assays in which a test antigen-binding protein (e.g., an antibody or an immunologically functional fragment thereof) prevents or inhibits (e.g., reduces) specific binding of a reference antigen-binding protein (e.g., a ligand or a reference antibody) to a common antigen. Numerous types of competitive binding assays are available for determining whether an antigen-binding protein competes with another, such as solid phase direct or indirect radioimmunoassay (RIA), solid phase direct or indirect enzyme immunoassay (EIA), and sandwich competition assay (see, e.g., Stahli et al., 1983, Methods in Enzymology 9: 242-253); solid phase direct biotin-avidin EIA (see, e.g., Kirkland et al., 1986, J. Immunol. 137: 3614-3619), solid phase direct labeled assay, and solid phase direct labeled sandwich assay (see, e.g., Harlow and Lane, 1988, Antibodies: A Laboratory Manual, Cold Spring Harbor Press); solid phase direct labeled RIA with I-125 label (see, e.g., Morel et al., 1988, Molec. Immunol. 25: 7-15); solid phase direct biotin-avidin EIA (see, e.g., Cheung, et al., 1990, Virology 176: 546-552) and direct labeled RIA (Moldenhauer et al., 1990, Scand. J. Immunol. 32: 77-82). Generally, the assay relates to the use of a purified antigen (which is on a solid surface or cell surface) that binds to an unlabeled detection antigen-binding protein and a labeled reference antigen-binding protein. Competitive inhibition is measured by measuring the amount of label bound to the solid surface or the cell in the presence of the test antigen-binding protein. Generally, the test antigen-binding protein exists in an excessive amount. Antigen-binding proteins identified by competitive assays (competitive antigen-binding proteins) include: antigen-binding proteins that bind to the same epitope as the reference antigen-binding protein; and antigen-binding proteins that bind to an epitope that is sufficiently close to the epitope to which the reference antigen-binding protein binds, and the two epitopes spatially hinder each other from binding. Generally, when the competitive antigen-binding protein exists in an excessive amount, the specific binding of the reference antigen-binding protein to the common antigen will be inhibited (e.g., reduced) by at least 40%-45%, 45%-50%, 50%-55%, 55%-60%, 60%-65%, 65%-70%, 70%-75% or 75% or more. In certain instances, the binding is inhibited by at least 80%-85%, 85%-90%, 90%-95%, 95%-97% or 97% or more.

Antibodies can be competitively screened for binding to the same epitope using conventional techniques known to those skilled in the art. For example, competition and cross-competition studies can be performed to obtain antibodies that compete or cross-compete with one another for binding to an antigen. A high-throughput method for obtaining antibodies that bind to the same epitope based on their cross-competition is described in International Patent Publication No. WO03/48731. Therefore, an antibody that competes for binding to the same epitope on the antigen protein with the antibody molecule of the present disclosure can be obtained by conventional techniques known to those skilled in the art.

The term "glycoprotein-A repetitions predominant (GARP)" is a protein with a single-pass transmembrane structure, and "GARP" is also known as leucine-rich repeat-containing 32 (LRRC32) and belongs to the leucine-rich repeat family. The GARP can be expressed on the cell surface of activated Tregs and forms a complex with a TGF-β precursor (e.g., latent TGF-β). The GARP sequence can be obtained from GenBank, UniProt, etc., such as human GARP (LRRC32, Uniprot: Q14392) and murine GARP (LRRC32, Uniprot: G3XA59).

The GARP should be understood in its broadest sense within the scope of the present disclosure. The term encompasses natural forms and naturally occurring variants of GARP in nature, also including artificially expressed forms, functional variants, etc. The GARP encompasses the category of intact proteins, extracellular domains, and epitopes thereof in cases where antigen-antibody interactions are involved, unless otherwise specified in the context.

The term "GARP-TGFβ1 complex", "GARP/TGFβ1 protein", or "GARP/TGFB1" refers to a protein complex comprising a precursor protein of transforming growth factor-β1 (TGFβ1) protein and glycoprotein-A repetitions predominant (GARP). In some embodiments, the pro-protein form or latent form of TGFβ1 protein can be referred to as "pro/latent TGFβ1 protein". In some embodiments, the GARP-TGFβ1 complex comprises GARP covalently linked to a TGFβ1 precursor protein (pro/latent TGFβ1 protein) by one or more disulfide bonds. In other embodiments, the GARP-TGFβ1 complex comprises GARP non-covalently linked to a TGFβ1 precursor protein (pro/latent TGFβ1 protein). In some embodiments, the GARP-TGFβ1 complex is a naturally occurring complex, such as a GARP-TGFβ1 complex in a cell.

The term "LTBP" refers to a latent transforming growth factor β binding protein. LTBP is an important component of the extracellular matrix (ECM), and its main function is related to the regulation of a fibrillin and transforming growth factor-β (TGF-β). In mammals, there are four known LTBPs (LTBP1-4), each of which has multiple splice variants (Robertson, I. B. et al., Matrix Biol, 2015. 47: p. 44-53).

The term "GARP-TGFβ1 complex", "GARP/TGFβ1 protein", or "GARP/TGFB1" refers to a protein complex comprising a precursor protein form or latent form of transforming growth factor-β1 (TGFβ1) protein and glycoprotein-A repetitions predominant (GARP). In some embodiments, the pro-protein form or latent form of TGFβ1 protein can be referred to as "pro/latent TGFβ1 protein". In some embodiments, the GARP-TGFβ1 complex comprises GARP covalently linked to a TGFβ1 precursor protein (pro/latent TGFβ1 protein) by one or more disulfide bonds. In other embodiments, the GARP-TGFβ1 complex comprises GARP non-covalently linked to a TGFβ1 precursor protein (pro/latent TGFβ1 protein). In some embodiments, the GARP-TGFβ1 complex is a naturally occurring complex, such as a GARP-TGFβ1 complex in a cell.

The term "LTBP1-TGFβ1 complex" refers to a protein complex comprising a precursor protein of transforming growth factor-β1 (TGFβ1) protein and a latent TGFβ-binding protein (e.g., LTBP1 and LTBP3). In some embodiments, the LTBP-TGFβ1 complex comprises LTBP1 covalently linked to a TGFβ1 precursor protein by one or more disulfide bonds. In other embodiments, the LTBP1-TGFβ1 complex comprises LTBP1 non-covalently linked to a TGFβ1 precursor protein. In some embodiments, the LTBP1-TGFβ1 complex is a naturally occurring complex, such as an LTBP1-TGFβ1 complex in a cell.

The term "antigen" refers to a molecule used for immunization of an immunocompetent vertebrate to produce an antibody that recognizes the antigen or to screen an expression library (e.g., particularly phage, yeast, or ribosome display library). In the present disclosure, the antigen is defined in a broader sense, and includes a target molecule that is specifically recognized by the antibody, and a portion or a mimic of a molecule used in an immunization process for producing the antibody or in library screening for selecting the antibody. For example, for the antibody of the present disclosure that binds to a human GARP-TGFβ1 complex, truncated variants and other variants of the human GARP-TGFβ1 complex are all referred to as antigens.

The term "epitope" refers to a site on an antigen to which an immunoglobulin or an antibody binds. An epitope may be formed from contiguous amino acids, or non-contiguous amino acids juxtaposed by tertiary folding of the protein. An epitope formed from contiguous amino acids is generally retained after exposure to a denaturing solvent, while an epitope formed by tertiary folding is generally lost after a denaturing solvent treatment. An epitope generally comprises, for example, at least 3-15 amino acids in a unique spatial conformation. Methods for determining what epitope is bound by a given antibody are well known in the art and include an immunoblotting assay, an immunoprecipitation assay, and the like. Methods for determining the spatial conformation of an epitope include techniques in the art and techniques described in the present disclosure, such as X-ray crystallography and two-dimensional nuclear magnetic resonance.

"Specific binding" or "selective binding" refers to binding of an antibody to an epitope on a predetermined antigen. For example, an antibody binds to a predetermined antigen or epitope thereof with an equilibrium dissociation constant (K_{D}) of about less than 10⁻⁷ M or even less and with an affinity that is at least twice as high as its affinity for binding to a non-specific antigen other than the predetermined antigen or the epitope thereof (or non-specific antigens other than closely related antigens, e.g., BSA, etc.), when measured by surface plasmon resonance (SPR) techniques in an instrument using a human GARP-TGFβ1 complex or an epitope thereof as an analyte and the antibody as a ligand. "Antigen-recognizing antibody" is used interchangeably in the present disclosure with "specifically bound antibody".

"Binding affinity" or "affinity" is used in the present disclosure as a measure of the strength of a non-covalent interaction between two molecules (e.g., an antibody or a portion thereof and an antigen). The binding affinity between two molecules can be quantified by determining the dissociation constant (KD). KD can be determined by measuring the kinetics of complex formation and dissociation using, for example, the surface plasmon resonance (SPR) method (Biacore). The rate constants corresponding to the association and dissociation of a monovalent complex are referred to as the association rate constant ka (or kon) and the dissociation rate constant kd (or koff), respectively. K_{D} is related to ka and kd by the equation K_{D} = kd/ka. The value of the dissociation constant can be determined directly by well-known methods and can be calculated by methods such as those described by Caceci et al (1984, Byte 9:340-362) even for complex mixtures. For example, K_{D} can be determined by using a dual filtration nitrocellulose filter binding assay such as that disclosed by Wong & Lohman (1993, Proc. Natl. Acad. Sci. USA 90:5428-5432). Other standard assays for evaluating the binding ability of an antibody to a target antigen are known in the art and include, for example, ELISA, western blot, RIA, and flow cytometry, as well as other assays exemplified elsewhere in the present disclosure. The binding kinetics and binding affinity of the antibody can also be evaluated by standard assays known in the art, such as surface plasmon resonance (SPR), e.g., by using the Biacore^{™} system or KinExA. The binding affinities associated with different molecular interactions, e.g., the binding affinities of different antibodies for a given antigen, can be compared by comparing the K_{D} values of antibody/antigen complexes. Similarly, the specificity of an interaction can be evaluated by determining and comparing the K_{D} value for the interaction of interest (e.g., a specific interaction between an antibody and an antigen) with the K_{D} value for an interaction not of interest (e.g., a control antibody known not to bind to the antigen).

The term "conservative substitution" or "conservative replacement" refers to replacement by another amino acid residue having similar properties to the original amino acid residue. For example, lysine, arginine, and histidine have similar properties in that they have basic side chains, and aspartic acid and glutamic acid have similar properties in that they have acidic side chains. In addition, glycine, asparagine, glutamine, serine, threonine, tyrosine, cysteine, and tryptophan have similar properties in that they have uncharged polar side chains, and alanine, valine, leucine, threonine, isoleucine, proline, phenylalanine, and methionine have similar properties in that they have nonpolar side chains. In addition, tyrosine, phenylalanine, tryptophan, and histidine have similar properties in that they have aromatic side chains. Thus, it will be apparent to those skilled in the art that even when an amino acid residue in a group exhibiting similar properties as described above is replaced, it will not exhibit a particular change in properties.

"Homology", "identity", or "sequence identity" refers to sequence similarity between two polynucleotide sequences or between two polypeptides. When positions in two compared sequences are occupied by identical nucleotides or amino acid monomers, e.g., if the position of each of two DNA molecules is occupied by an identical nucleotide, the molecules are homologous at that position. The homology percentage between two sequences is a function of the number of matching or homologous positions shared by the two sequences divided by the number of positions compared × 100%. For example, if 6 out of 10 positions are matched or homologous when two sequences are optimally aligned, the two sequences are 60% homologous. In general, when two sequences are aligned, a comparison is performed to obtain the maximum homology percentage.

The terms "nucleic acid molecule" and "polynucleotide" are used interchangeably and refer to a DNA molecule and an RNA molecule. The nucleic acid molecule may be single-stranded or double-stranded, and is preferably double-stranded DNA. A nucleic acid is "operably linked" when it is placed into a functional relationship with another nucleic acid sequence. For example, a promoter or enhancer is operably linked to a coding sequence if it affects the transcription of the coding sequence.

The term "host cell" includes individual cells or cell cultures which may be or have been the recipient of a vector for incorporation of a polynucleotide insert. The host cell includes progeny of a single host cell, and the progeny may not necessarily be completely identical (in morphology or genomic DNA complement) to the original parent cell due to natural, accidental, or deliberate mutations. The host cell includes cells transfected and/or transformed *in vivo* with the polynucleotide of the present disclosure. "Cell", "cell line", and "cell culture" are used interchangeably, and all such designations include their progeny. It should also be understood that all progeny may not be precisely identical in DNA content due to intentional or unintentional mutations. Mutant progeny with identical function or biological activity as those screened in the initially transformed cells are included.

The terms "inhibition" and "blocking" are used interchangeably and encompass both partial and complete inhibition/blocking. For example, "inhibitory antibody" refers to an antibody that inhibits mature growth factor release or reduces growth factor activity. Inhibitory antibodies include antibodies targeting any epitope that reduces growth factor release or activity when binding to such antibodies. Such epitopes may be located on prodomains of TGFβ proteins (e.g., TGFβ1), growth factors, or other epitopes that lead to reduced growth factor activity when bound by antibodies. Inhibitory antibodies of the present disclosure include, but are not limited to, TGFβ1-inhibiting antibodies.

"Effective amount" comprises an amount sufficient to ameliorate or prevent a symptom or sign of a medical disorder. An effective amount also means an amount sufficient to allow or facilitate diagnosis. The effective amount for a subject may vary depending on factors such as the disorder to be treated, the general health of the subject, the method and dose of administration, and the severity of side effects. An effective amount may be the maximum dose or administration regimen to avoid significant side effects or toxic effects. The subject of the present disclosure may be an animal or a human subject.

The term "pharmaceutical composition" refers to a mixture containing one or more of the active ingredients or the physiologically/pharmaceutically acceptable salts or pro-drugs thereof described herein and other chemical components, as well as other components such as physiologically/pharmaceutically acceptable carriers and excipients. The pharmaceutical composition is intended to promote administration to an organism and facilitate the absorption of the active ingredient so that it can exert its biological activity.

The term "pharmaceutically acceptable carrier" or "pharmaceutically acceptable excipient" includes any material that, when combined with an active ingredient, allows the ingredient to retain biological activity and is non-reactive with the immune system of a subject. Examples include, but are not limited to, any standard pharmaceutical carrier, such as phosphate-buffered saline solutions, water, emulsions such as oil/water emulsions, and various types of wetting agents. In some examples, the diluent for aerosol or parenteral administration is phosphate-buffered saline (PBS) or normal (0.9%) saline. Compositions containing such carriers are formulated by well-known conventional methods (see, e.g., Remington's Pharmaceutical Sciences, 18th edition, A. Gennaro, eds., Mack Publishing Co., Easton, PA, 1990; and R Remington, The Science and Practice of Pharmacy, 20th edition, Mack Publishing, 2000).

"Cancer", "cancerous", "proliferative disorder", and "tumor" are not mutually exclusive when referred to in the present disclosure.

The terms "giving", "administering", and "treating", when applied to animals, humans, experimental subjects, cells, tissues, organs, or biological fluid, refer to the contact of an exogenous drug, a therapeutic agent, a diagnostic agent, or a composition with the animals, humans, subjects, cells, tissues, organs or biological fluid, e.g., therapeutic, pharmacokinetic, diagnostic, research, and experimental methods. The treatment of cells comprises contacting a reagent with cells and contacting the reagent with a fluid, where the fluid is in contact with the cells. "Giving", "administering", and "treating" also refer to treating, e.g., a cell, by a reagent, diagnosis, a binding composition, or by another cell *in vitro* and *ex vivo.* When applied to humans, veterinary medicine, or research subjects, they refer to therapeutic treatment, preventive or prophylactic measures, and research and diagnostic applications.

The term "treat", "treated", "treating", or "treatment" refers to administering a therapeutic agent, such as a therapeutic agent comprising any one of the fusion proteins or the insulin analogs of the present disclosure, to a subject who has had, is suspected of having, or is predisposed to having one or more types of diabetes or hyperglycemia-related diseases or symptoms thereof on which the therapeutic agent is known to have a therapeutic effect. Generally, the therapeutic agent is administered in an amount effective to alleviate one or more disease symptoms in the subject or population being treated. This is achieved by preventing or delaying the onset of the symptoms or complications, alleviating the symptoms or complications, or eliminating the disease, condition, or disorder to any clinically measurable degree. The amount of therapeutic agent effective to alleviate any specific disease symptom (also referred to as "therapeutically effective amount") may vary depending on factors such as the disease state, the age, and the body weight of the subject, and the capability of the drug to produce a desired therapeutic effect in the subject. Whether a disease symptom has been alleviated can be evaluated by any clinical testing methods commonly used by doctors or other health care professionals to evaluate the severity or progression of the symptom. Although embodiments of the present disclosure (e.g., treatment methods or products) may be ineffective in alleviating a disease symptom of interest in a certain subject, they shall alleviate the disease symptom of interest in a statistically significant number of subjects as determined by any statistical test method known in the art, such as the Student's t-test, Chi-square test, U-test by Mann and Whitney, Kruskal-Wallis test (H-test), Jonckheere-Terpstra test and Wilcoxon test. The patient to be treated is a mammal, preferably a human.

The term "prevent", "prevented", "preventing", or "prevention" refers to reducing the risk or incidence of one or more conditions, symptoms, complications, or disorders, or eliminating or slowing the progression of one or more conditions, symptoms, complications, or disorders.

The term "subject" or "patient" means a mammal, particularly a primate, and particularly a human.

### BRIEF DESCRIPTION OF THE DRAWINGS

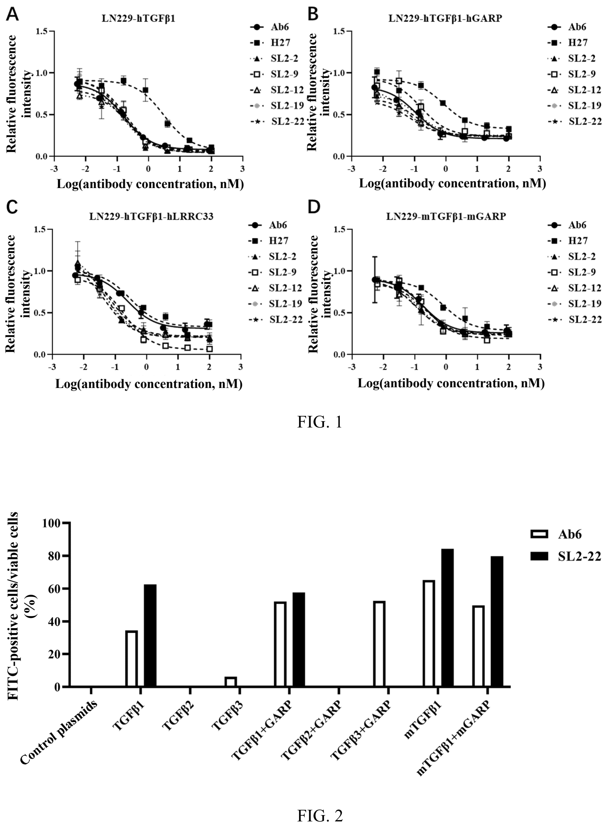

FIG. 1 shows the results of assessing the inhibition of the activity of human or mouse TGFβ1 complexes (human proTGFβ1, a GARP-TGFβ1 complex, an LRRC33-TGFβ1 complex, and a murine GARP-TGFβ1 complex) by anti-TGFβ1 antibodies (H27, SL2-2, SL2-9, SL2-12, SL2-19, and SL2-22). In FIG. 1: A shows the inhibitory effects of the antibodies on human proTGFβ1 activation, B shows the inhibitory effects of the antibodies on human GARP-TGFβ1 complex activation, C shows the inhibitory effects of the antibodies on human LRRC33-TGFβ1 complex activation, and D shows the inhibitory effects of the antibodies on murine GARP-TGFβ1 complex activation.
FIG. 2 shows the results of assessing the binding of anti-TGFβ1 antibodies (SL2-22 and Ab6) to HEK293E cells expressing human or mouse pro-TGFβ1, TGFβ2, TGFβ3, and TGFβ complexes (GARP-TGFβ1 complexes, a GARP-TGFβ2 complex, and a GARP-TGFβ3 complex).

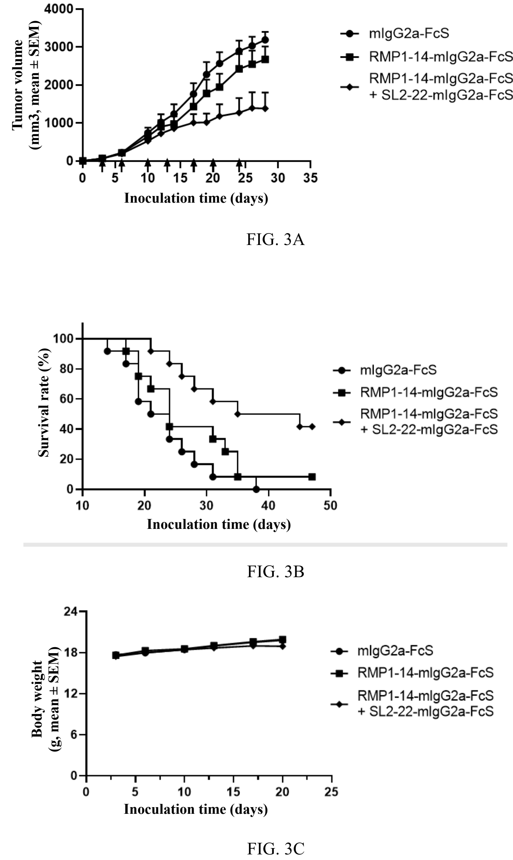

FIGs. 3A-3C show the results of the inhibition of tumor growth in a mouse EMT-6 model by the combination of an anti-TGFβ1 antibody and an anti-PD-1 antibody (RMP1-14-mIgG2a-FcS + SL2-22-mIgG2a-FcS). FIG. 3A shows mouse tumor volume curves, FIG. 3B shows mouse survival rate curves, and FIG. 3C shows mouse body weight curves.

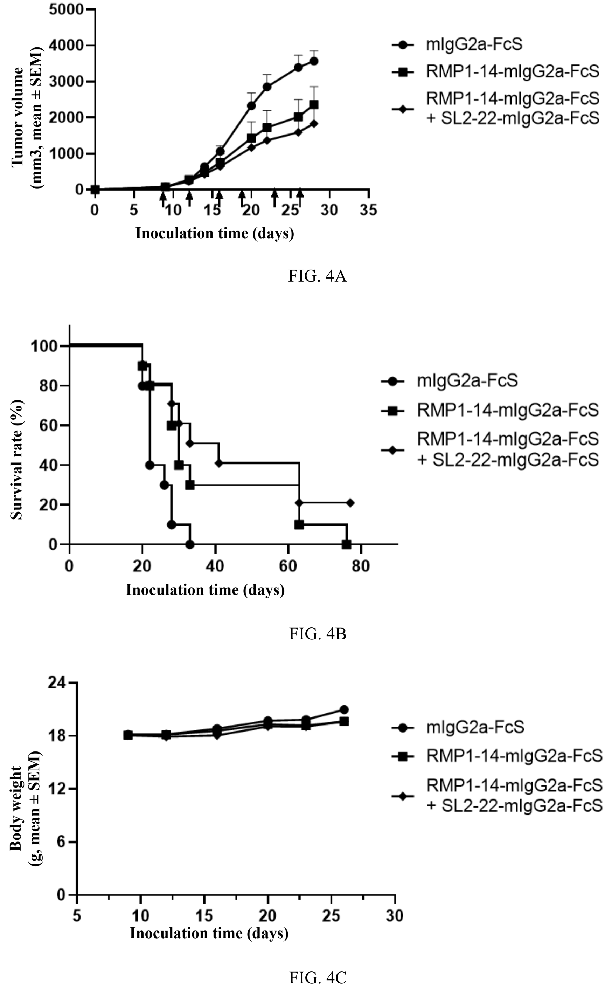

FIGs. 4A-4C show the results of the inhibition of tumor growth in a mouse CT26 model by the combination of an anti-TGFβ1 antibody and an anti-PD-1 antibody (RMP1-14-mIgG2a-FcS + SL2-22-mIgG2a-FcS). FIG. 4A shows mouse tumor volume curves, FIG. 4B shows mouse survival rate curves, and FIG. 4C shows mouse body weight curves.

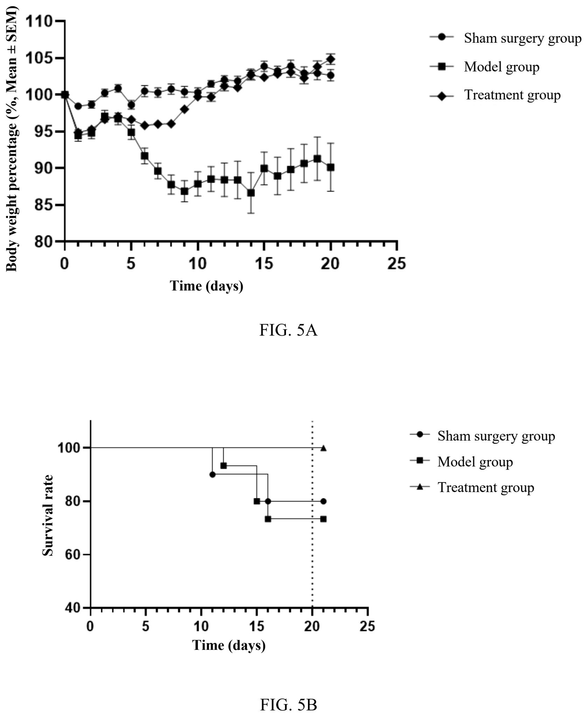

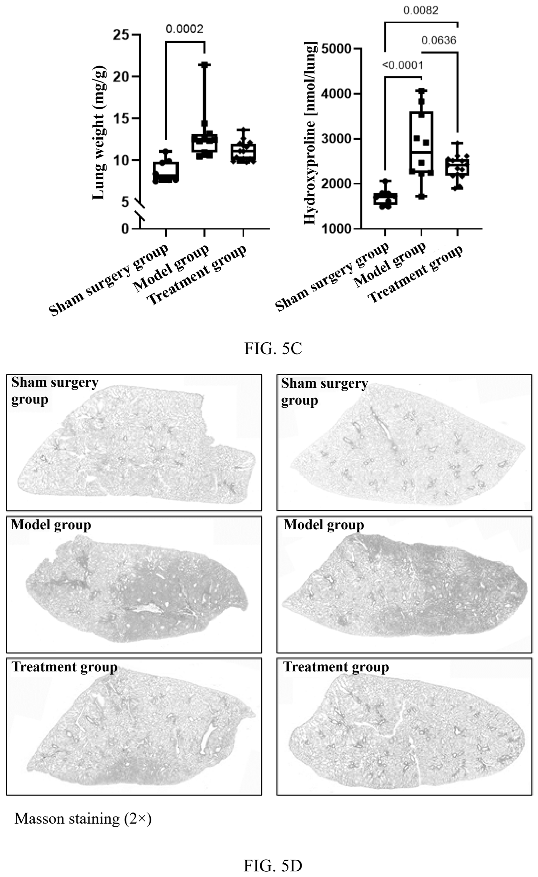

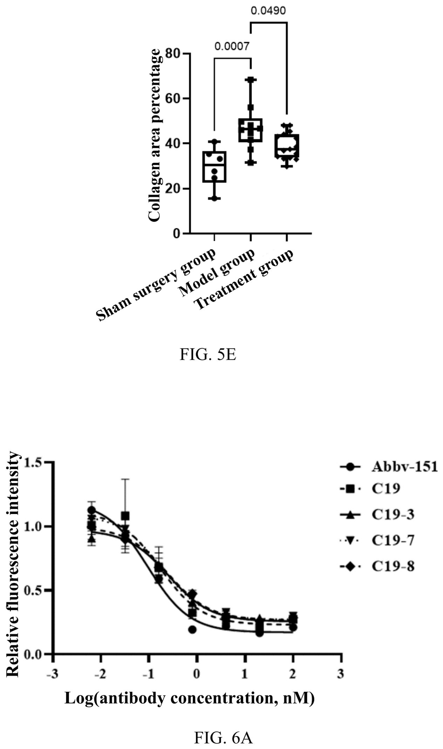

FIGs. 5A-5E show the results of the inhibition of fibrosis by an anti-TGFβ1 antibody (SL2-22-mIgG2a-FcS) in an idiopathic fibrosis mouse model. FIG. 5A shows body weight change curves of pulmonary fibrosis model mice, FIG. 5B shows survival rate curves of pulmonary fibrosis model mice, FIG. 5C shows the lung weight and the hydroxyproline content in lung tissue of pulmonary fibrosis model mice at the endpoint of the experiment, FIG. 5D shows lung sections of pulmonary fibrosis model mice at the endpoint of the experiment, and FIG. 5E shows Masson staining collagen area ratios.

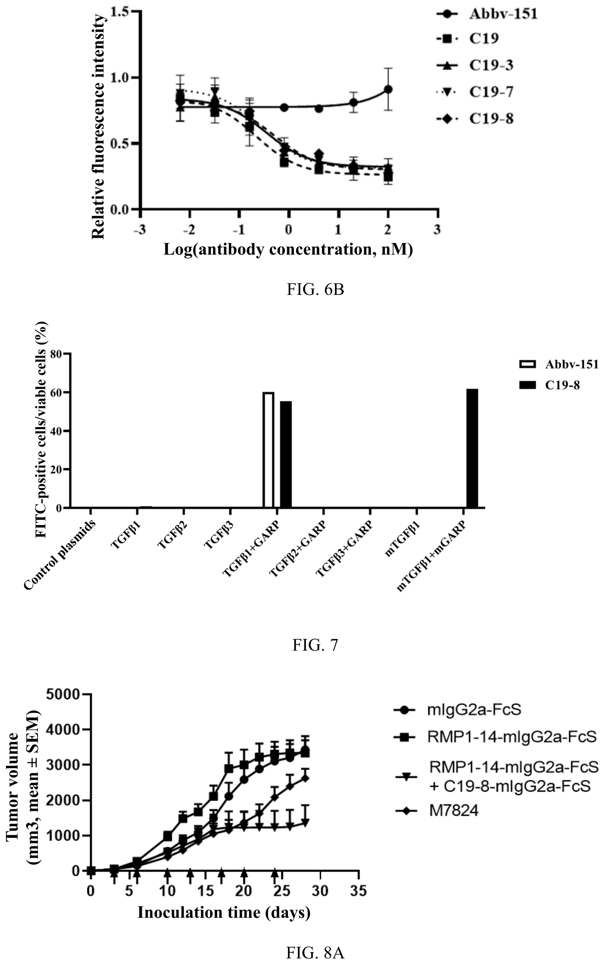

FIG. 6 shows an assessment of the inhibition of GARP-TGFβ1 complex activation by anti-GARP-TGFβ1 single-domain antibodies through an LN229 experiment. LN229 cells were transfected with human or murine pro-TGFβ1 and human or murine GARP expression plasmids, and the inhibition of human or murine GARP-TGFβ1 complex activation by Abbv-151, C19, C19-3, C19-7, and C19-8 was assessed using HepG2 CAGA12-luc luciferase reporter cells. FIG. 6A shows the inhibitory effects of the antibodies on human GARP-TGFβ1 complex activation, and FIG. 6B shows the inhibitory effects of the antibodies on murine GARP-TGFβ1 complex activation.
FIG. 7 shows the results of the binding of C19-8 to HEK293E cells expressing the human or mouse GARP-TGFβ1 complex. HEK293E cells were transfected with the plasmids in Table 3, and C19-8 and Abbv-151 were added. Staining and detection were performed, and the results were obtained.

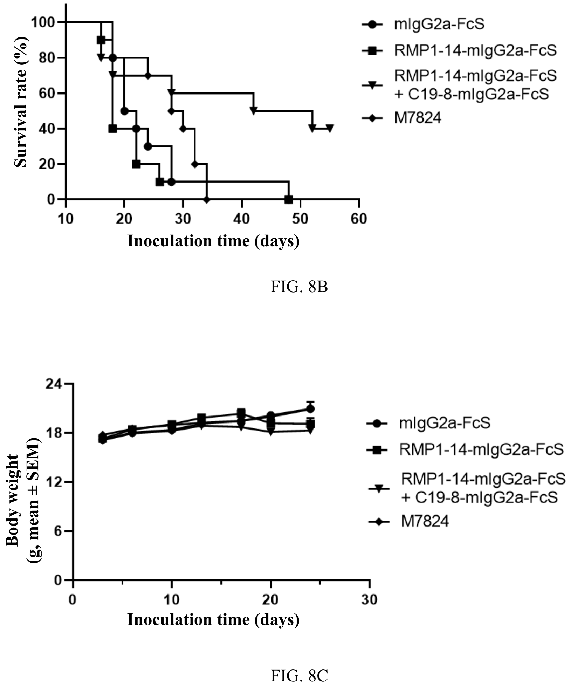

FIGs. 8A-8C show the results of the inhibition of tumor growth in a mouse EMT-6 model by the combination of C19-8 and an anti-PD-1 antibody (RMP1-14-mIgG2a-FcS + C19-8-mIgG2a-FcS). FIG. 8A shows mouse tumor volume curves, FIG. 8B shows mouse survival rate curves, and FIG. 8C shows mouse body weight curves.

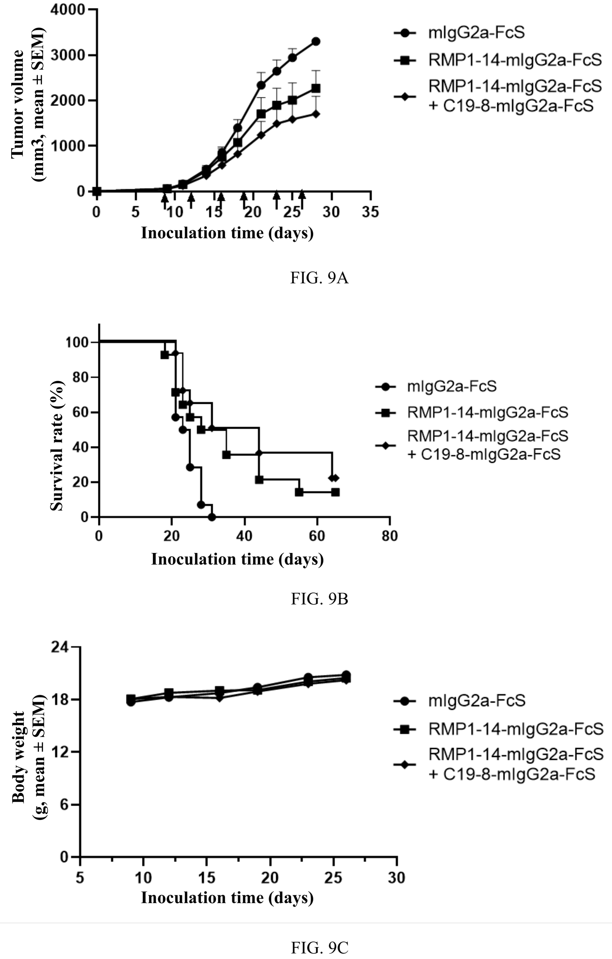

FIGs. 9A-9C show the results of the inhibition of tumor growth in a mouse CT26 model by the combination of C19-8 and an anti-PD-1 antibody (RMP1-14-mIgG2a-FcS + C19-8-mIgG2a-FcS). FIG. 9A shows mouse tumor volume curves, FIG. 9B shows mouse survival rate curves, and FIG. 9C shows mouse body weight curves.

### DETAILED DESCRIPTION

### Examples

The present disclosure is further described with reference to the following examples; however, these examples are not intended to limit the scope of the present disclosure.

Experimental procedures without specific conditions indicated in the examples or test examples of the present disclosure are generally conducted under conventional conditions, or under conditions recommended by the manufacturers of the starting materials or commercial products. See Sambrook et al., Molecular Cloning: A Laboratory Manual, Cold Spring Harbor Laboratory Press; Current Protocols in Molecular Biology, Ausubel et al., Greene Publishing Association, Wiley Interscience, NY. Reagents without specific origins indicated were commercially available conventional reagents.

### Example 1. Preparation of Antigens and Proteins for Detection

The amino acid sequences of related proteins for screening and detection were designed using human pro-TGFβ1 (Uniprot: P01137), human GARP (LRRC32, Uniprot: Q14392), murine pro-TGFβ1 (Uniprot: P04202), and murine GARP (LRRC32, Uniprot: G3XA59) as templates.
> Human pro-TGFβ1 full-length amino acid sequence: 
MPPSGLRLLLLLLPLLWLLVLTPGRPAAGLSTCKTIDMELVKRKRIEAIRGQILSKLR
LASPPSQGEVPPGPLPEAVLALYNSTRDRVAGESAEPEPEPEADYYAKEVTRVLM
VETHNEIYDKFKQSTHSIYMFFNTSELREAVPEPVLLSRAELRLLRLKLKVEQHV
ELYQKYSNNSWRYLSNRLLAPSDSPEWLSFDVTGVVRQWLSRGGEIEGFRLSA
HCSCDSRDNTLQVDINGFTTGRRGDLATIHGMNRPFLLLMATPLERAQHLQSSR
HRRALDTNYCFSSTEKNCCVRQLYIDFRKDLGWKWIHEPKGYHANFCLGPCPY
IWSLDTQYSKVLALYNQHNPGASAAPCCVPQALEPLPIVYYVGRKPKVEQLSNM
IVRSCKCS

(SEQ ID NO: 1)
 (Note: The signal peptide is *in italics;* the latency-associated peptide (LAP) is underlined; the TGFβ1 domain is ***in italics and bold***
> Mature human TGFβ1 (mature transforming growth factor beta-1): 
ALDTNYCFSSTEKNCCVRQLYIDFRKDLGWKWIHEPKGYHANFCLGPCPYIWS
LDTQYSKVLALYNQHNPGASAAPCCVPQALEPLPIVYYVGRKPKVEQLSNMIVR
SCKCS

(SEQ ID NO: 101)
> Human LAP: 
LSTCKTIDMELVKRKRIEAIRGQILSKLRLASPPSQGEVPPGPLPEAVLALYNSTR
DRVAGESAEPEPEPEADYYAKEVTRVLMVETHNEIYDKFKQSTHSIYMFFNTSE
 
LREAVPEPVLLSRAELRLLRLKLKVEQHVELYQKYSNNSWRYLSNRLLAPSDSP
EWLSFDVTGVVRQWLSRGGEIEGFRLSAHCSCDSRDNTLQVDINGFTTGRRGD
LATIHGMNRPFLLLMATPLERAQHLQSSRHRR
(SEQ ID NO: 102)
> Human GARP full-length amino acid sequence: 
MRPQILLLLALLTLGLAAQHQDKVPCKMVDKKVSCQVLGLLQVPSVLPPDTETL
DLSGNQLRSILASPLGFYTALRHLDLSTNEISFLQPGAFQALTHLEHLSLAHNRL
AMATALSAGGLGPLPRVTSLDLSGNSLYSGLLERLLGEAPSLHTLSLAENSLTRL
TRHTFRDMPALEQLDLHSNVLMDIEDGAFEGLPRLTHLNLSRNSLTCISDFSLQQ
LRVLDLSCNSIEAFQTASQPQAEFQLTWLDLRENKLLHFPDLAALPRLIYLNLSN
NLIRLPTGPPQDSKGIHAPSEGWSALPLSAPSGNASGRPLSQLLNLDLSYNEIELI
PDSFLEHLTSLCFLNLSRNCLRTFEARRLGSLPCLMLLDLSHNALETLELGARAL
GSLRTLLLQGNALRDLPPYTFANLASLQRLNLQGNRVSPCGGPDEPGPSGCVAF
SGITSLRSLSLVDNEIELLRAGAFLHTPLTELDLSSNPGLEVATGALGGLEASLEV
LALQGNGLMVLQVDLPCFICLKRLNLAENRLSHLPAWTQAVSLEVLDLRNNSFS
LLPGSAMGGLETSLRRLYLQGNPLSCCGNGWLAAQLHQGRVDVDATQDLICRF
SSQEEVSLSHVRPEDCEKGGLKNINLIIILTFILVSAILLTTLAACCCVRRQKFNQQ
YKA

(SEQ ID NO: 2)
 (Note: The signal peptide is *in italics;* the extracellular domain is underlined; the transmembrane domain is ***in italics and bold*;** the cytoplasmic domain is *in italics and underlined*)
> Human GARP extracellular domain: 
HQDKVPCKMVDKKVSCQVLGLLQVPSVLPPDTETLDLSGNQLRSILASPLGFYT
ALRHLDLSTNEISFLQPGAFQALTHLEHLSLAHNRLAMATALSAGGLGPLPRVTS
LDLSGNSLYSGLLERLLGEAPSLHTLSLAENSLTRLTRHTFRDMPALEQLDLHSN
VLMDIEDGAFEGLPRLTHLNLSRNSLTCISDFSLQQLRVLDLSCNSIEAFQTASQP
QAEFQLTWLDLRENKLLHFPDLAALPRLIYLNLSNNLIRLPTGPPQDSKGIHAPS
EGWSALPLSAPSGNASGRPLSQLLNLDLSYNEIELIPDSFLEHLTSLCFLNLSRNC
LRTFEARRLGSLPCLMLLDLSHNALETLELGARALGSLRTLLLQGNALRDLPPY
TFANLASLQRLNLQGNRVSPCGGPDEPGPSGCVAFSGITSLRSLSLVDNEIELLRA
GAFLHTPLTELDLSSNPGLEVATGALGGLEASLEVLALQGNGLMVLQVDLPCFI
CLKRLNLAENRLSHLPAWTQAVSLEVLDLRNNSFSLLPGSAMGGLETSLRRLYL
QGNPLSCCGNGWLAAQLHQGRVDVDATQDLICRFSSQEEVSLSHVRPEDCEKG
GLKNIN

(SEQ ID NO: 100)
> Mouse pro-TGFβ1 full-length amino acid sequence: 
MPPSGLRLLPLLLPLPWLLVLTPGRPAAGLSTCKTIDMELVKRKRIEAIRGQILSKL
RLASPPSQGEVPPGPLPEAVLALYNSTRDRVAGESADPEPEPEADYYAKEVTRVL
MVDRNNAIYEKTKDISHSIYMFFNTSDIREAVPEPPLLSRAELRLQRLKSSVEQH
VELYQKYSNNSWRYLGNRLLTPTDTPEWLSFDVTGVVRQWLNQGDGIQGFRFS
AHCSCDSKDNKLHVEINGISPKRRGDLGTIHDMNRPFLLLMATPLERAQHLHSS
RHRRALDTNYCFSSTEKNCCVRQLYIDFRKDLGWKWIHEPKGYHANFCLGPCP
YIWSLDTQYSKVLALYNQHNPGASASPCCVPQALEPLPIVYYVGRKPKVEQLSN
MIVRSCKCS

(SEQ ID NO: 3)
 (Note: The signal peptide is *in italics;* the latency-associated peptide (LAP) is underlined; the TGFβ1 domain is ***in italics and bold***)
> Mature mouse TGFβ1: 
ALDTNYCFSSTEKNCCVRQLYIDFRKDLGWKWIHEPKGYHANFCLGPCPYIWS
LDTQYSKVLALYNQHNPGASASPCCVPQALEPLPIVYYVGRKPKVEQLSNMIVR
SCKCS

(SEQ ID NO: 104)
> Mouse LAP: 
LSTCKTIDMELVKRKRIEAIRGQILSKLRLASPPSQGEVPPGPLPEAVLALYNSTR
DRVAGESADPEPEPEADYYAKEVTRVLMVDRNNAIYEKTKDISHSIYMFFNTSDI
REAVPEPPLLSRAELRLQRLKSSVEQHVELYQKYSNNSWRYLGNRLLTPTDTPE
WLSFDVTGVVRQWLNQGDGIQGFRFSAHCSCDSKDNKLHVEINGISPKRRGDL
GTIHDMNRPFLLLMATPLERAQHLHSSRHRR

(SEQ ID NO: 105)
> Mouse GARP full-length amino acid sequence: 
MSHQILLLLAMLTLGLAISQRREQVPCRTVNKEALCHGLGLLQVPSVLSLDIQALY
LSGNQLQSILVSPLGFYTALRHLDLSDNQISFLQAGVFQALPYLEHLNLAHNRLA
TGMALNSGGLGRLPLLVSLDLSGNSLHGNLVERLLGETPRLRTLSLAENSLTRLA
RHTFWGMPAVEQLDLHSNVLMDIEDGAFEALPHLTHLNLSRNSLTCISDFSLQQ
LQVLDLSCNSIEAFQTAPEPQAQFQLAWLDLRENKLLHFPDLAVFPRLIYLNVSN
NLIQLPAGLPRGSEDLHAPSEGWSASPLSNPSRNASTHPLSQLLNLDLSYNEIELV
PASFLEHLTSLRFLNLSRNCLRSFEARQVDSLPCLVLLDLSHNVLEALELGTKVL
GSLQTLLLQDNALQELPPYTFASLASLQRLNLQGNQVSPCGGPAEPGPPGCVDF
SGIPTLHVLNMAGNSMGMLRAGSFLHTPLTELDLSTNPGLDVATGALVGLEASL
EVLELQGNGLTVLRVDLPCFLRLKRLNLAENQLSHLPAWTRAVSLEVLDLRNNS
FSLLPGNAMGGLETSLRRLYLQGNPLSCCGNGWLAAQLHQGRVDVDATQDLIC
RFGSQEELSLSLVRPEDCEKGGLKNVNLILLLSFTLVSAIVLTTLATICFLRRQKLS
QQYKA
 (SEQ ID NO: 4) (Note: The signal peptide is *in italics;* the extracellular domain is underlined; the transmembrane domain is ***in italics and bold***; the cytoplasmic domain is *in italics and underlined*)
> Mouse GARP extracellular domain:

ISQRREQVPCRTVNKEALCHGLGLLQVPSVLSLDIQALYLSGNQLQSILVSPLGF
YTALRHLDLSDNQISFLQAGVFQALPYLEHLNLAHNRLATGMALNSGGLGRLP
LLVSLDLSGNSLHGNLVERLLGETPRLRTLSLAENSLTRLARHTFWGMPAVEQL
DLHSNVLMDIEDGAFEALPHLTHLNLSRNSLTCISDFSLQQLQVLDLSCNSIEAF
QTAPEPQAQFQLAWLDLRENKLLHFPDLAVFPRLIYLNVSNNLIQLPAGLPRGSE
DLHAPSEGWSASPLSNPSRNASTHPLSQLLNLDLSYNEIELVPASFLEHLTSLRFL
NLSRNCLRSFEARQVDSLPCLVLLDLSHNVLEALELGTKVLGSLQTLLLQDNAL
QELPPYTFASLASLQRLNLQGNQVSPCGGPAEPGPPGCVDFSGIPTLHVLNMAG
NSMGMLRAGSFLHTPLTELDLSTNPGLDVATGALVGLEASLEVLELQGNGLTVL
RVDLPCFLRLKRLNLAENQLSHLPAWTRAVSLEVLDLRNNSFSLLPGNAMGGLE
TSLRRLYLQGNPLSCCGNGWLAAQLHQGRVDVDATQDLICRFGSQEELSLSLVR
PEDCEKGGLKNVN

(SEQ ID NO: 103)

The human GARP/TGFβ1 complex for screening and detection was prepared as follows: Human GARP-avi-his and human natural TGFβ1 (set forth in SEQ ID NOs: 5 and 6, respectively) were co-expressed in ExpiCHO cells (ThermoFisher, A29127) and purified to obtain the protein complex; the human TGFβ1 C4S protein is set forth in SEQ ID NO: 7. The mouse GARP/TGFβ1 complex was prepared as follows: Mouse GARP-avi-his and mouse natural TGFβ1 (set forth in SEQ ID NOs: 8 and 9, respectively) were co-expressed in ExpiCHO cells (ThermoFisher, A29127) and purified to obtain the protein complex; the mouse TGFβ1 C4S protein is set forth in SEQ ID NO: 10:
> Human GARP-avi-his amino acid sequence: 
HQDKVPCKMVDKKVSCQVLGLLQVPSVLPPDTETLDLSGNQLRSILASPLGFYT
ALRHLDLSTNEISFLQPGAFQALTHLEHLSLAHNRLAMATALSAGGLGPLPRVTS
LDLSGNSLYSGLLERLLGEAPSLHTLSLAENSLTRLTRHTFRDMPALEQLDLHSN
VLMDIEDGAFEGLPRLTHLNLSRNSLTCISDFSLQQLRVLDLSCNSIEAFQTASQP
QAEFQLTWLDLRENKLLHFPDLAALPRLIYLNLSNNLIRLPTGPPQDSKGIHAPS
EGWSALPLSAPSGNASGRPLSQLLNLDLSYNEIELIPDSFLEHLTSLCFLNLSRNC
LRTFEARRLGSLPCLMLLDLSHNALETLELGARALGSLRTLLLQGNALRDLPPY
TFANLASLQRLNLQGNRVSPCGGPDEPGPSGCVAFSGITSLRSLSLVDNEIELLRA
GAFLHTPLTELDLSSNPGLEVATGALGGLEASLEVLALQGNGLMVLQVDLPCFI
CLKRLNLAENRLSHLPAWTQAVSLEVLDLRNNSFSLLPGSAMGGLETSLRRLYL
 
QGNPLSCCGNGWLAAQLHQGRVDVATQDLICRFSSQEEVSLSHVRPEDCEKG
GLKNINGGGGSGGGGSGLNDIFEAQKIEWHEGGGGSGGGGSHHHHHHHH
(SEQ ID NO: 5)
 (Note: The human GARP extracellular domain is underlined; the (G₄S)₂ linkers are *in italics*; the Avi tag is dotted-underlined; the His tag is wavy-underlined)
> Human TGFβ1-natural amino acid sequence: 
LSTCKTIDMELVKRKRIEAIRGQILSKLRLASPPSQGEVPPGPLPEAVLALYNSTR
DRVAGESAEPEPEPEADYYAKEVTRVLMVETHNEIYDKFKQSTHSIYMFFNTSE
LREAVPEPVLLSRAELRLLRLKLKVEQHVELYQKYSNNSWRYLSNRLLAPSDSP
EWLSFDVTGVVRQWLSRGGEIEGFRLSAHCSCDSRDNTLQVDINGFTTGRRGD
LATIHGMNRPFLLLMATPLERAQHLQSSRHRRALDTNYCFSSTEKNCCVRQLYI
DFRKDLGWKWIHEPKGYHANFCLGPCPYIWSLDTQYSKVLALYNQHNPGASA
APCCVPQALEPLPIVYYVGRKPKVEQLSNMIVRSCKCS

(SEQ ID NO: 6)
> Human TGFβ1 C4S amino acid sequence: 
HHHHHHHHGGGGSGGGGSGLNDIFEAQKIEWHEGGGGSGGGGSLSTSKTIDME
LVKRKRIEAIRGQILSKLRLASPPSQGEVPPGPLPEAVLALYNSTRDRVAGESAEP
EPEPEADYYAKEVTRVLMVETHNEIYDKFKQSTHSIYMFFNTSELREAVPEPVLL
SRAELRLLRLKLKVEQHVELYQKYSNNSWRYLSNRLLAPSDSPEWLSFDVTGV
VRQWLSRGGEIEGFRLSAHCSCDSRDNTLQVDINGFTTGRRGDLATIHGMNRPF
LLLMATPLERAQHLQSSRHRRALDTNYCFSSTEKNCCVRQLYIDFRKDLGWKW
IHEPKGYHANFCLGPCPYIWSLDTQYSKVLALYNQHNPGASAAPCCVPQALEPL
PIVYYVGRKPKVEQLSNMIVRSCKCS

(SEQ ID NO: 7)
 (Note: The His tag is wavy-underlined; the (G₄S)₂ linkers are *in italics;* the Avi tag is dotted-underlined; the full-length human TGFβ1 protein is underlined, with the amino acid C at position four mutated into S)
> Mouse GARP-avi-his amino acid sequence: 
ISQRREQVPCRTVNKEALCHGLGLLQVPSVLSLDIQALYLSGNQLQSILVSPLGF
YTALRHLDLSDNQISFLQAGVFQALPYLEHLNLAHNRLATGMALNSGGLGRLP
LLVSLDLSGNSLHGNLVERLLGETPRLRTLSLAENSLTRLARHTFWGMPAVEQL
DLHSNVLMDIEDGAFEALPHLTHLNLSRNSLTCISDFSLQQLQVLDLSCNSIEAF
QTAPEPQAQFQLAWLDLRENKLLHFPDLAVFPRLIYLNVSNNLIQLPAGLPRGSE
DLHAPSEGWSASPLSNPSRNASTHPLSQLLNLDLSYNEIELVPASFLEHLTSLRFL
NLSRNCLRSFEARQVDSLPCLVLLDLSHNVLEALELGTKVLGSLQTLLLQDNAL
QELPPYTFASLASLQRLNLQGNQVSPCGGPAEPGPPGCVDFSGIPTLHVLNMAG
NSMGMLRAGSFLHTPLTELDLSTNPGLDVATGALVGLEASLEVLELQGNGLTVL
 
RVDLPCFLRLKRLNLAENQLSHLPAWTRAVSLEVLDLRNNSFSLLPGNAMGGLE
TSLRRLYLQGNPLSCCGNGWLAAQLHQGRVDVDATQDLICRFGSQEELSLSLVR
PEDCEKGGLKNVNGGGGSGGGGSGLNDIFEAQKIEWHEGGGGSGGGGSHHHH
HHHH

(SEQ ID NO: 8)
 (Note: The mouse GARP extracellular domain is underlined; the (G₄S)₂ linkers are *in italics;* the Avi tag is dotted-underlined; the His tag is wavy-underlined)
> Mouse TGFβ1-natural amino acid sequence: 
LSTCKTIDMELVKRKRIEAIRGQILSKLRLASPPSQGEVPPGPLPEAVLALYNSTR
DRVAGESADPEPEPEADYYAKEVTRVLMVDRNNAIYEKTKDISHSIYMFFNTSDI
REAVPEPPLLSRAELRLQRLKSSVEQHVELYQKYSNNSWRYLGNRLLTPTDTPE
WLSFDVTGVVRQWLNQGDGIQGFRFSAHCSCDSKDNKLHVEINGISPKRRGDL
GTIHDMNRPFLLLMATPLERAQHLHSSRHRRALDTNYCFSSTEKNCCVRQLYID
FRKDLGWKWIHEPKGYHANFCLGPCPYIWSLDTQYSKVLALYNQHNPGASASP
CCVPQALEPLPIVYYVGRKPKVEQLSNMIVRSCKCS

(SEQ ID NO: 9)
> Mouse TGFβ1 C4S amino acid sequence: 
HHHHHHHHGGGGSGGGGSGLNDIFEAQKIEWHEGGGGSGGGGSLSTSKTIDME
LVKRKRIEAIRGQILSKLRLASPPSQGEVPPGPLPEAVLALYNSTRDRVAGESADP
EPEPEADYYAKEVTRVLMVDRNNAIYEKTKDISHSIYMFFNTSDIREAVPEPPLL
SRAELRLQRLKSSVEQHVELYQKYSNNSWRYLGNRLLTPTDTPEWLSFDVTGV
VRQWLNQGDGIQGFRFSAHCSCDSKDNKLHVEINGISPKRRGDLGTIHDMNRPF
LLLMATPLERAQHLHSSRHRRALDTNYCFSSTEKNCCVRQLYIDFRKDLGWKW
IHEPKGYHANFCLGPCPYIWSLDTQYSKVLALYNQHNPGASASPCCVPQALEPL
PIVYYVGRKPKVEQLSNMIVRSCKCS

(SEQ ID NO: 10)
 (Note: The His tag is wavy-underlined; the (G₄S)₂ linkers are *in italics;* the Avi tag is dotted-underlined; the full-length mouse TGFβ1 protein is underlined, with the amino acid C at position four mutated into S)

The above sequences can all be obtained by expression, purification, and separation using conventional methods in the art.

### Example 2. Screening for Anti-TGFβ1 Monoclonal Antibodies Specifically Binding to Human TGFβ1 Complex

Antibodies with high affinities for a human TGFβ1 complex were obtained by screening human-derived antibody phage libraries. There were a total of four human-derived antibody phage libraries (1: a semi-synthetic human Fab library 1 (germline 3-23); 2: a semi-synthetic human Fab library 2 (germline 1-69); 3: a fully human-derived Naive Fab library; and 4: a fully human-derived scFV library), and all were gifts from Shanghai Hengrui Pharmaceuticals Co., Ltd. 10 µg of biotinylated human GARP-TGFβ1 complex (containing SEQ ID NOs: 5 and 6) protein was used to bind to 1 mg of Dynabeads M-280 streptavidin (Cat No. 11206D, Invitrogen). The mixture was left to react at room temperature for half an hour. The beads were washed 3 times with 1× PBS and then blocked with 2% skim milk at room temperature for 1 h. Meanwhile, the human-derived antibody phage display libraries were blocked and depleted using 2% skim milk and 1 mg of Dynabeads M-280 streptavidin. The treated phage libraries were added to the antigen-bound beads, and the mixtures were incubated at room temperature for 1 h. The beads were washed 10 times with a 1× PBST (containing 0.05% Tween-20) solution (pH 7.4) to remove non-binding phages. After another 2 washes with 1× PBS, the phages specifically binding to human GARP/TGFβ1 were eluted with 0.5 mL of trypsin (1 mg/mL) and used to infect *Escherichia coli* TG1 in the logarithmic growth phase. The bacteria were grown overnight on a 2YT (containing 2% glucose) resistant plate. Bacteria were scraped from the plate, and phages were produced and purified for use in the next round of screening.

The same screening process was repeated twice. To obtain antibodies with cross-binding to a mouse TGFβ1 complex, biotinylated mouse TGFβ1_C4S (SEQ ID NO: 10) was used as a screening antigen in the second round. In the third round, a biotinylated human GARP-TGFβ1 complex (containing SEQ ID NOs: 5 and 6) was used as a screening antigen. After the three rounds of screening, positive clones were enriched.

From the screening-enriched clones, 6 × 92 single clones were picked. These single clones were packaged into monoclonal phages for phage ELISA testing. ELISA plates (Cat No. 9018, Corning) were coated with 2 µg/mL human GARP-TGFβ1 complex (SEQ ID NOs: 5 and 6) and mouse TGFβ1_C4S (SEQ ID NO: 10) protein, respectively. The plates were left to stand overnight at 4 °C, washed 3 times with 1× PBST, and blocked with 2% BSA at 37 °C for 1 h. After 3 washes with PBST, a phage supernatant diluted with the blocking solution was added. After 1 h of reaction at room temperature and 6 washes with PBST, anti-M13 HRP (Cat No. 11973-MM05T-H, Sino Biological) was added. After 1 h of reaction at room temperature and 3 washes with PBST, 100 µL of TMB chromogenic substrate was added, and the reactions were stopped with 100 µL of 1 M sulfuric acid. The absorbance at 450 nm was measured using a SpectraMax M5 microplate reader. Clones with OD450 values greater than 3 times that of the negative control in the ELISA binding test were sequenced. According to analysis, 43 specific antibody sequences were obtained. SPR experiments showed that antibodies H5, H14, H16, H17, H19, H20, H21, H23, H24, H27, H28, H30, H32, H34, and H39 are capable of binding to the TGFβ1 complex. However, cell function experiments showed that only H27 has a relatively good inhibitory function, and it well binds to the human and mouse GARP-TGFβ1 complexes, as well as human and mouse TGFβ1_C4S (i.e., TGFβ1 precursor proteins).

The sequences of H27 are shown below.
> H27 VH 
EVQLLESGGGLVQPGGSLRLSCAASGFTFSSYAMSWVRQAPGKGLEWVSAISGSGG
STYYADSVKGRFTISRDNSKNTLYLQMNSLRAEDTAVYYCARVDSSGGYWYFDLW
GRGTLVTVSS

(SEQ ID NO: 11)
> H27 VL

DIQMTQSPSSLSASVGDRVTITCRASQSISSYLNWYQQKPGKAPKLLIYAASSLQSGV
PSRFSGSGSGTDFTLTISSLQPEDFATYYCQQSYSTPLTFGGGTKLEIK
(SEQ ID NO: 12)

In the above sequences SEQ ID NOs: 11 and 12, the order is FR1-CDR1-FR2-CDR2-FR3-CDR3-FR4. In the sequences, the FR sequences are in italics, and the underlined portions are the CDR1, CDR2, and CDR3 sequences, respectively. The numbering scheme for the human-derived antibodies provided in the present disclosure was Kabat. Table 5 shows CDR sequences.

**Table 5. The CDR sequences of antibody H27**

| Heavy chain | | Light chain | |
|---|---|---|---|
| HCDR1 | SYAMS SEQ ID NO: 13 | LCDR1 | RASQSISSYLN SEQ ID NO: 16 |
| HCDR2 | AISGSGGSTYYADSVKG SEQ ID NO: 14 | LCDR2 | AASSLQS SEQ ID NO: 17 |
| HCDR3 | VDSSGGYWYFDL SEQ ID NO: 15 | LCDR3 | QQSYSTPLT SEQ ID NO: 18 |

### Example 3. Construction, Expression, and Purification of Complete Monoclonal Antibody

The sequences of the H27 obtained by screening the human-derived antibody phage libraries in Example 2 were used to construct a complete recombinant antibody: the light chain variable region VL of the antibody was cloned into a pTT5 expression vector containing a human-derived kappa light chain constant region (SEQ ID NO: 19), and the heavy chain variable region VH of the antibody was cloned into a pTT5 expression vector containing a human IgG4-S228P (CH1-CH2-CH3) heavy chain constant region (SEQ ID NO: 20).
> Human kappa light chain constant region:  

(SEQ ID NO: 19)
> Human IgG4-S228P (CH1-CH2-CH3) heavy chain constant region:

ASTKGPSVFPLAPCSRSTSESTAALGCLVKDYFPEPVTVSWNSGALTSGVHTFPA
VLQSSGLYSLSSVVTVPSSSLGTKTYTCNVDHKPSNTKVDKRVESKYGPPCPPCP
APEFLGGPSVFLFPPKPKDTLMISRTPEVTCVVVDVSQEDPEVQFNWYVDGVEV
HNAKTKPREEQFNSTYRVVSVLTVLHQDWLNGKEYKCKVSNKGLPSSIEKTISK
AKGQPREPQVYTLPPSQEEMTKNQVSLTCLVKGFYPSDIAVEWESNGQPENNYK
TTPPVLDSDGSFFLYSRLTVDKSRWQEGNVFSCSVMHEALHNHYTQKSLSLSLG
K

(SEQ ID NO: 20)

The sequences of the complete antibody of H27 are shown below:
> H27 light chain: 
DIQMTQSPSSLSASVGDRVTITCRASQSISSYLNWYQQKPGKAPKLLIYAASSLQ
SGVPSRFSGSGSGTDFTLTISSLQPEDFATYYCQQSYSTPLTFGGGTKLEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC
(SEQ ID NO: 65)
> H27 heavy chain:

EVQLLESGGGLVQPGGSLRLSCAASGFTFSSYAMSWVRQAPGKGLEWVSAISGS
GGSTYYADSVKGRFTISRDNSKNTLYLQMNSLRAEDTAVYYCARVDSSGGYWY
FDLWGRGTLVTVSSASTKGPSVFPLAPCSRSTSESTAALGCLVKDYFPEPVTVSW
NSGALTSGVHTFPAVLQSSGLYSLSSVVTVPSSSLGTKTYTCNVDHKPSNTKVDK
RVESKYGPPCPPCPAPEFLGGPSVFLFPPKPKDTLMISRTPEVTCVVVDVSQEDPE
VQFNWYVDGVEVHNAKTKPREEQFNSTYRVVSVLTVLHQDWLNGKEYKCKV
SNKGLPSSIEKTISKAKGQPREPQVYTLPPSQEEMTKNQVSLTCLVKGFYPSDIAV
EWESNGQPENNYKTTPPVLDSDGSFFLYSRLTVDKSRWQEGNVFSCSVMHEAL
HNHYTQKSLSLSLGK

(SEQ ID NO: 66)

The light and heavy chain plasmids obtained by cloning were paired and co-transfected into HEK293E cells (a gift from Shanghai Hengrui Pharmaceuticals Co., Ltd.) or ExpiCHO (Cat No. A29127, ThermoFisher) cells. The cell culture supernatant was harvested 5 days (37 °C, HEK293E cells) or 10-12 days (32 °C, expiCHO cells) after the transfection and centrifuged at 4000 rpm for 20 min, and the supernatant was filtered using a 0.45 µm filter. Affinity purification (first step) was then performed using a MabSelectSure LX column (GE Healthcare). The culture supernatant was passed through a PBS-equilibrated MabSelectSure LX column. After the column was washed with PBS, the protein of interest was eluted with a 0.1 M glycine acidic eluent (pH 3.0), then neutralized with 1 M MES (pH 6.0), and finally subjected to fine purification with a HiTrap SP HP ion column. According to analysis, the antibody of interest was obtained.

### Example 4. Determination of Affinities of TGFβ1 Monoclonal Antibody

The affinities of the antibody for human/mouse GARP/TGFβ1 and human/mouse TGFβ1_C4S were determined using Biacore T200 (GE Healthcare).

A Protein A biosensor chip (Cat No. # 29127556, GE) was used to affinity-capture a certain amount of the test antibody, and the human or mouse GARP-TGFβ1 complex or TGFβ1_C4S antigen, serially diluted, was then allowed to flow over the surface of the chip at a flow rate of 50 µL/min, with a dissociation time of 5 min. After the end of each cycle, the chip was regenerated with glycine-hydrochloric acid (pH 1.5) (Cat. # BR-1003-54, GE). The reaction buffer was an HBS-EP+ buffer solution (Cat No. # BR-1006-69, GE) (pH 7.4) diluted to 1× with distilled water.

Reaction signals were detected in real-time using Biacore T200 to obtain association and dissociation curves, and the data obtained were fitted using the Langmuir 1:1 binding model in BIAevaluation software version 4.1 (GE) to obtain affinity values. The results are shown in Table 6.

**Table 6. Data on the affinities of H27**

| Antigen | *kₐ* (1/Ms) | *k_{d}* (1/s) | K_{D} (M) |
|---|---|---|---|
| Human GARP-TGFβ1 complex | 8.64e+04 | 5.94e-04 | 6.88e-09 |
| Mouse GARP-TGFβ1 complex | 1.37e+05 | 7.08e-04 | 5.16e-09 |
| Human TGFβ1_C4S | 2.63e+05 | 7.43e-04 | 2.83e-09 |
| Mouse TGFβ1_C4S | 3.68e+05 | 8.06e-04 | 2.19e-09 |

### Example 5. Affinity Maturation of TGFβ1 Monoclonal Antibody H27

The three-dimensional structure of the H27 antibody molecule was simulated. With reference to the human germline gene mutation hotspots and the three-dimensional structure simulation results, some of the key amino acid residues in the framework regions and CDRs were selected to establish 4 random mutation phage libraries (Table 7). Functional antibodies with improved affinities were obtained by screening using phage library display technology. The new amino acid residues obtained from different libraries were combined and verified to obtain functional antibodies with improved affinities and functions.

**Table 7. Affinity maturation library designs for H27**

| Library | Library size | Heavy chain mutated residues | Light chain mutated residue(s) |
|---|---|---|---|
| 1 | 1x10⁷ | G99 G100 Y100A | Y32 Y92 |
| 2 | 1x10⁷ | / | S28 S31 A50 S53 Q55 |
| 3 | 1x10⁷ | S53 S56 Y58 | S93 T94 |
| 4 | 1x10⁷ | S31 A33 V95 W100B | R24 |

| | | | |
|---|---|---|---|
| Note: "/" indicates that no mutated residue was contained | | | |

Four random mutation phage libraries were screened, with biotinylated human GARP-TGFβ1 (containing SEQ ID NOs: 5 and 6) as a screening antigen. After 3-4 rounds of screening, the obtained clones were subjected to sequence analysis. According to the analysis results, combined with phage ELISA results, 62 antibody sequences were selected for clone construction. The light chain variable regions VL of the antibodies were cloned into pTT5 expression vectors containing a human-derived kappa light chain constant region (SEQ ID NO: 19), and the heavy chain variable regions VH of the antibodies were cloned into pTT5 expression vectors containing a human hIgG4-S228P (CH1-CH2-CH3) heavy chain constant region (SEQ ID NO: 20).

The light and heavy chain plasmids obtained by cloning were paired and co-transfected into HEK293E cells. After 5 days, the cell culture supernatant was collected and centrifuged at 4000 rpm to remove cells. After MabSelect Sure purification, an affinity assay was performed with reference to the method of Example 4.

It was determined by SPR protein interaction that antibodies with affinities that were more than 5 times higher than that of H27 were all from the second random mutation library, and the antibody heavy chain of this library was the H27 heavy chain. Their light chain variable region sequences are shown in Table 8 (the CDRs are underlined, and they are defined using the Kabat numbering scheme). In addition, their light chain full-length sequences and CDR combinations are shown in Tables 9 and 10, respectively.

**Table 8. Light chain variable region sequences after affinity maturation of TGFβ1 monoclonal antibody H27**

| No. | VL |
|---|---|
| SL2-1 | DIQMTQSPSSLSASVGDRVTITCRASQAISDYLNWYQQKPGKAPKLLIYTASY LTSGVPSRFSGSGSGTDFTLTISSLQPEDFATYYCQQSYSTPLTFGGGTKLEIK( SEQ ID NO: 21) |
| SL2-2 | DIQMTQSPSSLSASVGDRVTITCRASQFISDYLNWYQQKPGKAPKLLIYTASY LDSGVPSRFSGSGSGTDFTLTISSLQPEDFATYYCQQSYSTPLTFGGGTKLEIK( SEQ ID NO: 22) |
| SL2-3 | DIQMTQSPSSLSASVGDRVTITCRASQGISEYLNWYQQKPGKAPKLLIYTASY LDSGVPSRFSGSGSGTDFTLTISSLQPEDFATYYCQQSYSTPLTFGGGTKLEIK( SEQ ID NO: 23) |
| SL2-4 | DIQMTQSPSSLSASVGDRVTITCRASQIISDYLNWYQQKPGKAPKLLIYSASY LESGVPSRFSGSGSGTDFTLTISSLQPEDFATYYCQQSYSTPLTFGGGTKLEIK( SEQ ID NO: 24) |
| SL2-5 | DIQMTQSPSSLSASVGDRVTITCRASQPISDYLNWYQQKPGKAPKLLIYTASY LESGVPSRFSGSGSGTDFTLTISSLQPEDFATYYCQQSYSTPLTFGGGTKLEIK( SEQ ID NO: 25) |
| SL2-6 | DIQMTQSPSSLSASVGDRVTITCRASQYISDYLNWYQQKPGKAPKLLIYMAS ALDSGVPSRFSGSGSGTDFTLTISSLQPEDFATYYCQQSYSTPLTFGGGTKLEI K(SEQ ID NO: 26) |
| SL2-8 | DIQMTQSPSSLSASVGDRVTITCRASQYISDYLNWYQQKPGKAPKLLIYAASY LESGVPSRFSGSGSGTDFTLTISSLQPEDFATYYCQQSYSTPLTFGGGTKLEIK( SEQ ID NO: 27) |
| SL2-9 | DIQMTQSPSSLSASVGDRVTITCRASQFISPYLNWYQQKPGKAPKLLIYAASE LESGVPSRFSGSGSGTDFTLTISSLQPEDFATYYCQQSYSTPLTFGGGTKLEIK( SEQ ID NO: 28) |
| SL2-12 | DIQMTQSPSSLSASVGDRVTITCRASQYISDYLNWYQQKPGKAPKLLIYSASY LDSGVPSRFSGSGSGTDFTLTISSLQPEDFATYYCQQSYSTPLTFGGGTKLEIK( SEQ ID NO: 29) |
| SL2-13 | DIQMTQSPSSLSASVGDRVTITCRASQVISDYLNWYQQKPGKAPKLLIYAASY LDSGVPSRFSGSGSGTDFTLTISSLQPEDFATYYCQQSYSTPLTFGGGTKLEIK( SEQ ID NO: 30) |
| SL2-15 | DIQMTQSPSSLSASVGDRVTITCRASQKISDYLNWYQQKPGKAPKLLIYAASG LESGVPSRFSGSGSGTDFTLTISSLQPEDFATYYCQQSYSTPLTFGGGTKLEIK( SEQ ID NO: 31) |
| SL2-17 | DIQMTQSPSSLSASVGDRVTITCRASQGISHYLNWYQQKPGKAPKLLIYAASY LDSGVPSRFSGSGSGTDFTLTISSLQPEDFATYYCQQSYSTPLTFGGGTKLEIK( SEQ ID NO: 32) |
| SL2-18 | DIQMTQSPSSLSASVGDRVTITCRASQSISEYLNWYQQKPGKAPKLLIYTASY LESGVPSRFSGSGSGTDFTLTISSLQPEDFATYYCQQSYSTPLTFGGGTKLEIK( SEQ ID NO: 33) |
| SL2-19 | DIQMTQSPSSLSASVGDRVTITCRASQAISPYLNWYQQKPGKAPKLLIYAASE LESGVPSRFSGSGSGTDFTLTISSLQPEDFATYYCQQSYSTPLTFGGGTKLEIK (SEQ ID NO: 34) |
| SL2-22 | DIQMTQSPSSLSASVGDRVTITCRASQSISDYLNWYQQKPGKAPKLLIYTASY LDSGVPSRFSGSGSGTDFTLTISSLQPEDFATYYCQQSYSTPLTFGGGTKLEIK (SEQ ID NO: 35) |

**Table 9. Light chain sequences after affinity maturation of TGFβ1 monoclonal antibody H27**

| No. | Light chain LC |
|---|---|
| SL2-1 | DIQMTQSPSSLSASVGDRVTITCRASQAISDYLNWYQQKPGKAPKLLIYTASY LTSGVPSRFSGSGSGTDFTLTISSLQPEDFATYYCQQSYSTPLTFGGGTKLEIKR TVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRG EC (SEQ ID NO:67) |
| SL2-2 | DIQMTQSPSSLSASVGDRVTITCRASQFISDYLNWYQQKPGKAPKLLIYTASY LDSGVPSRFSGSGSGTDFTLTISSLQPEDFATYYCQQSYSTPLTFGGGTKLEIKR TVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRG EC (SEQ ID NO:68) |
| SL2-3 | DIQMTQSPSSLSASVGDRVTITCRASQGISEYLNWYQQKPGKAPKLLIYTASY LDSGVPSRFSGSGSGTDFTLTISSLQPEDFATYYCQQSYSTPLTFGGGTKLEIKR TVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRG EC (SEQ ID NO:69) |
| SL2-4 | DIQMTQSPSSLSASVGDRVTITCRASQIISDYLNWYQQKPGKAPKLLIYSASY LESGVPSRFSGSGSGTDFTLTISSLQPEDFATYYCQQSYSTPLTFGGGTKLEIKR TVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRG |
| | EC (SEQ ID NO:70) |
| SL2-5 | DIQMTQSPSSLSASVGDRVTITCRASQPISDYLNWYQQKPGKAPKLLIYTASY LESGVPSRFSGSGSGTDFTLTISSLQPEDFATYYCQQSYSTPLTFGGGTKLEIKR TVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRG EC (SEQ ID NO:71) |
| SL2-6 | DIQMTQSPSSLSASVGDRVTITCRASQYISDYLNWYQQKPGKAPKLLIYMAS ALDSGVPSRFSGSGSGTDFTLTISSLQPEDFATYYCQQSYSTPLTFGGGTKLEI KRTVAAPSVFIFPPSDEQLKSGTASVVCLLNNFYPREAKVQWKVDNALQSGN SQESVTEQDSKDSTYSLSSTLTLSKADYEKHKVYACEVTHQGLSSPVTKSFN RGEC (SEQ ID NO:72) |
| SL2-8 | DIQMTQSPSSLSASVGDRVTITCRASQYISDYLNWYQQKPGKAPKLLIYAASY LESGVPSRFSGSGSGTDFTLTISSLQPEDFATYYCQQSYSTPLTFGGGTKLEIKR TVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRG EC (SEQ ID NO:73) |
| SL2-9 | DIQMTQSPSSLSASVGDRVTITCRASQFISPYLNWYQQKPGKAPKLLIYAASE LESGVPSRFSGSGSGTDFTLTISSLQPEDFATYYCQQSYSTPLTFGGGTKLEIKR TVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRG EC (SEQ ID NO:74) |
| SL2-12 | DIQMTQSPSSLSASVGDRVTITCRASQYISDYLNWYQQKPGKAPKLLIYSASY LDSGVPSRFSGSGSGTDFTLTISSLQPEDFATYYCQQSYSTPLTFGGGTKLEIKR TVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRG EC (SEQ ID NO:75) |
| SL2-13 | DIQMTQSPSSLSASVGDRVTITCRASQVISDYLNWYQQKPGKAPKLLIYAASY LDSGVPSRFSGSGSGTDFTLTISSLQPEDFATYYCQQSYSTPLTFGGGTKLEIKR TVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRG EC (SEQ ID NO:76) |
| SL2-15 | DIQMTQSPSSLSASVGDRVTITCRASQKISDYLNWYQQKPGKAPKLLIYAASG LESGVPSRFSGSGSGTDFTLTISSLQPEDFATYYCQQSYSTPLTFGGGTKLEIKR TVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRG EC (SEQ ID NO:77) |
| SL2-17 | DIQMTQSPSSLSASVGDRVTITCRASQGISHYLNWYQQKPGKAPKLLIYAASY LDSGVPSRFSGSGSGTDFTLTISSLQPEDFATYYCQQSYSTPLTFGGGTKLEIKR TVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRG EC (SEQ ID NO:78) |
| SL2-18 | DIQMTQSPSSLSASVGDRVTITCRASQSISEYLNWYQQKPGKAPKLLIYTASY LESGVPSRFSGSGSGTDFTLTISSLQPEDFATYYCQQSYSTPLTFGGGTKLEIKR TVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRG EC (SEQ ID NO:79) |
| SL2-19 | DIQMTQSPSSLSASVGDRVTITCRASQAISPYLNWYQQKPGKAPKLLIYAASE LESGVPSRFSGSGSGTDFTLTISSLQPEDFATYYCQQSYSTPLTFGGGTKLEIKR TVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRG EC (SEQ ID NO:80) |
| SL2-22 | DIQMTQSPSSLSASVGDRVTITCRASQSISDYLNWYQQKPGKAPKLLIYTASY LDSGVPSRFSGSGSGTDFTLTISSLQPEDFATYYCQQSYSTPLTFGGGTKLEIKR TVAAPSVFIFPPSDEQLKSGTASVVCLLNNFYPREAKVQWKVDNALQSGNSQ ESVTEQDSKDSTYSLSSTLTLSKADYEKHKVYACEVTHQGLSSPVTKSFNRG EC (SEQ ID NO:81) |

**Table 10. Light chain CDR sequences after affinity maturation of TGFβ1 monoclonal antibody H27**

| No. | Sequence | |
|---|---|---|
| SL2-1 | LCDR1 | RASQAISDYLN (SEQ ID NO: 36) |
| | LCDR2 | TASYLTS(SEQ ID NO: 37) |
| | LCDR3 | QQSYSTPLT(SEQ ID NO: 18) |
| SL2-2 | LCDR1 | RASQFISDYLN(SEQ ID NO: 39) |
| | LCDR2 | TASYLDS(SEQ ID NO: 40) |
| | LCDR3 | QQSYSTPLT(SEQ ID NO: 18) |
| SL2-3 | LCDR1 | RASQGISEYLN(SEQ ID NO: 41) |
| | LCDR2 | TASYLDS(SEQ ID NO: 40) |
| | LCDR3 | QQSYSTPLT(SEQ ID NO: 18) |
| SL2-4 | LCDR1 | RASQIISDYLN(SEQ ID NO: 42) |
| | LCDR2 | SASYLES(SEQ ID NO: 43) |
| | LCDR3 | QQSYSTPLT(SEQ ID NO: 18) |
| SL2-5 | LCDR1 | RASQPISDYLN(SEQ ID NO: 44) |
| | LCDR2 | TASYLES(SEQ ID NO: 45) |
| | LCDR3 | QQSYSTPLT(SEQ ID NO: 18) |
| SL2-6 | LCDR1 | RASQYISDYLN(SEQ ID NO: 46) |
| | LCDR2 | MASALDS(SEQ ID NO: 47) |
| | LCDR3 | QQSYSTPLT(SEQ ID NO: 18) |
| SL2-8 | LCDR1 | RASQYISDYLN(SEQ ID NO: 46) |
| | LCDR2 | AASYLES(SEQ ID NO: 48) |
| | LCDR3 | QQSYSTPLT(SEQ ID NO: 18) |
| SL2-9 | LCDR1 | RASQFISPYLN(SEQ ID NO: 49) |
| | LCDR2 | AASELES(SEQ ID NO: 50) |
| | LCDR3 | QQSYSTPLT(SEQ ID NO: 18) |
| SL2-12 | LCDR1 | RASQYISDYLN(SEQ ID NO: 46) |
| | LCDR2 | SASYLDS(SEQ ID NO: 51) |
| | LCDR3 | QQSYSTPLT(SEQ ID NO: 18) |
| SL2-13 | LCDR1 | RASQVISDYLN(SEQ ID NO: 52) |
| | LCDR2 | AASYLDS(SEQ ID NO: 53) |
| | LCDR3 | QQSYSTPLT(SEQ ID NO: 18) |
| SL2-15 | LCDR1 | RASQKISDYLN(SEQ ID NO: 54) |
| | LCDR2 | AASGLES(SEQ ID NO: 55) |
| | LCDR3 | QQSYSTPLT(SEQ ID NO: 18) |
| SL2-17 | LCDR1 | RASQGISHYLN(SEQ ID NO: 56) |
| | LCDR2 | AASYLDS(SEQ ID NO: 53) |
| | LCDR3 | QQSYSTPLT(SEQ ID NO: 18) |
| SL2-18 | LCDR1 | RASQSISEYLN(SEQ ID NO: 57) |
| | LCDR2 | TASYLES(SEQ ID NO: 45) |
| | LCDR3 | QQSYSTPLT(SEQ ID NO: 18) |
| SL2-19 | LCDR1 | RASQAISPYLN(SEQ ID NO: 58) |
| | LCDR2 | AASELES(SEQ ID NO: 50) |
| | LCDR3 | QQSYSTPLT(SEQ ID NO: 18) |
| SL2-22 | LCDR1 | RASQSISDYLN(SEQ ID NO: 59) |
| | LCDR2 | TASYLDS(SEQ ID NO: 40) |
| | LCDR3 | QQSYSTPLT(SEQ ID NO: 18) |

H27, SL2-1, SL2-2, SL2-3, SL2-4, SL2-5, SL2-6, SL2-8, SL2-9, SL2-12, SL2-13, SL2-15, SL2-17, SL2-18, SL2-19, and SL2-22 have structures of the following general formulas:
LCDR1 is RASQX₁ISX₂YLN (SEQ ID NO: 38), wherein X₁ is selected from the group consisting of S, A, F, G, I, P, Y, V, and K, and X₂ is selected from the group consisting of S, D, E, P, and H;
LCDR2 is X₃ASX₄LX₅S (SEQ ID NO: 64), wherein X₃ is selected from the group consisting of A, T, S, and M, X₄ is selected from the group consisting of S, Y, A, E, and G, and X₅ is selected from the group consisting of Q, T, D, and E.

SL2-2, SL2-9, SL2-12, SL2-19, and SL2-22 have structures of the following general formulas:
LCDR1 is RASQX₁ISX₂YLN (SEQ ID NO: 38), wherein X₁ is selected from the group consisting of F, Y, and A, and X₂ is selected from the group consisting of D and P;
LCDR2 is X₃ASX₄LX₅S (SEQ ID NO: 64), wherein X₃ is selected from the group consisting of A, T, and S, X₄ is selected from the group consisting of S, Y, and E, and X₅ is selected from the group consisting of Q, D, and E.

### Example 6. Determination of Affinities of TGFβ1 Affinity-Matured Monoclonal Antibodies

In this example, the negative control was HBS-EP, and the positive controls were Ab6 (Scholar rock, WO2020014460A1) and Abbv-151 (Abbvie, US10793627B2). Ab6 binds to GARP-TGFβ1 complexes, LRRC33-TGFβ1 complexes, LTBP3-TGFβ1 complexes, and LTBP1-TGFβ1 complexes, and the binding epitope is completely on TGFβ1 (Martin et al., 2020). The binding epitope of Abbv-151 includes the GARP and TGFβ1 in GARP-TGFβ1 complexes, and it does not bind to GARP or TGFβ1 alone (Streel et al., 2020). The sequences of Ab6 and Abbv-151 are shown below:
> Ab6 antibody heavy chain sequence:

EVQLVESGGGLVQPGGSLRLSCTASGFTFSSFSMDWVRQAPGKGLEWVSYISPS
ADTIYYADSVKGRFTISRDNAKNTLYLQMNSLRAEDTAVYYCARGVLDYGDML
MPWGQGTLVTVSSASTKGPSVFPLAPCSRSTSESTAALGCLVKDYFPEPVTVSW
NSGALTSGVHTFPAVLQSSGLYSLSSVVTVPSSSLGTKTYTCNVDHKPSNTKVDK
RVESKYGPPCPPCPAPEFLGGPSVFLFPPKPKDTLMISRTPEVTCVVVDVSQEDPE
VQFNWYVDGVEVHNAKTKPREEQFNSTYRVVSVLTVLHQDWLNGKEYKCKV
SNKGLPSSIEKTISKAKGQPREPQVYTLPPSQEEMTKNQVSLTCLVKGFYPSDIAV

EWESNGQPENNYKTTPPVLDSDGSFFLYSRLTVDKSRWQEGNVFSCSVMHEAL
HNHYTQKSLSLSLGK
(SEQ ID NO: 60)

> Ab6 antibody light chain sequence:

DIQMTQSPSSLSASVGDRVTITCQASQDITNYLNWYQQKPGKAPKLLIYDASNL
ETGVPSRFSGSGSGTDFTFTISSLQPEDIATYYCQQADNHPPWTFGGGTKVEIKR
TVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC

(SEQ ID NO: 61)

> Abbv-151 antibody heavy chain sequence: 
QVQLVQPGAEVRKPGASVKVSCKASGYRFTSYYIDWVRQAPGQGLEWMGRID
PEDAGTKYAQKFQGRVTMTADTSTSTVYVELSSLRSEDTAVYYCARYEWETVV
VGDLMYEYEYWGQGTLVTVSSASTKGPSVFPLAPCSRSTSESTAALGCLVKDYF
PEPVTVSWNSGALTSGVHTFPAVLQSSGLYSLSSVVTVPSSSLGTKTYTCNVDHK
PSNTKVDKRVESKYGPPCPPCPAPEFLGGPSVFLFPPKPKDTLMISRTPEVTCVV
VDVSQEDPEVQFNWYVDGVEVHNAKTKPREEQFNSTYRVVSVLTVLHQDWLN
GKEYKCKVSNKGLPSSIEKTISKAKGQPREPQVYTLPPSQEEMTKNQVSLTCLV
KGFYPSDIAVEWESNGQPENNYKTTPPVLDSDGSFFLYSRLTVDKSRWQEGNVF
SCSVMHEALHNHYTQKSLSLSLG

(SEQ ID NO: 62)
> Abbv-151 antibody light chain:

DIQMTQSPSSLSASVGDRVTITCQASQSISSYLAWYQQKPGQAPKILIYGASRLK
TGVPSRFSGSGSGTSFTLTISSLEPEDAATYYCQQYASVPVTFGQGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC
(SEQ ID NO: 63)

The affinities of several affinity-matured monoclonal antibodies for the human and mouse GARP-TGFβ1 complexes were determined using Biacore T200 (GE Healthcare) according to the method of Example 4. The results of the affinity assay are shown in Tables 11 and 12.

**Table 11. Data on the affinities of affinity-matured antibodies of H27**

| Antibody | Antigen | *kₐ* (1/Ms) | *k_{d}* (1/s) | K_{D} (M) |
|---|---|---|---|---|
| H27 | Human GARP-TGFβ1 complex | 3.61E+05 | 9.57E-04 | 2.65E-09 |
| SL2-2 | | 3.87E+05 | 5.24E-05 | 1.35E-10 |
| SL2-22 | | 3.79E+05 | 6.08E-05 | 1.61E-10 |
| SL2-12 | | 3.20E+05 | 8.51E-05 | 2.66E-10 |
| SL2-19 | | 1.92E+05 | 6.95E-05 | 3.62E-10 |
| SL2-9 | | 1.80E+05 | 8.54E-05 | 4.75E-10 |

**Table 12. Data on the affinities of SL2-22**

| Antibody | Antigen | *kₐ* (1/Ms) | *k_{d}* (1/s) | K_{D} (M) |
|---|---|---|---|---|
| Abbv151 | Human GARP-TGFβ1 complex | 1.54E+05 | 1.51E-04 | 9.80E-10 |
| Ab6 | | 4.17E+05 | 1.38E-04 | 3.32E-10 |
| SL2-22 | | 3.75E+05 | 9.50E-05 | 2.54E-10 |
| Abbv151 | Murine GARP-TGFβ1 complex | / | / | / |
| Ab6 | | 4.69E+05 | 1.06E-04 | 2.26E-10 |
| SL2-22 | | 3.89E+05 | 8.86E-05 | 2.28E-10 |

### Example 7. In Vitro Inhibitory Function of TGFβ1 Monoclonal Antibodies Against GARP-TGFβ1 Complex

2 × 10⁶ LN229 cells (Procell, CL-0578) were transferred to a T75 culture flask (Nunc). After 24 h, the cells were mono-transfected with human or mouse pro-TGFβ1 or co-transfected with human or mouse pro-TGFβ1 and GARP or LRRC33 plasmids using the 293fectin^{™} (Invitrogen) transfection reagent. After 24 h, the cells were transferred to a white opaque 96-well cell culture plate (PerkinElmer) at 1 × 10⁴ cells per well. After 24 h, the culture medium was removed using a pipette, and HepG2 CAGA12-luc luciferase reporter cells resuspended in DMEM + 0.5% BSA were added, along with serially diluted H27, affinity-matured antibodies of H27, and Ab6 (control antibody). After 20 h of mixed culture, the ONE-Glo^{™} luciferase reagent was added. After 5 min of incubation, chemiluminescence was measured using a multi-mode microplate reader (SpectraMax M5). The luciferase activity value of the vehicle treatment group was normalized to 1, and relative activity = luciferase of antibody treatment group ÷ vehicle control group. Plotting was performed using Prism 9 through non-linear fitting and a three-parameter log(inhibitor) vs. response model.

The results are shown in FIGs. 1A and 1B. The inhibitory activity of the SL2-2, SL2-9, SL2-12, SL2-19, and SL2-22 after affinity maturation against pro-TGFβ1 and the TGFβ1_complexes was higher than that of the parent antibody H27. The inhibitory activity of SL2-2, SL2-9, SL2-12, SL2-19, and SL2-22 against human pro-TGFβ1, the GARP-TGFβ1 complex, and the murine GARP-TGFβ1 complex was comparable to that of the positive control Ab6, and their inhibitory activity against the human LRRC33-TGFβ1 complex was superior to that of Ab6.

### Example 8. Binding of TGFβ1 Monoclonal Antibody SL2-22 to Cells Expressing Different TGFβ Complexes

A 1 × 10⁶ cells/mL suspension of HEK293E cells was prepared, and 5 mL of the cell suspension was placed in a 50 mL culture tube. Then the cells were transfected with a plasmid shown in Table 13 using the 293fectin^{™} reagent. After 48 h, the cells were washed twice with 1× PBS and placed in a 96-well round-bottom cell culture plate (Nunc) at 1 × 10⁵ cells per well. 100 µL of 10 nM antibody or control antibody prepared in 1× PBS was added, and the cells were stained at room temperature for 1 h. After 3 washes with 1× PBS, 100 µL of FITC-labeled anti-human Fc antibody was added, and the cells were stained at room temperature for 30 min. After the staining, the plate was washed 3 times with 1× PBS. 7-AAD was added, and the plate was incubated for 5 min. After 3 washes with 1× PBS, the cells were analyzed using a flow cytometer. The number of FITC-positive cells among viable cells was analyzed by Flowjo, and the percentage of the number of FITC-positive cells to the total number of viable cells was calculated and plotted.

**Table 13. Plasmid structures**

| Plasmid name | Structure |
|---|---|
| Control plasmids (Control plasmids) | pcDNA3.1 |
| TGFβ1 | Human TGFβ1 sequence expressed by hTGFβ1 expression plasmid (Genbank Accession NO. NC 000019.10) |
| TGFβ2 | Human TGFβ2 sequence expressed by hTGFβ2 expression plasmid (Genbank Accession NO. NC 000001.11) |
| TGFβ3 | Human TGFβ3 sequence expressed by hTGFβ3 expression plasmid (Genbank Accession NO. NC_000014.9) |
| TGFβ1-GARP complex | Human TGFβ1 sequence expressed by hTGFβ1 expression plasmid (Genbank Accession NO. NC_000019.10) |
| | Human GARP sequence expressed by GARP expression plasmid (Genbank Accession NO. NC 000011.10) |
| TGFβ2-GARP complex | Human TGFβ2 sequence expressed by hTGFβ2 expression plasmid (Genbank Accession NO. NC_000001.11) |
| | Human GARP sequence expressed by GARP expression plasmid (Genbank Accession NO. NC 000011.10) |
| TGFβ3-GARP complex | Human TGFβ3 sequence expressed by hTGFβ3 expression plasmid (Genbank Accession NO. NC_000014.9) |
| | Human GARP sequence expressed by GARP expression plasmid (Genbank Accession NO. NC 000011.10) |
| mTGFβ1 | Mouse TGFβ1 sequence expressed by mTGFβ1 expression plasmid (Genbank Accession NO. NC 000073.7) |
| mTGFβ1-mGARP complex | Mouse TGFβ1 sequence expressed by mTGFβ1 expression plasmid (Genbank Accession NO. NC_000073.7) |
| | Mouse GARP sequence expressed by mGARP expression plasmid (Genbank Accession NO. NC 000073.7) |

The results are shown in FIG. 2. Both SL2-22 and Ab6 bound to human and murine TGFβ1 (corresponding to TGFβ1 and mTGFβ1, respectively, in FIG. 2) and the GARP-TGFβ1 complexes (corresponding to TGFβ1 + GARP and mTGFβ1 + mGARP, respectively, in FIG. 2) and did not bind to TGFβ2 or the GARP-TGFβ2 complex (corresponding to TGFβ2 + GARP in FIG. 2). The difference was that Ab6 also bound to human TGFβ3 and the GARP-TGFβ3 complex (corresponding to TGFβ3 + GARP in FIG. 2) to a certain extent, while SL2-22 specifically bound to the TGFβ1 complexes only.

### Example 9. Study on Epitope Competition of TGFβ1 Antibody

**In** this example, the negative control was HBS-EP, and the positive controls were Ab6 and Abbv-151.

The epitope competition of the anti-human TGFβ1 antibody SL2-22 with the positive control antibodies for the human GARP-TGFβ1 complex was studied using Biacore T200 (GE Healthcare).

Anti-His tag antibody coupling was performed on a CM5 sensor chip (Cat. 29-1049-88, GE) using an amino coupling kit (Cat No. BR-1000-50, GE) and a His capture kit (Cat No. 28-9950-56, GE) according to the kit instructions, such that the surface response value reached about 10,000 RU. Then the chip was blocked with ethanolamine for later use.

The steps of the epitope competition study (a dual mode was used for samples) were as follows (the antibodies were paired in a cycle):
1) Antigen capture: the human GARP-TGFβ1 complex
2) First antibody: concentration: 100 µg/mL; flow rate: 50 µL/min; 60 s; HBS-EP buffer as a control
3) Second antibody: concentration: 100 µg/mL; flow rate: 50 µL/min; 60 s; HBS-EP buffer as a control
4) Sensorgrams were analyzed. SL2-22 partially competed with Ab6 for the binding epitope of the human GARP-TGFβ1 complex, and SL2-22 did not compete with Abbv151 for the binding epitope of the human GARP-TGFβ1 complex at all (Table 14).

**Table 14. The assessment of the epitope competition of SL2-22 with the positive antibodies Ab6 and Abbv151**

| | SL2-22 | Ab6 | Abbv151 |
|---|---|---|---|
| SL2-22 | | Partial competition | × |
| Ab6 | Partial competition | | × |
| Abbv151 | × | × | |

| | | | |
|---|---|---|---|
| (Note: "×" indicates that there was no competition at all) | | | |

### Example 10. Inhibition of Tumor Growth in EMT-6 Mouse Model by TGFβ1 Monoclonal Antibody SL2-22

To determine the *in vivo* tumor inhibition activity of SL2-22, BALB/c mice (female, 6 weeks old, purchased from Beijing Vital River Laboratory Animal Technology Co., Ltd.) were acclimatized for 1 week, and numbered and weighed on the day of the experiment. Mouse breast cancer cells (EMT-6, Procell, CL-0573) in the logarithmic growth phase were collected and resuspended in PBS to a concentration of 5 × 10⁶ cells/mL, and 0.1 mL of the suspension was subcutaneously inoculated into the right flank of each BALB/c mouse. On day 3 after the inoculation, when the mean tumor volume reached about 60 mm³, mice with moderate individual tumor volume were selected and randomly divided into groups as shown in Table 15. mIgG2a-FcS was an mIgG2a Fc with L234A/L235E/G237A/D327Q/A330S/P331S whose ADCC effect was removed, and was used as an isotype control. RMP1-14-mIgG2a-FcS was an antibody formed by fusing the variable regions of the anti-PD1 antibody RMP1-14 (the sequences 285 (heavy chain variable region) and 286 (light chain variable region) whose signal peptides were removed in WO2018223182A1; WO2018223182A1 is incorporated herein by reference in its entirety) with an mIgG2a heavy chain constant region with L234A/L235E/G237A/D327Q/A330S/P331S whose ADCC effect was removed/a murine kappa light chain constant region. SL2-22-mIgG2a-FcS was an antibody formed by fusing the variable regions of SL2-22 with an mIgG2a heavy chain constant region with L234A/L235E/G237A/D327Q/A330S/P331S whose ADCC effect was removed/a murine kappa light chain constant region; the sequences are shown below:
> SL2-22-mIgG2a-FcS antibody heavy chain: 
EVQLLESGGGLVQPGGSLRLSCAASGFTFSSYAMSWVRQAPGKGLEWVSAISGS
GGSTYYADSVKGRFTISRDNSKNTLYLQMNSLRAEDTAVYYCARVDSSGGYWY
FDLWGRGTLVTVSSAKTTAPSVYPLAPVCGDTTGSSVTLGCLVKGYFPEPVTLT
WNSGSLSSGVHTFPAVLQSDLYTLSSSVTVTSSTWPSQSITCNVAHPASSTKVDK
KIEPRGPTIKPCPPCKCPAPNAEGAPSVFIFPPKIKDVLMISLSPIVTCVVVDVSED
DPDVQISWFVNNVEVHTAQTQTHREDYNSTLRVVSALPIQHQDWMSGKEFKC
KVNNKQLPSSIERTISKPKGSVRAPQVYVLPPPEEEMTKKQVTLTCMVTDFMPE
DIYVEWTNNGKTELNYKNTEPVLDSDGSYFMYSKLRVEKKNWVERNSYSCSV
VHEGLHNHHTTKSFSRTPGK

(SEQ ID NO: 82)
> SL2-22-mIgG2a-FcS antibody light chain: 
DIQMTQSPSSLSASVGDRVTITCRASQSISDYLNWYQQKPGKAPKLLIYTASYLD
SGVPSRFSGSGSGTDFTLTISSLQPEDFATYYCQQSYSTPLTFGGGTKLEIKRADA
APTVSIFPPSSEQLTSGGASVVCFLNNFYPKDINVKWKIDGSERQNGVLNSWTD
QDSKDSTYSMSSTLTLTKDEYERHNSYTCEATHKTSTSPIVKSFNRNEC
(SEQ ID NO: 83)
Administration was started on the day of grouping. The antibodies were administered by intraperitoneal injection at the same molar dose at a frequency of twice weekly, and they were administered 7 times in total. The body weight and tumor volume of the mice were measured twice weekly. The tumor volume was calculated using the formula: TV = L_{long} × Lₛₕₒᵣₜ² / 2. The tumor volume of each group was expressed as mean ± standard deviation. Statistical analysis was performed using two-way ANOVA, and the tumor growth inhibition rate (%TGI) was calculated using the formula: %TGI = [1 - (T - T0) / (C - C0)] × 100%.

The results are shown in FIGs. 3A and 3B and Table 15. The combined use of SL2-22-mIgG2a-FcS and the anti-PD-1 antibody RMP1-14-mIgG2a-FcS significantly inhibited the growth of EMT-6 tumors in mice (p = 0.0127, two-way ANOVA) and extended the survival of the animals (p = 0.0323, Log-rank (Mantel-Cox) test) compared to the RMP1-14-mIgG2a-FcS monotherapy group. During treatment, the mice showed good tolerance, and no significant weight loss occurred (FIG. 3C).

**Table 15. The administration regimen, anti-tumor effect, and median survival time of SL2-22 in the EMT-6 model**

| Group | Number of animals | Dose (mg/kg) | %TGI (day 28) | Median survival time (days) |
|---|---|---|---|---|
| mIgG2a-FcS | 12 | 3.5 | - | 22.5 |
| RMP1-14-mIgG2a-FcS | 12 | 10 | 15.98 | 24 |
| RMP1-14-mIgG2a-FcS + SL2-22-mIgG2a-FcS | 12 | 10 + 10 | 56.60 | 40 |

| | | | | |
|---|---|---|---|---|
| Note: mIgG2a-FcS, RMP1-14-mIgG2a-FcS, and SL2-22-mIgG2a-FcS were administered at the same molar concentration, which was about 68 µmol/kg. | | | | |

### Example 11. Inhibition of Tumor Growth in CT26 Mouse Model by TGFβ1 Antibody SL2-22

To determine the *in vivo* tumor inhibition activity of SL2-22, BALB/c mice (female, 6 weeks old, purchased from Beijing Vital River Laboratory Animal Technology Co., Ltd.) were acclimatized for 1 week, and numbered and weighed on the day of the experiment. Mouse colon cancer cells (CT26, National Biomedical Cell-Line Resource, 1101MOU-PUMC000275) in the logarithmic growth phase were collected and resuspended in PBS to a concentration of 3 × 10⁶ cells/mL, and 0.1 mL of the suspension was subcutaneously inoculated into the right flank of each BALB/c mouse. On days 8-9 after the inoculation, when the mean tumor volume reached about 60 mm³, mice with moderate individual tumor volume were selected and randomly divided into groups as shown in Table 16. Administration was started on the day of grouping. The antibodies were administered by intraperitoneal injection at the same molar dose at a frequency of twice weekly, and they were administered 6 times in total. The body weight and tumor volume of the mice were measured twice weekly. The tumor volume was calculated using the formula: TV = L_{long} × Lₛₕₒᵣₜ² / 2. The tumor volume of each group was expressed as mean ± standard deviation. The tumor growth inhibition rate (%TGI) was calculated using the formula: %TGI = [1 - (T - T0) / (C - C0)] × 100%.

The results are shown in FIGs. 4A and 4B and Table 16. The combined use of SL2-22-mIgG2a-FcS and the anti-PD-1 antibody RMP1-14-mIgG2a-FcS inhibited the growth of CT26 tumors in mice and extended the survival of the animals. During treatment, the mice showed good tolerance, and no significant weight loss occurred (FIG. 4C).

**Table 16. The administration regimen, anti-tumor effect, and median survival time of SL2-22 in the CT26 model**

| Group | Number of animals | Dose (mg/kg) | %TGI (day 28) | Median survival time (days) |
|---|---|---|---|---|
| mIgG2a-FcS | 10 | 3.5 | - | 22 |
| RMP1-14-mIgG2a-FcS | 10 | 10 | 33.96 | 30 |
| RMP1-14-mIgG2a-FcS + SL2-22-mIgG2a-FcS | 10 | 10 + 10 | 48.55 | 37 |

| | | | | |
|---|---|---|---|---|
| Note: mIgG2a-FcS, RMP1-14-mIgG2a-FcS, and SL2-22-mIgG2a-FcS were administered at the same molar concentration, which was about 68 µmol/kg | | | | |

### Example 12. Inhibition of Fibrosis Process in Idiopathic Fibrosis Mouse Model by TGFβ1 Monoclonal Antibody SL2-22

To assess the *in vivo* inhibition of the fibrosis process by the TGFβ1 antibody SL2-22, C57BL/6J mice (male, 8 weeks old, purchased from Beijing Vital River Laboratory Animal Technology Co., Ltd.) were acclimatized for 1 week, and numbered and weighed on the day of the experiment. On the day of modeling, the mice were numbered and weighed, and randomly divided into groups of 15 according to the weight distribution. The specific groups are shown in Table 17 below. On day 1 of the experiment, an antibody drug or vehicle control was administered by intraperitoneal injection according to body weight. After 2 h, bleomycin was transtracheally administered using an infusion drip and a blunt needle. At a dose of 2 U/kg, about 50 µL of bleomycin was slowly administered to each animal using an infusion drip. The mice were then dosed twice weekly for a total of 3 weeks according to the groups described above. On day 22 of the experiment, bronchoalveolar lavage fluid and lung tissue were separately collected and analyzed for the degree of immune cell infiltration and the hydroxyproline level in lung tissue, and Masson staining analysis was performed.

**Table 17. The experimental groups and administration regimen**

| Group | Modeling type | Administration type | Number of animals | Dose (mg/kg) |
|---|---|---|---|---|
| Sham surgery group | PBS | PBS | 10 | / |
| Model group | Bleomycin | PBS | 15 | / |
| Treatment group | Bleomycin | SL2-22-mIgG2a-FcS | 15 | 15 mg/kg |

The SL2-22-mIgG2a-Fc treatment group significantly improved the overall body weight and survival rate of the animals (FIGs. 5A and 5B), and reduced the hydroxyproline level in lung tissue by 40.44% compared to the model group (FIG. 5C). Meanwhile, it significantly reduced the collagen deposition and fibrosis levels in the lungs after bleomycin modeling by 46.51% compared to the model group (FIGs. 5D and 5E).

### Example 13. Screening for and Preparation of Anti-Human and Mouse GARP-TGFβ1 Single-Domain Antibodies (VHHs)

### 1. Camel immunization and library construction

A Bactrian camel was immunized with the human GARP/TGFβ1 protein (a protein complex obtained by co-expressing and purifying the proteins set forth in SEQ ID NOs: 5 and 6) as an antigen. After Freund's complete adjuvant was mixed with the antigen in a volume ratio of 1:1, the camel was subjected to subcutaneous multi-site immunization every two weeks. The dose of the first immunization was 200 µg of protein, and the dose of the following four immunizations was 100 µg of protein/immunization. A total of five immunizations were performed, and the titer was then determined using the human GARP/TGFβ1 protein. When the titer was up to standard, peripheral blood was collected from the camel, lymphocytes were isolated and lysed with Trizol, RNA was extracted and reverse-transcribed into cDNA, and a phage library was constructed.

### 2. Screening for single-domain antibodies (VHHs)

20 µg of biotinylated human GARP/TGFβ1 protein was used to bind to 100 µL of Dynabeads^{™} M-280 streptavidin. After 1 h of standing at 37 °C, the beads were blocked with 2% skim milk at room temperature for 1 h. The aforementioned phage library was added, and the mixture was incubated at room temperature for 1 h. The beads were washed 9 times with PBST (PBS containing 0.05% Tween-20) to remove non-binding phages. Phages specifically binding to the human GARP/TGFβ1 protein were eluted with 1 mg/mL trypsin and used to infect *Escherichia coli* TG1 in the logarithmic growth phase, and first-round positive phages were produced and purified. Based on the positive phages obtained from the first round of screening, single-domain antibodies (VHHs) that also had high affinities for the mouse GARP-TGFbβ1 complex protein were obtained from the second round of screening.

From the screening-enriched positive clones, 96 monoclonal colonies were picked. These monoclonal colonies were packaged into phage single-chain antibodies for phage ELISA testing. ELISA plates were coated with 2 µg/mL human GARP-TGFβ1 complex protein, and a phage supernatant diluted with the blocking solution was added. An assay was performed using an anti-M13 HRP-labeled antibody. Clones with OD450 value/background value > 5 in the ELISA binding test results were sequenced to obtain their sequences. The C19-VHH sequence is shown below.
> C19-VHH

QVQLVESGGGSVQAGGSLRLSCTASGFTYCEYDMSWYRQAPGKEREFVSRIASDGR

TSYVDSVKGRFTISQDNARNTGYLEMNSLKPEDTAMYYCKTEAVKYSGNWCVAAP
GFAYWGQGTQVTVSS (SEQ ID NO: 84)

In SEQ ID NO: 84, the order is FR1-CDR1-FR2-CDR2-FR3-CDR3-FR4. In the sequence, the FR sequences are in italics, and the underlined portions are the CDR1, CDR2, and CDR3 sequences, respectively. The numbering scheme for the anti-GARP-TGFβ1 single-domain antibodies provided in the present disclosure was Kabat.

**Table 18. The CDR sequences of C19 VHH**

| | |
|---|---|
| CDR1 | EYDMS (SEQ ID NO: 85) |
| CDR2 | RIASDGRTSYVDSVKG (SEQ ID NO: 86) |
| CDR3 | EAVKYSGNWCVAAPGFAY (SEQ ID NO: 87) |

### 3. Preparation of complete antibody

The C19-VHH sequence was fused with the following human IgG4-Fc (CH2-CH3, containing S228P) fragment to obtain C19.
> hIgG4-Fc (S228P) 
ESKYGPPCPPCPAPEFLGGPSVFLFPPKPKDTLMISRTPEVTCVVVDVSQEDPEV
QFNWYVDGVEVHNAKTKPREEQFNSTYRVVSVLTVLHQDWLNGKEYKCKVS
NKGLPSSIEKTISKAKGQPREPQVYTLPPSQEEMTKNQVSLTCLVKGFYPSDIAVE
WESNGQPENNYKTTPPVLDSDGSFFLYSRLTVDKSRWQEGNVFSCSVMHEALH
NHYTQKSLSLSLGK
(SEQ ID NO: 88)
> C19

QVQLVESGGGSVQAGGSLRLSCTASGFTYCEYDMSWYRQAPGKEREFVSRIAS
DGRTSYVDSVKGRFTISQDNARNTGYLEMNSLKPEDTAMYYCKTEAVKYSGN
WCVAAPGFAYWGQGTQVTVSSESKYGPPCPPCPAPEFLGGPSVFLFPPKPKDTL
MISRTPEVTCVVVDVSQEDPEVQFNWYVDGVEVHNAKTKPREEQFNSTYRVVS
VLTVLHQDWLNGKEYKCKVSNKGLPSSIEKTISKAKGQPREPQVYTLPPSQEEM
TKNQVSLTCLVKGFYPSDIAVEWESNGQPENNYKTTPPVLDSDGSFFLYSRLTVD
KSRWQEGNVFSCSVMHEALHNHYTQKSLSLSLGK

(SEQ ID NO: 89)

The C19 sequence was cloned into a mammalian expression vector pTT5, and HEK293E or ExpiCHO (ThermoFisher, A29127) cells were transfected. Five days (HEK293E cells, 37 °C) or 10-12 days (ExpiCHO cells, 32 °C) after the transfection, centrifugation was performed at 4000 rpm for 20 min, and the cell culture supernatant was harvested and filtered using a 0.45 µm filter. Affinity purification (first step) was then performed using a MabSelectSure LX column (GE Healthcare). The culture supernatant was passed through a PBS-equilibrated MabSelectSure LX column. After the column was washed with PBS, the protein of interest was eluted with a 0.1 M glycine acidic eluent (pH 3.0), then neutralized with 1 M Tris-HCl (pH 8.0), and finally subjected to fine purification with a HiTrap Q HP ion column. According to analysis, the antibody of interest was obtained.

It was found by SPR and cell function experiments that C19 has a relatively good inhibitory function and well binds to both the human and mouse GARP-TGFβ1 complexes.

### Example 14. Humanization Engineering of Anti-GARP-TGFβ1 Single-Domain Antibody

Three-dimensional structure homologous modeling was performed on the selected specific TGFβ1 single-domain antibody C19. According to the modeling results, combined with the results of alignments with the V-base human germline sequence database and the IMGT human antibody heavy chain variable region germline gene database, the heavy chain variable region germline gene IGHV3-23, which is highly homologous with the C19 sequence, was selected as a template for FR1, FR2, and FR3, and IGJH4 was used as a template for FR4. The CDRs of the camelid-derived single-domain antibody were grafted into the corresponding human templates, forming a variable region sequence in the order of FR1-CDR1-FR2-CDR2-FR3-CDR3-FR4. To keep the original activity of the single-domain antibody after humanization, a series of back mutations were performed in the framework regions. The humanized VHH framework regions comprise at least one amino acid mutation selected from the group consisting of the following: 23T, 29Y, 30C, 37Y, 44E, 45R, 47F, 71Q, 74A, 75R, 78G, 81E, 93K, and 94T. The above amino acid positions are numbered according to the Kabat numbering scheme.

The humanized sequences obtained are shown below, with the CDRs underlined and defined using the Kabat numbering scheme:
> C19-hu3 
EVQLLESGGGLVQPGGSLRLSCTASGFTYCEYDMSWYRQAPGKEREFVSRIASD
GRTSYVDSVKGRFTISQDNARNTGYLQMNSLRAEDTAVYYCKTEAVKYSGNW
CVAAPGFAYWGQGTLVTVSS

(SEQ ID NO: 90)
> C19-hu7 
EVQLLESGGGLVQPGGSLRLSCTASGFTYCEYDMSWYRQAPGKEREFVSRIASD
GRTSYVDSVKGRFTISQDNARNTGYLEMNSLRAEDTAVYYCKTEAVKYSGNW
CVAAPGFAYWGQGTLVTVSS

(SEQ ID NO: 91)
> C19-hu8

EVQLLESGGGLVQPGGSLRLSCTASGFTYCEYDMSWYRQAPGKEREFVSRIASD
GRTSYVDSVKGRFTISQDNSKNTGYLEMNSLRAEDTAVYYCKTEAVKYSGNWC
VAAPGFAYWGQGTLVTVSS
(SEQ ID NO: 92)

Full antibody sequences with the humanized single-domain antibodies (VHHs) fused with the Fc region of hIgG4 were constructed using the method in Example 2. The humanized complete antibody sequences obtained are shown below:
> C19-3 
EVQLLESGGGLVQPGGSLRLSCTASGFTYCEYDMSWYRQAPGKEREFVSRIASD
GRTSYVDSVKGRFTISQDNARNTGYLQMNSLRAEDTAVYYCKTEAVKYSGNW
CVAAPGFAYWGQGTLVTVSSESKYGPPCPPCPAPEFLGGPSVFLFPPKPKDTLMI
SRTPEVTCVVVDVSQEDPEVQFNWYVDGVEVHNAKTKPREEQFNSTYRVVSVL
TVLHQDWLNGKEYKCKVSNKGLPSSIEKTISKAKGQPREPQVYTLPPSQEEMTK
NQVSLTCLVKGFYPSDIAVEWESNGQPENNYKTTPPVLDSDGSFFLYSRLTVDKS
RWQEGNVFSCSVMHEALHNHYTQKSLSLSLGK

(SEQ ID NO: 93)
> C19-7 
EVQLLESGGGLVQPGGSLRLSCTASGFTYCEYDMSWYRQAPGKEREFVSRIASD
GRTSYVDSVKGRFTISQDNARNTGYLEMNSLRAEDTAVYYCKTEAVKYSGNW
CVAAPGFAYWGQGTLVTVSSESKYGPPCPPCPAPEFLGGPSVFLFPPKPKDTLMI
SRTPEVTCVVVDVSQEDPEVQFNWYVDGVEVHNAKTKPREEQFNSTYRVVSVL
TVLHQDWLNGKEYKCKVSNKGLPSSIEKTISKAKGQPREPQVYTLPPSQEEMTK
NQVSLTCLVKGFYPSDIAVEWESNGQPENNYKTTPPVLDSDGSFFLYSRLTVDKS
RWQEGNVFSCSVMHEALHNHYTQKSLSLSLGK

(SEQ ID NO: 94)
> C19-8

EVQLLESGGGLVQPGGSLRLSCTASGFTYCEYDMSWYRQAPGKEREFVSRIASD
GRTSYVDSVKGRFTISQDNSKNTGYLEMNSLRAEDTAVYYCKTEAVKYSGNWC
VAAPGFAYWGQGTLVTVSSESKYGPPCPPCPAPEFLGGPSVFLFPPKPKDTLMISR
TPEVTCVVVDVSQEDPEVQFNWYVDGVEVHNAKTKPREEQFNSTYRVVSVLT
VLHQDWLNGKEYKCKVSNKGLPSSIEKTISKAKGQPREPQVYTLPPSQEEMTK
NQVSLTCLVKGFYPSDIAVEWESNGQPENNYKTTPPVLDSDGSFFLYSRLTVDKS
RWQEGNVFSCSVMHEALHNHYTQKSLSLSLGK

(SEQ ID NO: 95)

### Example 15. Determination of Affinities of Anti-GARP-TGFβ1 Single-Domain Antibodies for GARP-TGFβ1 Complexes

The negative control in this example was HBS-EP (10 mM HEPES, 150 mM NaCl, 3 mM EDTA, 0.005% P20, pH 7.4). The positive control was Abbv-151 (Abbvie, US10793627B2). Its sequences are shown below:
> Abbv-151 antibody heavy chain: 
QVQLVQPGAEVRKPGASVKVSCKASGYRFTSYYIDWVRQAPGQGLEWMGRID
PEDAGTKYAQKFQGRVTMTADTSTSTVYVELSSLRSEDTAVYYCARYEWETVV
VGDLMYEYEYWGQGTLVTVSSASTKGPSVFPLAPCSRSTSESTAALGCLVKDYF
PEPVTVSWNSGALTSGVHTFPAVLQSSGLYSLSSVVTVPSSSLGTKTYTCNVDHK
PSNTKVDKRVESKYGPPCPPCPAPEFLGGPSVFLFPPKPKDTLMISRTPEVTCVV
VDVSQEDPEVQFNWYVDGVEVHNAKTKPREEQFNSTYRVVSVLTVLHQDWLN
GKEYKCKVSNKGLPSSIEKTISKAKGQPREPQVYTLPPSQEEMTKNQVSLTCLV
KGFYPSDIAVEWESNGQPENNYKTTPPVLDSDGSFFLYSRLTVDKSRWQEGNVF
SCSVMHEALHNHYTQKSLSLSLG

(SEQ ID NO: 62)
> Abbv-151 antibody light chain:

DIQMTQSPSSLSASVGDRVTITCQASQSISSYLAWYQQKPGQAPKILIYGASRLK
TGVPSRFSGSGSGTSFTLTISSLEPEDAATYYCQQYASVPVTFGQGTKVEIKRTVA
APSVFIFPPSDEQLKSGTASVVCLLNNFYPREAKVQWKVDNALQSGNSQESVTE
QDSKDSTYSLSSTLTLSKADYEKHKVYACEVTHQGLSSPVTKSFNRGEC
(SEQ ID NO: 63)

The affinities of C19 and its humanized antibodies and the positive control antibody Abbv-151 for the human GARP-TGFβ1 complex (formed by protein complexing of the sequences set forth in SEQ ID NOs: 5 and 6) or the mouse GARP-TGFβ1 complex (formed by protein complexing of the sequences set forth in SEQ ID NOs: 8 and 9) were determined using a Biacore T200 (GE Healthcare) instrument. The test antibodies were captured to the surface of a chip (Series S sensor chip Protein A, GE Healthcare, 29127556), and the human or mouse GARP-TGFβ1 complex, at different concentrations, was then allowed to flow over the surface of the chip. Reaction signals were detected in real time to obtain association and dissociation curves, and the binding capacity constant was obtained by fitting. The solution used in the experiment was an HBS-EP solution. At the end of each experimental cycle, the chip was regenerated with a pH 1.5 glycine (GE Healthcare, BR-1003-54) solution. The affinity results for the antibodies are shown in Table 19. The results show that the affinities of the antibodies C19, C19-3, C19-7, and C19-8 obtained by screening in the present disclosure for the human GARP-TGFβ1 complex were comparable to that of the positive control Abbv-151, and the difference was that they also bound to the mouse GARP-TGFβ1 complex to a certain extent.

**Table 19. The determination of the affinities of C19 and humanized antibodies of C19 for human or mouse GARP/TGFβ1**

| Antibody name | Antigen | *kₐ* (1/Ms) | *k_{d}* (1/s) | K_{D} (M) |
|---|---|---|---|---|
| C19 | Human GARP-TGFβ1 complex | 7.38E+04 | 1.57E-04 | 2.12E-09 |
| C19-3 | Human GARP-TGFβ1 complex | 3.08E+04 | 1.45E-04 | 4.69E-09 |
| C19-7 | Human GARP-TGFβ1 complex | 4.12E+04 | 1.01E-04 | 2.46E-09 |
| C19-8 | Human GARP-TGFβ1 complex | 4.48E+04 | 1.52E-04 | 3.39E-09 |
| Abbv151 | Human GARP-TGFβ1 complex | 6.45E+05 | 2.83E-03 | 4.39E-09 |
| C19 | Mouse GARP-TGFβ1 complex | 5.00E+04 | 5.07E-04 | 1.01E-08 |
| C19-3 | Mouse GARP-TGFβ1 complex | 1.40E+04 | 6.25E-04 | 4.46E-08 |
| C19-7 | Mouse GARP-TGFβ1 complex | 2.48E+04 | 6.10E-04 | 2.46E-08 |
| C19-8 | Mouse GARP-TGFβ1 complex | 2.38E+04 | 6.67E-04 | 2.80E-08 |
| Abbv151 | Mouse GARP-TGFβ1 complex | / | / | / |

| | | | | |
|---|---|---|---|---|
| (Note: "/" indicates that there was no binding) | | | | |

### Example 16. In Vitro Inhibitory Function of Anti-GARP/TGFβ1 Single-Domain Antibodies Against GARP/TGFβ1

2 × 10⁶ LN229 cells (Procell, CL-0578) were transferred to a T75 culture flask (Nunc). After 24 h, the cells were mono-transfected with human or mouse pro-TGFβ1 or co-transfected with human and mouse pro-TGFβ1 and GARP plasmids using the 293fectin^{™} (Invitrogen) transfection reagent. After 24 h, the cells were transferred to a white opaque 96-well cell culture plate (PerkinElmer) at 1 × 10⁴ cells per well. After 24 h, the culture medium was removed using a pipette, and HepG2 CAGA12-luc luciferase reporter cells resuspended in DMEM + 0.5% BSA were added, along with serially diluted C19, humanized antibodies of C19, and Abbv-151 (control antibody). After 20 h of mixed culture, the ONE-Glo^{™} luciferase reagent was added. After 5 min of incubation, chemiluminescence was measured using a multi-mode microplate reader (SpectraMax M5). The luciferase activity value of the vehicle treatment group was normalized to 1, and relative activity = luciferase of antibody treatment group ÷ vehicle control group. Plotting was performed using Prism 9 through non-linear fitting and a three-parameter log(inhibitor) vs. response model.

The results are shown in FIGs. 6A and 6B. C19, C19-3, C19-7, and C19-8 were comparable to the positive control Abbv-151 in inhibiting the activity of the human GARP-TGFβ1 complex, and the difference was that they also inhibited the mouse GARP-TGFβ1 complex.

### Example 17. Binding of Anti-GARP-TGFβ1 Single-Domain Antibody C19-8 to GARP-TGFβ1-Expressing Cells

A 1 × 10⁶ cells/mL suspension of HEK293E cells was prepared, and 5 mL of the cell suspension was placed in a 50 mL culture tube. Then the cells were transfected with a plasmid shown in Table 20 using the 293fectin^{™} reagent. After 48 h, the cells were washed twice with 1× PBS and placed in a 96-well round-bottom cell culture plate (Nunc) at 1 × 10⁵ cells per well. 100 µL of 10 nM antibody or control antibody prepared in 1× PBS was added, and the cells were stained at room temperature for 1 h. After 3 washes with 1× PBS, 100 µL of FITC-labeled anti-human Fc antibody was added, and the cells were stained at room temperature for 30 min. After the staining, the plate was washed 3 times with 1× PBS. 7-AAD was added, and the plate was incubated for 5 min. After 3 washes with 1× PBS, the cells were analyzed using a flow cytometer. The number of FITC-positive cells among viable cells was analyzed by Flowjo, and the percentage of the number of FITC-positive cells to the total number of viable cells was calculated and plotted.

**Table 20. Plasmid structures**

| Plasmid name | Structure |
|---|---|
| Control plasmids (Control plasmids) | pcDNA3.1 |
| TGFβ1 | Human TGFβ1 sequence expressed by hTGFβ1 expression plasmid (Genbank Accession NO. NC_000019.10) |
| TGFβ2 | Human TGFβ2 sequence expressed by hTGFβ2 expression plasmid (Genbank Accession NO. NC 000001.11) |
| TGFβ3 | Human TGFβ3 sequence expressed by hTGFβ3 expression plasmid (Genbank Accession NO. NC_000014.9) |
| TGFβ1-GARP complex | Human TGFβ1 sequence expressed by hTGFβ1 expression plasmid (Genbank Accession NO. NC_000019.10) |
| | Human GARP sequence expressed by GARP expression plasmid (Genbank Accession NO. NC_000011.10) |
| TGFβ2-GARP complex | Human TGFβ2 sequence expressed by hTGFβ2 expression plasmid (Genbank Accession NO. NC_000001.11) |
| | Human GARP sequence expressed by GARP expression plasmid (Genbank Accession NO. NC_000011.10) |
| TGFβ3-GARP complex | Human TGFβ3 sequence expressed by hTGFβ3 expression plasmid (Genbank Accession NO. NC_000014.9) |
| | Human GARP sequence expressed by GARP expression plasmid (Genbank Accession NO. NC_000011.10) |
| mTGFβ1 | Mouse TGFβ1 sequence expressed by mTGFβ1 expression plasmid (Genbank Accession NO. NC_000073.7) |
| mTGFβ1-mGARP complex | Mouse TGFβ1 sequence expressed by mTGFβ1 expression plasmid (Genbank Accession NO. NC_000073.7) |
| | Mouse GARP sequence expressed by mGARP expression plasmid (Genbank Accession NO. NC 000073.7) |

The results are shown in FIG. 7. Both C19-8 and Abbv-151 bound to the human GARP-TGFβ1 complex (corresponding to TGFβ1 + GARP in FIG. 7), and the difference was that C19-8 also bound to the mouse GARP-TGFβ1 complex (corresponding to mTGFβ1 + mGARP in FIG. 7) to a certain extent. In addition, neither C19-8 nor Abbv-151 bound to the TGFβ2 complex (TGFβ2 + GARP) and the TGFβ3 complex (TGFβ3 + GARP).

### Example 18. Study on Epitope Competition of Anti-GARP/TGFβ1 Single-Domain Antibody C19-8 with Ab6 and Abbv-151

In this example, the negative control was HBS-EP, and the positive controls were Ab6 (Scholar rock, WO2020014460A1, which is incorporated herein by reference in its entirety) and Abbv-151. Ab6 binds to GARP-TGFβ1 complexes, LRRC33-TGFβ1 complexes, LTBP1-TGFβ1 complexes, and LTBP3-TGFβ1 complexes, and the binding epitope is completely on TGFβ1 (Martin et al., 2020). The binding epitope of Abbv-151 includes the GARP and TGFβ1 in GARP-TGFβ1 complexes, and it does not bind to GARP or TGFβ1 alone (Streel et al., 2020). The sequences of Ab6 are shown below:
> Ab6 antibody heavy chain: 
EVQLVESGGGLVQPGGSLRLSCTASGFTFSSFSMDWVRQAPGKGLEWVSYISPS
ADTIYYADSVKGRFTISRDNAKNTLYLQMNSLRAEDTAVYYCARGVLDYGDML
MPWGQGTLVTVSSASTKGPSVFPLAPCSRSTSESTAALGCLVKDYFPEPVTVSW
NSGALTSGVHTFPAVLQSSGLYSLSSVVTVPSSSLGTKTYTCNVDHKPSNTKVDK
RVESKYGPPCPPCPAPEFLGGPSVFLFPPKPKDTLMISRTPEVTCVVVDVSQEDPE
VQFNWYVDGVEVHNAKTKPREEQFNSTYRVVSVLTVLHQDWLNGKEYKCKV
SNKGLPSSIEKTISKAKGQPREPQVYTLPPSQEEMTKNQVSLTCLVKGFYPSDIAV
EWESNGQPENNYKTTPPVLDSDGSFFLYSRLTVDKSRWQEGNVFSCSVMHEAL
HNHYTQKSLSLSLGK

(SEQ ID NO: 60)
> Ab6 antibody light chain:

DIQMTQSPSSLSASVGDRVTITCQASQDITNYLNWYQQKPGKAPKLLIYDASNL
ETGVPSRFSGSGSGTDFTFTISSLQPEDIATYYCQQADNHPPWTFGGGTKVEIKR
TVAAPSVFIFPPSDEQLKSGTASVVCLLNNFYPREAKVQWKVDNALQSGNSQES
VTEQDSKDSTYSLSSTLTLSKADYEKHKVYACEVTHQGLSSPVTKSFNRGEC
(SEQ ID NO: 61)

The epitope competition of C19-8 with the positive antibodies Abbv-151 and Ab6 for human GARP/TGFβ1 was assessed using a Biacore T200 (GE Healthcare) instrument. An anti-histidine tag antibody was coupled using a chip (Series S sensor chip CM5, GE Healthcare, Br100530). The kit used was a His capture kit (GE Healthcare, 28-9950-56). The antigen protein human GARP-TGFβ1 complex was captured to the surface of the chip, and antibodies at the same concentration (500 nM) were then allowed to sequentially flow over the surface of the chip in combination. Antibody combinations are shown in Table 21, where the 1^{st} antibody was the first antibody that flowed over the chip, and the 2^{nd} antibody was the second antibody that flowed over the chip.

**Table 21. Antibody combinations**

| 1^{st} antibody | 2^{nd} antibody |
|---|---|
| C19-8 | C19-8 |
| Ab6 | C19-8 |
| Abbv-151 | C19-8 |
| HBS-EP | C19-8 |
| C19-8 | Ab6 |
| Ab6 | Ab6 |
| Abbv-151 | Ab6 |
| HBS-EP | Ab6 |
| C19-8 | Abbv-151 |
| Ab6 | Abbv-151 |
| Abbv-151 | Abbv-151 |
| HBS-EP | Abbv-151 |

Reaction signals were detected in real time using a Biacore instrument to obtain an epitope competition curve. The solution used in the experiment was an HBS-EP solution. At the end of each experimental cycle, the chip was regenerated with a pH 1.5 glycine (GE Healthcare, BR-1003-54) solution. The results of the epitope competition assessment of the antibodies are shown in Table 22.

The results show that C19-8 completely competed with Ab6 and did not compete with Abbv-151 at all. The results, combined with the aforementioned *in vitro* inhibition and cell binding experiments, indicate that C19-8 is an antibody that only inhibits GARP-TGFβ1 complexes like Abbv-151 does, but binds to a different epitope.

**Table 22. The assessment of the epitope competition of C19-8 with the positive antibodies Abbv-151 and Ab6**

| | C19-8 | Ab6 | Abbv151 |
|---|---|---|---|
| C19-8 | | √ | × |
| Ab6 | √ | | × |
| Abbv151 | × | × | |

| | | | |
|---|---|---|---|
| (Note: "√" indicates complete competition; "×" indicates that there was no competition at all) | | | |

### Example 19. Inhibition of Tumor Growth in EMT-6 Mouse Model by Anti-GARP-TGFβ1 Single-Domain Antibody C19-8

To determine the *in vivo* tumor inhibition activity of C19-8, BALB/c mice (female, 6 weeks old, purchased from Beijing Vital River Laboratory Animal Technology Co., Ltd.) were acclimatized for 1 week, and numbered and weighed on the day of the experiment. Mouse breast cancer cells (EMT-6, Procell, CL-0573) in the logarithmic growth phase were collected and resuspended in PBS to a concentration of 5 × 10⁶ cells/mL, and 0.1 mL of the suspension was subcutaneously inoculated into the right flank of each BALB/c mouse. On day 3 after the inoculation, when the mean tumor volume reached about 60 mm³, mice with moderate individual tumor volume were selected and randomly divided into groups as shown in Table 23.

The positive drug M7824 was PD-L1/TGFβ-trap (Merck KGaA, the sequence 3 (heavy chain) and sequence 1 (light chain) in WO2018029367A1 (incorporated herein by reference in its entirety)). mIgG2a-FcS was an mIgG2a Fc with L234A/L235E/G237A/D327Q/A330S/P331S whose ADCC effect was removed, and was used here as an isotype control. RMP1-14-mIgG2a-FcS was an antibody formed by fusing the variable regions of the anti-PD1 antibody RMP1-14 (the sequences 285 (heavy chain variable region) and 286 (light chain variable region) whose signal peptides were removed in WO2018223182A1 (incorporated herein by reference in its entirety)) with an mIgG2a heavy chain constant region with L234A/L235E/G237A/D327Q/A330S/P331S whose ADCC effect was removed/a murine kappa light chain constant region. C19-8-mIgG2a-FcS was an antibody formed by fusing the variable regions of C19-8 with an mIgG2a heavy chain Fc with L234A/L235E/G237A/D327Q/A330S/P331S whose ADCC effect was removed; the sequence is shown below:
> C19-8-mIgG2a-FcS:

EVQLLESGGGLVQPGGSLRLSCTASGFTYCEYDMSWYRQAPGKEREFVSRIASD
GRTSYVDSVKGRFTISQDNSKNTGYLEMNSLRAEDTAVYYCKTEAVKYSGNWC
VAAPGFAYWGQGTLVTVSSPTIKPCPPCKCPAPNAEGAPSVFIFPPKIKDVLMISL
SPIVTCVVVDVSEDDPDVQISWFVNNVEVHTAQTQTHREDYNSTLRVVSALPIQ
HQDWMSGKEFKCKVNNKQLPSSIERTISKPKGSVRAPQVYVLPPPEEEMTKKQ
VTLTCMVTDFMPEDIYVEWTNNGKTELNYKNTEPVLDSDGSYFMYSKLRVEK
KNWVERNSYSCSVVHEGLHNHHTTKSFSRTPGK

(SEQ ID NO: 106)

Administration was started on the day of grouping. The antibodies were administered by intraperitoneal injection at the same molar dose at a frequency of twice weekly, and they were administered 7 times in total. The body weight and tumor volume of the mice were measured twice weekly. The tumor volume was calculated using the formula: TV = L_{long} × Lₛₕₒᵣₜ² / 2. The tumor volume of each group was expressed as mean ± standard deviation. Statistical analysis was performed using two-way ANOVA, and the tumor growth inhibition rate (%TGI) was calculated using the formula: %TGI = [1 - (T - T0) / (C - C0)] × 100%.

The results are shown in FIGs. 8A and 8B. The combined use of C19-8-mIgG2a-FcS and the anti-PD-1 antibody RMP1-14-mIgG2a-FcS significantly inhibited the growth of EMT-6 tumors in mice (p = 0.0017, two-way ANOVA) and extended the survival of the animals (p = 0.0157, Log-rank (Mantel-Cox) test) compared to the RMP1-14-mIgG2a-FcS monotherapy group. During treatment, the mice showed good tolerance, and no significant weight loss occurred (FIG. 8C).

**Table 23. The administration regimen, anti-tumor effect, and median survival time of C19-8 in the EMT-6 model**

| Group | Number of animals | Dose (mg/kg) | %TGI (day 28) | Median survival time (days) |
|---|---|---|---|---|
| mIgG2a-FcS | 10 | 3.5 | - | 21 |
| RMP1-14-mIgG2a-FcS | 10 | 10 | 2.88 | 18 |
| RMP1-14-mIgG2a-FcS + C19-8-mIgG2a-FcS | 10 | 10 + 5.5 | 60.60 | 47 |
| M7824 | 10 | 12 | 23.56 | 29 |

| | | | | |
|---|---|---|---|---|
| Note: mIgG2a-FcS, RMP1-14-mIgG2a-FcS, C19-8-mIgG2a-FcS, and M7824 were administered at the same molar concentration, which was about 68 µmol/kg. | | | | |

### Example 9. Inhibition of Tumor Growth in CT26 Mouse Model by Anti-GARP-TGFβ1 Single-Domain Antibody C19-8

To determine the *in vivo* tumor inhibition activity of C19-8, BALB/c mice (female, 6 weeks old, Beijing Vital River Laboratory Animal Technology Co., Ltd.) were acclimatized for 1 week, and numbered and weighed on the day of the experiment. Mouse colon cancer cells (CT26, National Biomedical Cell-Line Resource, 1101MOU-PUMC000275) in the logarithmic growth phase were collected and resuspended in PBS to a concentration of 3 × 10⁶ cells/mL, and 0.1 mL of the suspension was subcutaneously inoculated into the right flank of each BALB/c mouse. On days 8-9 after the inoculation, when the mean tumor volume reached about 60 mm³, mice with moderate individual tumor volume were selected and randomly divided into groups as shown in Table 24. Administration was started on the day of grouping. The antibodies were administered by intraperitoneal injection at the same molar dose at a frequency of twice weekly, and they were administered 6 times in total. The body weight and tumor volume of the mice were measured twice weekly. The tumor volume was calculated using the formula: TV = L_{long} × Lₛₕₒᵣₜ² / 2. The tumor volume of each group was expressed as mean ± standard deviation. The tumor growth inhibition rate (%TGI) was calculated using the formula: %TGI = [1 - (T - T0) / (C - C0)] × 100%.

The results are shown in FIGs. 9A and 9B. The combined use of C19-8-mIgG2a-FcS and the anti-PD-1 antibody RMP1-14-mIgG2a-FcS inhibited the growth of CT26 tumors in mice and extended the survival of the animals. During treatment, the mice showed good tolerance, and no significant weight loss occurred (FIG. 9C).

**Table 24. The administration regimen, anti-tumor effect, and median survival time of C19-8 in the CT26 model**

| Group | Number of animals | Dose (mg/kg) | %TGI (day 28) | Median survival time (days) |
|---|---|---|---|---|
| mIgG2a-FcS | 14 | 3.5 | - | 24 |
| RMP1-14-mIgG2a-FcS | 14 | 10 | 31.29 | 31.5 |
| RMP1-14-mIgG2a-FcS + C19-8-mIgG2a-FcS | 14 | 10 + 5.5 | 49.26 | 37.5 |

| | | | | |
|---|---|---|---|---|
| Note: mIgG2a-FcS, RMP1-14-mIgG2a-FcS, and C19-8-mIgG2a-FcS were administered at the same molar concentration, which was about 68 µmol/kg | | | | |

Although specific embodiments of the present disclosure have been described above, it will be appreciated by those skilled in the art that these embodiments are merely illustrative and that many changes or modifications can be made to these embodiments without departing from the principles and spirit of the present disclosure. The scope of protection of the present disclosure is therefore defined by the appended claims.

## Claims

1. A TGFβ1-binding molecule, comprising a heavy chain variable region (VH) and a light chain variable region (VL), wherein:
the VH comprises a HCDR1, a HCDR2, and a HCDR3 from the amino acid sequence set forth in SEQ ID NO: 11, and the VL comprises a LCDR1, a LCDR2, and a LCDR3 from the amino acid sequence set forth in any one of SEQ ID NOs: 12 and 21-35; the CDRs are defined according to the Kabat, IMGT, Chothia, AbM, or Contact numbering scheme;
preferably, the VH comprises a HCDR1, a HCDR2, and a HCDR3 set forth in SEQ ID NOs: 13-15, respectively, and the VL comprises a LCDR1, a LCDR2, and a LCDR3 set forth in SEQ ID NOs: 38, 64, and 18, respectively;
preferably, the TGFβ1-binding molecule comprises:
a HCDR1 set forth in SEQ ID NO: 13,
a HCDR2 set forth in SEQ ID NO: 14,
a HCDR3 set forth in SEQ ID NO: 15,
a LCDR1 set forth in any one of SEQ ID NOs: 16, 36, 39, 41-42, 44, 46, 49, 52, 54, and 56-59,
a LCDR2 set forth in any one of SEQ ID NOs: 17, 37, 40, 43, 45, 47-48, 50-51, 53, and 55, and
a LCDR3 set forth in SEQ ID NO: 18.

2. The TGFβ1-binding molecule according to claim 1, wherein:
a) the VH comprises a HCDR1, a HCDR2, and a HCDR3 set forth in SEQ ID NOs: 13-15, respectively, and the VL comprises a LCDR1, a LCDR2, and a LCDR3 set forth in SEQ ID NOs: 59, 40, and 18, respectively;
b) the VH comprises a HCDR1, a HCDR2, and a HCDR3 set forth in SEQ ID NOs: 13-15, respectively, and the VL comprises a LCDR1, a LCDR2, and a LCDR3 set forth in SEQ ID NOs: 36-37 and 18, respectively;
c) the VH comprises a HCDR1, a HCDR2, and a HCDR3 set forth in SEQ ID NOs: 13-15, respectively, and the VL comprises a LCDR1, a LCDR2, and a LCDR3 set forth in SEQ ID NOs: 39-40 and 18, respectively;
d) the VH comprises a HCDR1, a HCDR2, and a HCDR3 set forth in SEQ ID NOs: 13-15, respectively, and the VL comprises a LCDR1, a LCDR2, and a LCDR3 set forth in SEQ ID NOs: 41, 40, and 18, respectively;
e) the VH comprises a HCDR1, a HCDR2, and a HCDR3 set forth in SEQ ID NOs: 13-15, respectively, and the VL comprises a LCDR1, a LCDR2, and a LCDR3 set forth in SEQ ID NOs: 42-43 and 18, respectively;
f) the VH comprises a HCDR1, a HCDR2, and a HCDR3 set forth in SEQ ID NOs: 13-15, respectively, and the VL comprises a LCDR1, a LCDR2, and a LCDR3 set forth in SEQ ID NOs: 44-45 and 18, respectively;
g) the VH comprises a HCDR1, a HCDR2, and a HCDR3 set forth in SEQ ID NOs: 13-15, respectively, and the VL comprises a LCDR1, a LCDR2, and a LCDR3 set forth in SEQ ID NOs: 46-47 and 18, respectively;
h) the VH comprises a HCDR1, a HCDR2, and a HCDR3 set forth in SEQ ID NOs: 13-15, respectively, and the VL comprises a LCDR1, a LCDR2, and a LCDR3 set forth in SEQ ID NOs: 46, 48, and 18, respectively;
j) the VH comprises a HCDR1, a HCDR2, and a HCDR3 set forth in SEQ ID NOs: 13-15, respectively, and the VL comprises a LCDR1, a LCDR2, and a LCDR3 set forth in SEQ ID NOs: 49-50 and 18, respectively;
k) the VH comprises a HCDR1, a HCDR2, and a HCDR3 set forth in SEQ ID NOs: 13-15, respectively, and the VL comprises a LCDR1, a LCDR2, and a LCDR3 set forth in SEQ ID NOs: 46, 51, and 18, respectively;
l) the VH comprises a HCDR1, a HCDR2, and a HCDR3 set forth in SEQ ID NOs: 13-15, respectively, and the VL comprises a LCDR1, a LCDR2, and a LCDR3 set forth in SEQ ID NOs: 52-53 and 18, respectively;
m) the VH comprises a HCDR1, a HCDR2, and a HCDR3 set forth in SEQ ID NOs: 13-15, respectively, and the VL comprises a LCDR1, a LCDR2, and a LCDR3 set forth in SEQ ID NOs: 54-55 and 18, respectively;
n) the VH comprises a HCDR1, a HCDR2, and a HCDR3 set forth in SEQ ID NOs: 13-15, respectively, and the VL comprises a LCDR1, a LCDR2, and a LCDR3 set forth in SEQ ID NOs: 56, 53, and 18, respectively;
o) the VH comprises a HCDR1, a HCDR2, and a HCDR3 set forth in SEQ ID NOs: 13-15, respectively, and the VL comprises a LCDR1, a LCDR2, and a LCDR3 set forth in SEQ ID NOs: 57, 45, and 18, respectively;
p) the VH comprises a HCDR1, a HCDR2, and a HCDR3 set forth in SEQ ID NOs: 13-15, respectively, and the VL comprises a LCDR1, a LCDR2, and a LCDR3 set forth in SEQ ID NOs: 58, 50, and 18, respectively; or,
q) the VH comprises a HCDR1, a HCDR2, and a HCDR3 set forth in SEQ ID NOs: 13-15, respectively, and the VL comprises a LCDR1, a LCDR2, and a LCDR3 set forth in SEQ ID NOs: 16-18, respectively.

3. The TGFβ1-binding molecule according to any one of claims 1-2, wherein: the VH comprises the amino acid sequence set forth in SEQ ID NO: 11 or an amino acid sequence having at least 90% sequence identity thereto;
and,
the VL comprises the amino acid sequence set forth in any one of SEQ ID NOs: 12 and 21-35 or an amino acid sequence having at least 90% sequence identity thereto.

4. The TGFβ1-binding molecule according to any one of claims 1-3, further comprising an immunoglobulin Fc region, wherein preferably, the immunoglobulin Fc region is derived from IgG1, IgG2, IgG3, IgG4, or a variant of any one of the foregoing; more preferably, the immunoglobulin Fc region is derived from human IgG4 or a variant thereof, and the variant comprises mutation 228P.

5. The TGFβ1-binding molecule according to any one of claims 1-4, comprising a heavy chain and a light chain, wherein:
the heavy chain comprises the amino acid sequence set forth in SEQ ID NO: 66 or an amino acid sequence having at least 90% sequence identity thereto, and the light chain comprises the amino acid sequence set forth in any one of SEQ ID NOs: 65 and 67-81 or an amino acid sequence having at least 90% sequence identity thereto; or,
the heavy chain comprises the amino acid sequence set forth in SEQ ID NO: 82 or an amino acid sequence having at least 90% sequence identity thereto, and the light chain comprises the amino acid sequence set forth in SEQ ID NO: 83 or an amino acid sequence having at least 90% sequence identity thereto.

6. The TGFβ1-binding molecule according to any one of claims 1-5, being an anti-TGFβ1 antibody or an antigen-binding fragment thereof, wherein:
preferably, the anti-TGFβ1 antibody is a murine antibody, a chimeric antibody, a humanized antibody, or a human antibody, preferably a humanized antibody, and more preferably a humanized antibody engineered by affinity maturation;
preferably, the antigen-binding fragment is selected from the group consisting of an scFv, a dsFv, a (dsFv)₂, a dsFv-dsFv', an Fv fragment, a Fab, a Fab', and a F(ab')₂.

7. The TGFβ1-binding molecule according to any one of claims 1-6, having at least one of the following properties:
(1) binding to a TGFβ1 precursor protein and/or binding to a TGFβ1 complex,
(2) not binding to a TGFβ2 or TGFβ2 complex,
(3) not binding to a TGFβ3 or TGFβ3 complex,
(4) inhibiting TGFβ1 activity,
(5) inhibiting immunosuppressive activity of regulatory T (T_{reg}) cells,
(6) inhibiting tumor growth, and
(7) inhibiting fibrosis,
wherein preferably, the inhibiting TGFβ1 activity comprises: inhibiting TGFβ1 activation, inhibiting release of mature TGFβ1 from a TGFβ1 complex, and/or inhibiting TGFβ1 signal transduction;
preferably, the TGFβ1 in the TGFβ1 complex is present as a TGFβ1 precursor protein, and the TGFβ1 precursor protein preferably comprises a mature TGFβ1 domain and a latency-associated peptide (LAP);
preferably, the TGFβ1 complex is selected from the group consisting of an LTBP1-TGFβ1 complex, an LTBP3-TGFβ1 complex, an LRRC33-TGFβ1 complex, and/or a GARP-TGFβ1 complex.

8. A GARP-TGFβ1-binding molecule, comprising at least one immunoglobulin single variable domain, wherein the immunoglobulin single variable domain comprises a CDR1, a CDR2, and a CDR3 from the amino acid sequence set forth in any one of SEQ ID NOs: 84 and 90-92, and the CDRs are defined according to the Kabat, IMGT, Chothia, AbM, or Contact numbering scheme;
preferably, the CDR1, CDR2, and CDR3 comprise the amino acid sequences set forth in SEQ ID NOs: 85, 86, and 87, respectively.

9. The GARP-TGFβ1-binding molecule according to claim 8, wherein the immunoglobulin single variable domain is engineered by humanization, affinity maturation, T cell epitope removal, reduction of antibody deamidation, and/or reduction of antibody isomerization;
preferably, a human germline template used during humanization engineering is selected from the group consisting of IGHV3-23 and/or IGJH4.

10. The GARP-TGFβ1-binding molecule according to any one of claims 8-9, wherein the immunoglobulin single variable domain comprises the amino acid sequence set forth in any one of SEQ ID NOs: 84 and 90-92 or an amino acid sequence having at least 90% sequence identity thereto; preferably, the immunoglobulin single variable domain is a VHH.

11. The GARP-TGFβ1-binding molecule according to any one of claims 8-10, further comprising an immunoglobulin Fc region, wherein preferably, the Fc region is derived from IgG1, IgG2, IgG3, IgG4, or a variant of any one of the foregoing; more preferably, the Fc region is derived from human IgG4 or a variant thereof, and the variant comprises mutation 228P.

12. The GARP-TGFβ1-binding molecule according to claim 11, comprising the amino acid sequence set forth in any one of SEQ ID NOs: 89, 93-95, and 106 or an amino acid sequence having at least 90% sequence identity thereto.

13. The GARP-TGFβ1-binding molecule according to any one of claims 8-12, being an antibody or an antigen-binding fragment thereof that binds to a GARP-TGFβ1 complex, wherein:
preferably, the antibody or the antigen-binding fragment thereof is selected from the group consisting of a linear antibody, a single-chain antibody, a nanobody, a peptibody, a domain antibody, a multispecific antibody, and an antigen-binding fragment thereof;
preferably, the antibody or the antigen-binding fragment thereof is a camelid antibody, a chimeric antibody, a humanized antibody, a fully human antibody, or an antigen-binding fragment thereof.

14. A GARP-TGFβ1-binding molecule, comprising an immunoglobulin single variable domain that binds to a GARP-TGFβ1 complex, wherein the GARP-TGFβ1 complex comprises:
a TGFβ1 precursor protein and glycoprotein-A repetitions predominant (GARP); the TGFβ1 precursor protein comprises: a mature TGFβ1 domain and a latency-associated peptide (LAP);
preferably, the GARP comprises the amino acid sequence set forth in SEQ ID NO: 100 or 103 or an amino acid sequence having at least 90% sequence identity thereto, the mature TGFβ1 domain comprises the amino acid sequence set forth in SEQ ID NO: 101 or 104 or an amino acid sequence having at least 90% sequence identity thereto, and/or the LAP comprises the amino acid sequence set forth in SEQ ID NO: 102 or 105 or an amino acid sequence having at least 90% sequence identity thereto.

15. The GARP-TGFβ1-binding molecule according to any one of claims 8-14, having at least one of the following properties:
(1) binding to a GARP-TGFβ1 complex,
(2) not binding to a TGFβ2 protein or a TGFβ2 complex,
(3) not binding to a TGFβ3 protein or a TGFβ3 complex,
(4) not binding to free mature TGFβ1,
(5) inhibiting TGFβ1 activity,
(6) inhibiting immunosuppressive activity of regulatory T (T_{reg}) cells, and
(7) inhibiting tumor growth,
wherein preferably, the inhibiting TGFβ1 activity comprises: inhibiting TGFβ1 activation, inhibiting release of mature TGFβ1 from a GARP-TGFβ1 complex, and/or inhibiting TGFβ1 signal transduction;
preferably, the GARP-TGFβ1 complex comprises (i) GARP and (ii) a TGFβ1 precursor protein, wherein the TGFβ1 precursor protein preferably comprises a mature TGFβ1 domain and a latency-associated peptide (LAP).

16. A polynucleotide, encoding the TGFβ1-binding molecule according to any one of claims 1-7 or the GARP-TGFβ1-binding molecule according to any one of claims 8-15.

17. A vector, comprising the polynucleotide according to claim 16.

18. A host cell, comprising the polynucleotide according to claim 16 or the vector according to claim 17,
wherein preferably, the host cell is a bacterial, yeast, or mammalian cell; more preferably, the host cell is *Escherichia coli, Pichia pastoris,* a Chinese hamster ovary cell, or a human embryonic kidney 293 cell.

19. A pharmaceutical composition, comprising the TGFβ1-binding molecule according to any one of claims 1-7, the GARP-TGFβ1-binding molecule according to any one of claims 8-15, the polynucleotide according to claim 16, or the vector according to claim 17, wherein:
preferably, the pharmaceutical composition further comprises one or more pharmaceutically acceptable excipients, diluents, or auxiliary materials;
preferably, the pharmaceutical composition further comprises an immune checkpoint inhibitor;
preferably, the pharmaceutical composition further comprises an anti-PD-1 antibody or an antigen-binding fragment thereof.

20. A method for preparing the TGFβ1-binding molecule according to any one of claims 1-7, comprising:
expressing the TGFβ1-binding molecule according to any one of claims 1-7 in a host cell, and,
isolating the TGFβ1-binding molecule from the host cell,
wherein optionally, the method further comprises a step of purifying the TGFβ1-binding molecule.

21. A method for preparing the GARP-TGFβ1-binding molecule according to any one of claims 8-15, comprising:
expressing the GARP-TGFβ1-binding molecule according to any one of claims 8-15 in a host cell, and,
isolating the GARP-TGFβ1-binding molecule from the host cell,
wherein optionally, the method further comprises a step of purifying the GARP-TGFβ1-binding molecule.

22. Use of the TGFβ1-binding molecule according to any one of claims 1-7, the GARP-TGFβ1-binding molecule according to any one of claims 8-15, the polynucleotide according to claim 16, the vector according to claim 17, or the pharmaceutical composition according to claim 19 in the manufacture of a medicament for a disease or symptom associated with a TGFβ signaling pathway, wherein:
preferably, the disease associated with the TGFβ signaling pathway is selected from the group consisting of cancer and fibrosis;
preferably, the cancer is selected from the group consisting of lung cancer, intestinal cancer, renal cancer, bladder cancer, liver cancer, gastric cancer, breast cancer, colon cancer, cervical cancer, prostate cancer, and head and neck cancer.

23. A method for preventing or treating a disease or disorder associated with a TGFβ signaling pathway, comprising administering to a subject a prophylactically or therapeutically effective amount of the TGFβ1-binding molecule according to any one of claims 1-7, the GARP-TGFβ1-binding molecule according to any one of claims 8-15, the polynucleotide according to claim 16, the vector according to claim 17, or the pharmaceutical composition according to claim 19,
or,
comprising administering to a subject a prophylactically or therapeutically effective amount of the TGFβ1-binding molecule according to any one of claims 1-7 or the GARP-TGFβ1-binding molecule according to any one of claims 8-15 and a prophylactically or therapeutically effective amount of an immune checkpoint inhibitor,
wherein preferably, the immune checkpoint inhibitor is an anti-PD-1 antibody or an antigen-binding fragment thereof;
preferably, the disease or disorder associated with the TGFβ signaling pathway is selected from the group consisting of cancer and fibrosis; preferably, the cancer is selected from the group consisting of lung cancer, intestinal cancer, renal cancer, bladder cancer, liver cancer, gastric cancer, breast cancer, colon cancer, cervical cancer, prostate cancer, and head and neck cancer.

24. A method for inhibiting TGFβ1 activity *in vitro* or in a subject, comprising:
administering the TGFβ1-binding molecule according to any one of claims 1-7, the GARP-TGFβ1-binding molecule according to any one of claims 8-15, the polynucleotide according to claim 16, the vector according to claim 17, or the pharmaceutical composition according to claim 19 *in vitro* or *in vivo.*

25. Use of a TGFβ1-binding molecule or a GARP-TGFβ1-binding molecule in combination with an immune checkpoint inhibitor in the manufacture of a medicament for treating cancer, wherein the TGFβ1-binding molecule is as defined in any one of claims 1-7, and the GARP-TGFβ1-binding molecule is as defined in any one of claims 8-15;
preferably, the immune checkpoint inhibitor is an anti-PD-1 antibody or an antigen-binding fragment thereof;
preferably, the cancer is selected from the group consisting of lung cancer, intestinal cancer, renal cancer, bladder cancer, liver cancer, gastric cancer, breast cancer, colon cancer, cervical cancer, prostate cancer, and head and neck cancer.
